# EUROPÄISCHE PATENTSCHRIFT

(11) **EP 0 723 969 B1**
(45) Veröffentlichungstag und Bekanntmachung des Hinweises auf die Patenterteilung: **14.11.2001**
(21) Anmeldenummer: 96100762.2
(22) Anmeldetag: 19.01.1996
(51) Int. Cl.: C07F 9/38, C07F 9/40

(54) **Phosphonocarbonsäure-Derivate und deren Verwendung zur Behandlung von degenerativen Gelenkserkrankungen**
Phosphonocarboxylic acid derivatives and their use in treating degenerative articular ailments
Dérivés de l'acide phosphonocarboxylique et leur utilisation dans le traitement de maladies dégénératives des articulations

(30) Priorität: 25.01.1995 DE 19502209
(43) Veröffentlichungstag der Anmeldung: 31.07.1996
(73) Patentinhaber: HOECHST AKTIENGESELLSCHAFT, 65929 Frankfurt am Main (DE)
(72) Erfinder: Graeve, Rolf, Dr., D-65232 Taunusstein (DE); Thorwart, Werner, Dr., D-65239 Hochheim (DE); Raiss, Ruth, Dr., D-60322 Frankfurt (DE); Weithmann, Klaus-Ulrich, Dr., D-65719 Hofheim (DE); Müllner, Stefan, Dr., D-65239 Hochheim (DE)

(56) Entgegenhaltungen:
- EP-A- 0 559 079
- EP-A- 0 601 573
- WO-A-93/24131
- WO-A-94/19358
- CHEMICAL ABSTRACTS, vol. 114, no. 19, 1991 Columbus, Ohio, US; abstract no. 185122e, XP002001286 & J. MED. CHEM., Bd. 34, Nr. 4, 1991, Seiten 1503-1505, H. CHO ET AL:
- CHEMICAL ABSTRACTS, vol. 102, no. 3, 1985 Columbus, Ohio, US; abstract no. 24234e, XP002001287 & SYNTH. COMMUN., Bd. 14, Nr. 11, 1984, Seiten 1059-1065, J. CHENAULT ET AL:
- CHEMICAL ABSTRACTS, vol. 66, no. 13, 1967 Columbus, Ohio, US; abstract no. 55549d, XP002001288 & J. ORG. CHEM., Bd. 31, Nr. 12, - 1966 Seiten 4325-4326, C.N. ROBINSON ET AL:
- JOURNAL OF THE AMERICAN CHEMICAL SOCIETY, Bd. 107, 1985, Seiten 4984-4988, XP002001284 P. MAGNUS ET AL:
- TETRAHEDRON, Bd. 37, Nr. 24, - 1981 Seiten 4321-4326, XP002001285 C. POLLERS-WIEERS ET AL:

## Beschreibung

Arthrose ist eine degenerative Gelenkserkrankung mit entzündlichen Episoden und progredienter Knorpelzerstörung, die zu Funktionsbeeinträchtigung bis hin zu völliger Versteifung führen kann. Bisher sind zwar die begleitenden Entzündungen und Schmerzzustände bei dieser Erkrankung therapierbar, es stehen aber keine Arzneimittel zur Verfügung, die erwiesenermaßen den fortschreitenden Knorpelabbau aufzuhalten oder zu heilen vermögen. Bekannte Therapeutika der Arthrose sind beispielsweise Mischungen von sulfatierten Glukosaminoglykanen (Current Therapeutic Research, 40, 6 (1986) 1034) oder nichtsteroidale Antiinflammatorika, die jedoch den Knorpelverlust nicht. aufzuhalten vermögen. Wenn auch die Pathogenese von Arthrose und Arthritis noch nicht im einzelnen aufgeklärt ist, so gilt jedoch als gesichert, daß die Chondrozyten (Knorpelzellen) maßgeblich an dem verstärkten Matrixverlust beteiligt sind, und daß von den Hauptbestandteilen dieser Matrix vor allem die Proteoglykane (PG) als erste enzymatisch abgebaut werden.

Erfolgversprechende Medikamente zur Arthrosetherapie sind daher solche, die aufgrund ihres Wirkprofils die Proteoglykan-Synthese in Chondrozyten stimulieren und darüberhinaus einem pathologisch beschleunigten Knorpelabbau entgegenwirken. Dabei kann der Abbau der Proteoglykane entweder durch die Hemmung von Matrix-Metalloproteinasen aber auch durch Desaktivierung reaktiver Sauerstoffradikale vermindert werden.

Ein weiterer Ansatzpunkt zur Arthritistherapie ist die erhöhte Konzentration der Substanz P bei der Arthritis. Substanz P (SP) ist ein Neuropeptid, welches sowohl im Zentral- als auch im peripheren Nervensystem weit verbreitet ist (Pernow, Pharmakological Reviews 35: 85 - 141, 1983). Seit 1984 ist die Wirkung und Bedeutung von SP bei der Arthritis bekannt (J. Levine et al., Science, 226: 547 - 549, 1984,). Offensichtlich korreliert die SP-Konzentration in den Gelenken mit der Schwere der Arthritis.

Robinson et al. (J. Org. Chem., Bd. 31, Nr. 12, 1966, Seiten 4325-4326) beschreiben die Herstellung von verschiedenen Arylidenphosphonoacetaten. In der Patentanmeldung WO 93/24131 werden Phosphoncarbonsäure-Derivate zur Behandlung von Erkrankungen des Kalzium- und Phosphat-Stoffwechsels beschrieben. Die Patentanmeldung EP-A-0601573 offenbart α-Phosphocarbonsäure-Derivate als Inhibitoren der Squalensynthese. Cho et al. (J. Med. Chem., Bd. 34, 1991, Seiten 1503-1505) beschreibt Kaffeeinsäure-Derivate die durch Knoevenagel-Kondensation herstellbar sind und sich zur Inhibition der 12-Lipoxygenase, Cyclooxygenase und der Thromboxan-Synthese eignen.

Es wurde nun gefunden, daß die erfindungsgemäßen Verbindungen der Formel I die Proteoglykansynthese im Knorpel stimulieren, den pathologisch gesteigerten enzymatischen Knorpelabbau inhibieren und die durch Sauerstoffradikale ausgelöste Knorpeldestruktion wirksam vermindern. Ferner wurde gefunden, daß die erfindungsgemäßen Verbindungen die Effekte der Substanz P antagonisieren.

In den folgenden Dokumenten werden Phosphonoessigäure-Derivate beschrieben: Pollers-Wieër, C. et al., Tetrahedron 37: 4321 bis 4326, 1981; Magnus, P. et al., J. Am. Chem. Soc. 107: 4984 bis 4988, 1985; Chenault, J. und Dupin, J., Synth. Commun. 14: 1059 bis 1065, 1984. Im Dokument Nippon Shinyako Co. Ltd., Jpn. Kokai Tokkyo Koho 41 ff., 1984, wird die vasodilatorische Wirkung von einem Phosphonoessigsäure-Derivat beschrieben.

Die Erfindung betrifft eine Verbindung der Formel (I) 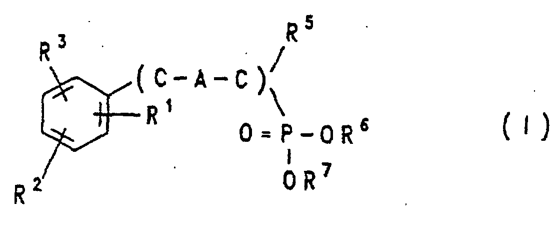 und/oder ein physiologisch verträgliches Salz der Verbindung der Formel (I) und/oder eine stereoisomere Form der Verbindung der Formel (I), wobei mindestens zwei der Reste R¹, R² und R³ vorhanden sind und unabhängig voneinander für
1) OH,
2) (C₁-C₁₂)-Alkoxy,
3) -O-(C₁-C₁₂)-Alkyl-COOH,
4) -O-(C₁-C₁₂)-Alkyl-C(O)-O-(C₁-C₁₂)-Alkyl,
5) (C₃-C₁₂)-Cycloalkoxy,
6) (C₃-C₆)-Alkenyloxy,
7) (C₅-C₇)-Cycloalkyl-(C₁-C₃)-alkoxy,
8) Heteroaryl-(C₁-C₃)-alkoxyl wobei die Heteroatome N, S und/oder O sind,
9) Heterocycloalkyl-(C₁-C₃)-alkoxy, wobei die Heteroatome N, S und/oder O sind, der Heterocycloalkylrest unsubstituiert oder ein- bis dreifach mit (C₁-C₃)-Alkyl substituiert ist und die Heterocycloalkylgruppe fünf- oder sechsgliedrig ist,
10) Phenyl-(C₁-C₃)-alkoxy,
11) Benzyloxy, ein- bis dreifach substituiert durch Halogenmethyl oder (C₁-C₃)-Alkoxy,
12) Phenoxy, ein- bis dreifach substituiert durch (C₁-C₃)-Alkoxy, stehen,
13) zwei der Reste R¹, R² oder R³, welche zwei direkt benachbarte C-Atome des aromatischen Rings substituieren, bilden zusammen einen Methylendioxy- oder Ethylendioxyrest am aromatischen Ring,
14) einen Rest der Formeln II, III oder IV 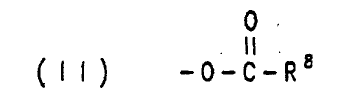 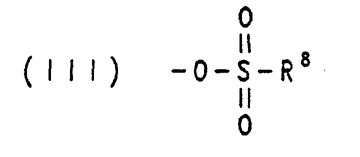 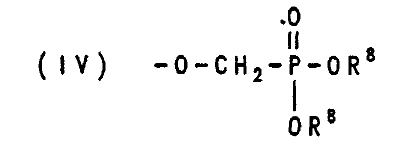 wobei
   - R⁸: (C₁-C₄)-Alkyl oder Wasserstoffatom bedeutet,
15) eine Gruppe der Formel V 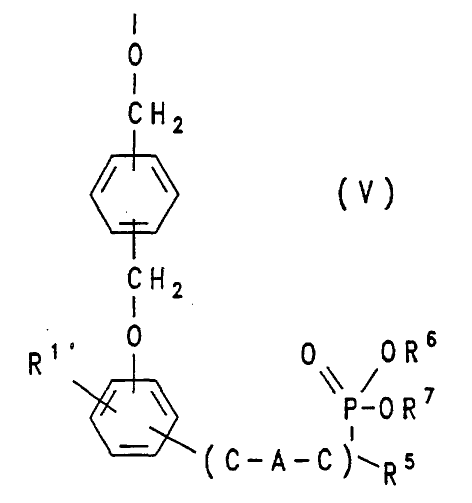 stehen, wobei
   - R^{1'}: wie für R¹ von 1) bis 12) definiert ist und (C-A-C), R⁵, R⁶ und R⁷ wie unten definiert sind, oder
16) R¹ und R⁷ eine kovalente Bindung sind und dadurch eine Verbindung der Formel la bilden 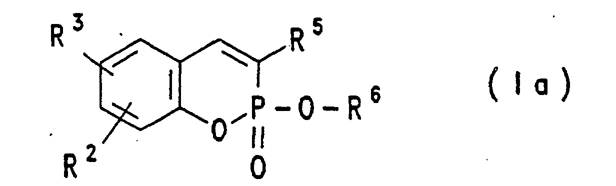 wobei, R² und R³ wie oben und R⁵ und R⁶ wie unten definiert sind, oder
17) R¹ und R⁵ eine kovalente Bindung sind und dadurch eine Verbindung der Formel Ib bilden 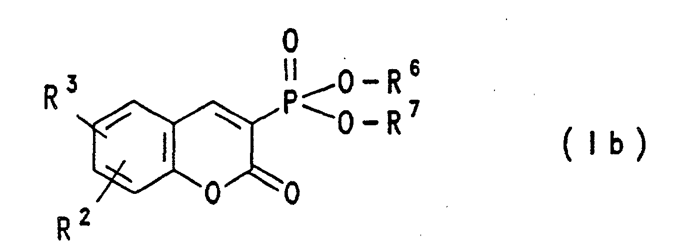 wobei, R² und R³ wie oben und R⁶ und R⁷ wie unten definiert sind, und
   - R⁵: 1) CN,
   2) CH₂NHR⁹ bedeutet, wobei R⁹ Wasserstoffatom oder -C(O)-(C₁-C₃)-Alkyl bedeutet, oder
   3) für einen Rest der Formel VI 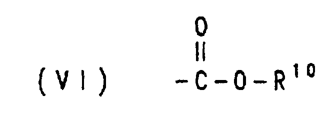 steht, wobei
      R¹⁰
      1) (C₁-C₆)-Alkyl, unsubstituiert oder ein- bis vierfach substituiert durch 1.1 -COOH, 1.2-C(O)-O-(C₁-C₃)-Alkyl oder 1.3 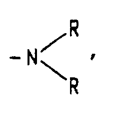 wobei R
         1.3.1 Wasserstoffatom, 2.3.2 (C₁-C₃)-Alkyl bedeutet oder
         1.2.3 zusammen mit dem N-Atom an das es gebunden ist einen Morpholinring bildet, oder
      2) Trialkylsilyl bedeutet,

   R⁶ und R⁷ unabhängig voneinander
   1) Wasserstoffatom oder
   2) (C₁-C₆)-Alkyl bedeuten,
   - (C-A-C) für: 1) (CH=CH-CH=C),
   2) (CH₂-CH₂-CH₂-CH),
   3) (CH₂-CH₂-CH),
   4) (-CH₂-CH) oder
   5) (-CH=C) steht,
mit der Maßgabe, daß
wenn der Rest R⁵ eine CN-Gruppe bedeutet, höchstens einer der Reste R¹, R² oder R³ für einen Hydroxylrest steht, oder wenn der Rest R¹⁰ Methyl, Ethyl oder t-Butyl bedeutet, die Reste R¹, R² oder R³ nicht für Methoxy stehen, sowie die Verbindungen 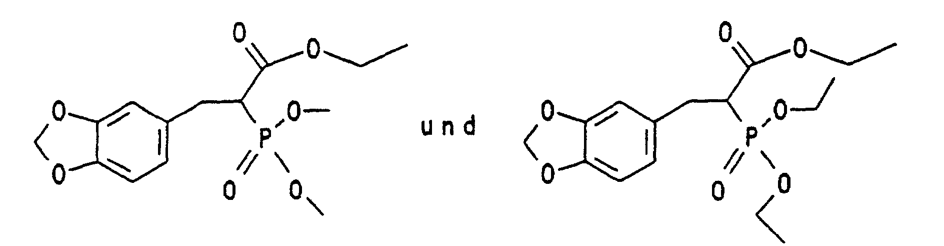 ausgenommen sind.

Bevorzugt ist eine Verbindung der Formel I, wobei mindestens zwei der Reste R¹, R² und R³ vorhanden sind und unabhängig voneinander für
1) OH,
2) (C₁-C₆)-Alkoxy,
3) -O-(C₁-C₆)-Alkyl-COOH,
4) -O-(C₁-C₆)-Alkyl-C(O)-O-(C₁-C₆)-Alkyl,
5) (C₅-C₇)-Cycloalkoxy,
6) (C₃-C₆)-Alkenyloxy,
7) (C₅-C₇)-Cycloalkyl-(C₁-C₂)-alkoxy,
8) Heteroaryl-(C₁-C₂)-alkoxy, wobei die Heteroatome N und/oder O sind,
9) Heterocycloalkyl-(C₁-C₂)-alkoxy, wobei die Heteroatome N und/oder O sind, der Heterocycloalkylrest unsubstituiert oder ein- bis dreifach mit (C₁-C₃)-Alkyl substituiert ist und die Heterocycloalkylgruppe fünf- oder sechsgliedrig ist,
10) Phenyl-(C₁-C₂)-alkoxy,
11) Benzyloxy, ein- bis dreifach substituiert durch Halogenmethyl oder (C₁-C₃)-Alkoxy oder
12) Phenoxy, ein- bis dreifach substituiert durch (C₁-C₃)-Alkoxy, stehen, oder
13) zwei der Reste R¹, R² oder R³ gleichzeitig für eine Gruppe der Formel (II) 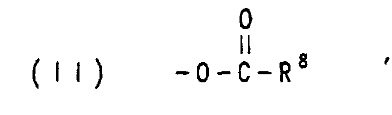 wobei R⁸ (C₁-C₄)-Alkyl bedeutet,
   stehen, und

- R⁵: 1) CN,
2) CH₂NHR⁹ bedeutet, wobei R⁹ Wasserstoffatom oder -C(O)-(C₁-C₃)-Alkyl bedeutet, oder
3) für einen Rest der Formel VI 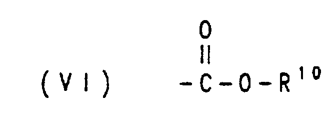 steht, wobei
   R¹⁰
   1) (C₁-C₆)-Alkyl, unsubstituiert oder einfach substituiert durch 1) -COOH, 2) -C(O)-(C₁-C₃)-Alkyl oder 3) 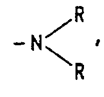 wobei R für (C₁-C₃)-Alkyl steht, oder
   2) Trialkylsilyl bedeutet,
- R⁶ und R⁷: unabhängig voneinander Wasserstoffatom oder (C₁-C₄)-Alkyl bedeuten und
(C-A-C) für 1) (-CH₂-CH) oder 2) (-CH=C) steht.

Insbesondere bevorzugt ist eine Verbindung der Formel I, wobei
- R¹: Methoxy,
- R²: Methoxy oder Benzyloxy,
- R³: Methoxy oder Benzyloxy bedeutet, und
- R⁵: 1) CN,
2) CH₂NHR⁹ bedeutet, wobei R⁹ Wasserstoffatom oder -C(O)-(C₁-C₃)-Alkyl bedeutet, oder
3) für einen Rest der Formel VI steht, wobei
   R¹⁰ (C₁-C₄)-Alkyl, substituiert durch 1) -COOH, 2) 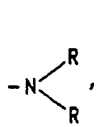 wobei
   R für Wasserstoffatom und/oder (C₁-C₃)-Alkyl steht,
   bedeutet,
- R⁶ und R⁷: Wasserstoffatom oder (C₁-C₄)-Alkyl bedeuten und
(C-A-C) für einen (-CH=C) Rest steht.

Ferner ist eine Verbindung der Formel I bevorzugt, wobei
- R¹: Wasserstoffatom und
- R² und R³: beide Methoxy bedeuten, und
- R⁵: für eine Gruppe der Formel VI steht, wobei
R¹⁰ Isopropyl bedeutet,
- R⁶ und R⁷: gleichzeitig Ethyl oder Methyl bedeuten und
(C-A-C) für einen (-CH₂-CH) Rest steht.

Ganz besonders bevorzugt ist eine Verbindung der Formel I, wobei
- R¹: Wasserstoffatom und
- R² und R³: beide Benzyloxy bedeuten, und
- R⁵: für eine Gruppe der Formel VI steht, wobei
R¹⁰ Isopropyl bedeutet,
- R⁶ und R⁷: Methyl oder Ethyl bedeuten und
(C-A-C) für einen (-CH =C) Rest steht.

Unter den Begriffen Alkyl und Alkoxy werden Reste verstanden, deren Kohlenstoffketten geradkettig oder verzweigt sind. Die cyclischen Alkylreste der Cycloalkylgruppen sind insbesondere 5- bis 7-gliedrige Monocyclen wie Cyclopentyl, Cyclohexyl und Cycloheptyl. Die Heteroarylreste der Heteroarylalkoxygruppen sind insbesondere Reste wie Pyridyl und Thienyl. Die Heterocycloalkylreste der Heterocycloalkylalkoxygruppen sind insbesondere Reste wie Piperidinyl und Morpholinyl. Halogen, im Rest Halogenmethyl, steht für Fluor, Chlor, Brom oder Jod.

Zu der Definition "zwei der Reste R¹, R² oder R³, die zwei direkt benachbarte C-Atome des aromatischen Rings substituieren, bilden zusammen einen Methylendioxy- oder Ethylendioxyrest am aromatischen Ring" gehören beispielsweise die 1.3-Dioxolen- oder 1.4-Dioxan-2-en-reste.

Die Erfindung betrifft weiterhin ein Verfahren zur Herstellung der Verbindung der Formel I, wobei eine Ausführungsform darin besteht, daß man
a) eine Verbindung der Formel VII 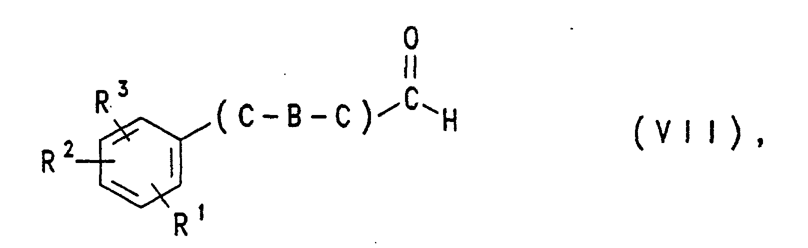 wobei R¹, R² und R³ wie in Formel I definiert sind und (C-B-C) für eine kovalente Bindung, (-CH=CH-), (-CH₂-CH₂-CH₂), (-CH₂-CH₂) oder (-CH₂-) steht,
   mit einer Verbindung der Formel VIII oder mit einem Salz einer Verbindung der Formel VIII 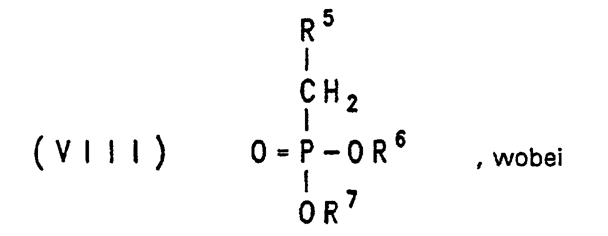 R⁵, R⁶ und R⁷ wie in Formel I definiert sind,
   in Anwesenheit von Tetrahydrofuran und Titantetrachlorid oder in Anwesenheit von Tetrahydrofuran und einem Orthotitansäuretriester der Formel IX

   (IX) (R¹¹)₃TiCl,

   wobei R¹¹ für -O-(C₁-C₆)-Alkyl steht, umsetzt, oder
b) eine nach Verfahren a) hergestellte Verbindung der Formel I, die aufgrund ihrer chemischen Struktur in enantiomeren oder stereoisomeren Formen auftritt, durch Salzbildung mit enantiomerenreinen Säuren oder Basen, Chromatographie an chiralen Stationärphasen oder Derivatisierung mit Hilfe von chiraler enantiomerenreiner Verbindungen wie Aminosäuren, Trennung der somit erhaltenen Diastereomeren, und Abspaltung der chiralen Hilfsgruppe in die reinen Enantiomeren auftrennt oder die Stereoisomeren durch Chromatographie auftrennt, oder
c) eine nach a) hergestellte Verbindung der Formel I, wobei mindestens ein R¹, R² oder R³ ein Rest der Formel II oder III ist, zum entsprechenden Phenol verseift, oder
d) die Umsetzung gemäß Verfahren c) in Gegenwart von Natriumhydrogencarbonat durchführt,
e) eine nach a) hergestellte Verbindung der Formel I, wobei R⁵ für einen Rest der Formel VI steht und/oder mindestens ein R¹, R² oder R³ ein Rest -O-(C₁-C₁₂)-Alkyl-C(O)-O-(C₁-C₁₂)-Alkyl ist, zur Carbonsäure verseift, oder
f) die Umsetzung gemäß Verfahren e) in Gegenwart von einer ethanolischen Kaliumhydroxidlösung oder einer Salzsäurelösung, durchführt, oder
g) eine nach a) hergestellte Verbindung der Formel I, enthaltend eine oder zwei Doppelbindungen mit α.) Wasserstoff und einem Pd/C-Katalysator oder Raney-Nickel oder β.) Natriumborhydrid hydriert, oder
h) einen nach a) hergestellten Phosphonsäuremonoalkyl- oder Phosphonsäuredialkylester der Formel I zum Phosphonsäurehalbester oder zur Phosphonsäure verseift, wobei die Phosphonsäureesterspaltung in Gegenwart von Bromtrimethylsilan in Dichlormethan durchgeführt wird, oder
i) die nach Verfahren a), b), c), e), g), h), k) oder l) hergestellte Verbindung der Formel I entweder in freier Form isoliert oder, im Falle des vorliegen von sauren oder basischen Gruppen, in physiologisch verträgliche kristalline Salze umwandelt, oder
k) eine Verbindung der Formel I, wobei mindestens einer der Reste R¹, R² oder R³ ein Hydroxylrest ist mit einer Verbindung der Formel Xl

   (XI) X-R¹³,

   wobei
   - X: Halogen oder substituierte Phenylsulfonyloxy ist und
   - R¹³: wie für R¹ 2) bis 12) in Formel I definiert ist, wobei X das O-Atom von R¹ ersetzt, welches mit dem Phenylrest verknüpft ist,
   nach Behandlung mit Natriumhydrid oder in Gegenwart von Kaliumcarbonat in Acetonitril, Dimethylformamid oder cyclischen Ketonen umsetzt oder mit R¹³COCl oder 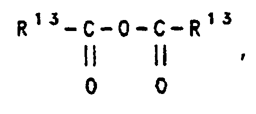 wobei R¹³ wie R⁸ definiert ist,
   gegebenenfalls katalysiert durch Stickstoffbasen wie Pyridin, zu den entsprechenden Phenolethern oder Phenolestern umsetzt,
l) eine nach Verfahren a) hergestellte Verbindung der Formel I, wobei R⁵ für CN steht, in Gegenwart von Wasserstoff und einem Pd/C-Katalysator oder Raney-Nickel, gemäß dem unter C)α.) beschriebenen Verfahren, hydriert, oder
m) eine nach Verfahren l) erhaltene Aminoverbindung mit einem (C₁-C₃)-Alkylcarbonsäureanhydrid, zum entsprechenden Carbonsäureamid umsetzt, oder
n) eine nach Verfahren a) hergestellte Verbindung der Formel I, wobei R⁵ für -COOH steht, durch Veresterung in den entsprechenden Carbonsäureester umsetzt, wobei die Carbonsäuren mit Hilfe von Oxalylchlorid in das Carbonsäurechlorid überführt und dieses mit R¹⁰-OH umgesetzt wird.

Die Erfindung betrifft auch Arzneimittel, gekennzeichnet durch einen wirksamen Gehalt an mindestens einer Verbindung der Formel (I) 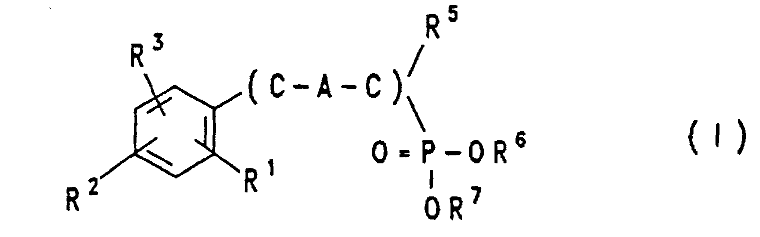 und/oder eines physiologisch verträglichen Salzes der Verbindung der Formel I und/oder eine gegebenenfalls stereoisomere Form der Verbindung der Formel I, wobei die Reste R¹, R², R³, R⁵, R⁶, (C-A-C) und R⁷ wie in Formel I definiert sind, die Maßgaben aber eingeschlossen sind, aber mit der Maßgabe, daß die Verbindung der Formel I, wobei (C-A-C) für (C=CH) steht, R⁵ für eine CN-Gruppe steht, R⁶ und R⁷ gleich sind und für Ethyl stehen, R¹ und R² gleichzeitig -OH bedeuten und in meta- und para-Stellung zu (C-A-C) gebunden sind und R³ wasserstoff atom bedeutet, ausgenommen ist zusammen mit einem pharmazeutisch geeigneten und physiologisch verträglichen Trägerstoff, Zusatzstoff und/oder anderen Wirk- und Hilfsstoff.

Aufgrund ihrer pharmakologischen Eigenschaften eignen sich die erfindungsgemäßen Verbindungen hervorragend zur Behandlung und Prophylaxe von degenerativen Gelenkserkrankungen, von Erkrankungen des rheumatischen Formenkreises, die von einem Knorpelabbau begleitet werden wie die chronische Polyarthritis, das Gelenktrauma sowie der Knorpelschwund infolge längerer Immobilisation des Gelenks, von Entzündungen, septischem Schock, Erkrankungen mit gestörter Leukozyten-Adhäsion, Erkrankungen, die durch eine erhöhte Konzentration an Tumor Nekrose Faktor alpha bedingt sind wie Cachexie oder Morbus Crohn.

Degenerative Gelenkserkrankungen sind beispielsweise Arthrose, sonstige Erkrankungen des rheumatischen Formenkreises mit Knorpelabbau, chronische Polyarthritis, Knorpelschwund nach Gelenktrauma, beispielsweise nach Meniskus- oder Patellaverletzungen oder Bänderrissen, oder Knorpelschwund bei längerer Stillegung von Gelenken.

Die erfindungsgemäßen Arzneimittel können oral, intramuskulär, periartikulär, intraartikulär, intravenös, intraperitoneal, subkutan oder rektal verabreicht werden.

Die Erfindung betrifft auch ein Verfahren zur Herstellung eines Arzneimittels, das dadurch gekennzeichnet ist, daß man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylenglykole und Lösungsmittel, wie etwa steriles Wasser und ein- oder mehrwertige Alkohole, z.B. Glycerin, genannt.

Vorzugsweise werden die Arzneimittel in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien kann diese Dosis bis zu etwa 1000 mg, bevorzugt etwa 50 bis 300 mg, und bei Injektionslösungen in Ampullenform bis zu etwa 300 mg, vorzugsweise etwa 10 bis 100 mg, betragen.

Für die Behandlung eines erwachsenen, etwa 70 kg schweren Patienten sind - je nach Wirksamkeit der Verbindungen gemäß Formel I - Tagesdosen von etwa 20 bis 1000 mg Wirkstoff, vorzugsweise etwa 100 bis 500 mg, indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Die Strukturen aller nachstehend beschriebenen Verbindungen wurden durch Elementaranalysen, Massenspektren, IR- und/oder ¹H-NMR-Spektren belegt. Die NMR-Spektren wurden auf einem Varian Associates. Inc. Gemini 200 - (200 MHz) - Gerät erhalten. Die chemischen Verschiebungen werden in δ-Werten (ppm [parts per million] Tieffeld - verschoben von Tetramethylsilan) ausgedrückt. Die HPLC-Bedingungen für die Bestimmungen des E/Z-Stereoisomerenverhältnisses sind wie folgt:
Analytische Säule: LiChroCART® 125-4 LichroSper® 100 RP-18 endcapped 5 *µ*m (Merck 1.508.0001) bei 1,5 ml/min; Fließmittel A = Acetonitril; Fließmittel B = 0,1 molare Phosphorsäure. Gute Trennungen sind bei linearen Gradienten erhältlich. Hierzu erhöhte man innerhalb von 0 bis 10 Minuten den Lösungsmittelgehalt von m % A auf n % A, anschließend wird für 5 Minuten isokratisch bei einem Lösungsmittelgehalt von n % A eluiert. Der R-Wert [%] gibt den Gehalt vom Stereomeren mit der zugehörigen Retentionszeit t [min] an. UV (254 nm) Detektion. Für die Dünnschichtchromatographie werden Kieselgelplatten (Kieselgel 60 F₂₅₄ spezial 0,25 mm, Riedel-de Haen AG, Seelze) verwendet.
"Eindampfen unter vermindertem Druck" wird mit dem Rotationsverdampfer (Büchi RE 140) bei den vom Gerätehersteller empfohlenen Bedingungen durchgeführt. Säulenchromatographie wird auf Kieselgel 60 (Korngröße 40 bis 63 *µ*m, Merck) durchgeführt.

Die angegebenen Ausbeuten sind nicht optimiert. Die nach den Beispielnummern in Klammern angegebenen Zahlen geben die Nummern der entsprechenden Verbindung(en) in Tabelle I an.

### Beispiel 1 (62)

### 3-(3,4-Dibenzyloxyphenyl)-2-(diethoxyphosphinyl)-propensäureisopropylester (E-Stereomer)

Orthotitansäuretriisopropylesterchlorid [(CH₃)₂CHO]₃TiCl (8,1 ml; 0,034 mol) wird bei 0°C 50 ml Tetrahydrofuran (THF) zugetropft und gerührt, gefolgt von Lösungen von 3,4-Dibenzyloxybenzaldehyd (8 g; 0,0157 mol) und von Phosphonoessigsäuretriethylester (3,52 g; 0,0157 mol) in je 12,5 ml Tetrahydrofuran. Nach anschließendem Weiterrühren (30 Minuten) wird eine Lösung von N-Methylmorpholin (5,56 ml; 0,05 mol) in THF (25 ml) bei 0°C zugetropft. Nach Erwärmen auf Raumtemperatur wird 10 Stunden weitergerührt. Dann wird mit Wasser versetzt (40 ml) und das ausgefallene Titandioxid über eine Nutsche entfernt. Das Filtrat wird dreimal mit je 75 ml Diethylether extrahiert. Die vereinigten organischen Phasen werden nach dem Trocknen über Na₂SO₄ unter vermindertem Druck eingedampft. Das verbleibende Öl wird in einer Kugelrohrapparatur bei einer Temperatur von 60°C und einem Druck von 0,02 mm Hg getrocknet. Man erhält ein hellgelbes Öl.
Ausbeute: 6 g (71 % der Theorie)
Gehalt an E-Stereomeren: ≥ 93 %
HPLC: m = n = 20; R = 94,2; t = 12,34
¹H-NMR: (in CDCl₃; 200 MHz): δ = 8,0 (d; < 0,1 H; J = 43 Hz), 7,2
(m; 14 H), 5,15 (m; 5 H), 4,15 (m; 4 H), 1,36 (t; 6 H), 1,24 (d; 6 H)

| Elementaranalyse für C₃₀H₃₅O₇P (Molekulargewicht (MW) = 538,58 g/mol): | | | |
|---|---|---|---|
| berechnet: | C 66,90 | H 6,56 | P 5,75 |
| gefunden: | C 67,46 | H 6,72 | P 5,58 |

### Beispiel 2 (57)

### 3-(3,4-Diacetoxyphenyl)-2-(diethoxyphosphinyl)-propensäureethylester

Zu 100 ml absolutem Tetrahydrofuran (THF) läßt man unter Feuchtigkeitsausschluß und intensivem Rühren eine Lösung von Titantetrachlorid (7,74 ml; 0,0706 mol) in 16 ml trockenem Dichlormethan beginnend bei 0°C zutropfen. Die exotherme Reaktion läßt man bis 15°C fortschreiten. Nach erneuten Abkühlen auf 0°C fügt man 3,4-Diacetoxybenzaldehyd (8 g; 0,035 mol; E. Pascu und L. v. Vargha, Ber. dtsch. Chem. Ges., 59: 2817, 1926) und Phosphonoessigsäuretriethylester (7,38 g; 0,035 mol) zu. Unter guter Kühlung wird jetzt bei 0°C nach 30 Minuten eine Lösung von trockenem N-Methylmorpholin (14,4 ml; 0,131 mol) in 30 ml absolutem Tetrahydrofuran zugetropft. Man läßt langsam (3 Stunden) auf Raumtemperatur kommen und mindestens 30 Minuten bis maximal über Nacht stehen. Zur Aufarbeitung wird mit Wasser (40 ml) hydrolysiert, abgenutscht und das Filtrat wiederholt mit Diethylether ausgeschüttelt. Die vereinigten organischen Phasen werden mit gesättigter Kochsalz-Lösung gewaschen und über Natriumsulfat getrocknet. Nach dem Einengen unter vermindertem Druck verbleibt ein Öl, das über Kieselgel chromatographiert wird (mit Ethylacetat und Dichlormethan als Elutionsmittel). Man erhält so ein helles Öl. Ausbeute: 8,9 g (58,9 % der Theorie) Gehalt an E-Stereomeren: ≥ 96 % (gemäß HPLC und NMR Daten, s.u.)
HPLC: m = 25; n = 40; R = 96,03; t = 6,21
¹H-NMR: (in CDCl₃; 200 MHz): δ = 8,1 (d; < 0,1 H; J = 43 Hz), 7,55 (d; 1 H; J = 24 Hz), 7,25 (m; 3 H), 4,20 (m; 6 H), 2,3 (S; 6 H), 1,39 (t; 6 H), 1,24 (t; 3 H)

| Elementaranalyse für C₁₉H₂₅O₉P (MW = 428,37 g/mol): | | | |
|---|---|---|---|
| berechnet | C 53,28 | H 5,89 | P 7,23 |
| gefunden | C 52,70 | H 5,62 | P 7,60 |

### Beispiel 3 (47)

### 2-(Diethoxyphosphinyl)-3-(3,4-dihydroxyphenyl)-propensäureethylester

3-(3,4-Diacetoxyphenyl)-2-(diethoxyphosphinyl)-propensäureethylester (3 g; 0,007 mol) aus Beispiel 2 wird unter Schutzgas mit 60 ml gesättigter Natriumbicabonat-Lösung 10 Stunden bei Raumtemperatur gerührt. Man säuert mit 5N Salzsäure an und schüttelt mit Dichlormethan aus. Die organische Phase wird über Natriumsulfat getrocknet und unter vermindertem Druck eingedampft. Der Rückstand wird bei einer Temperatur von 60°C und einem Druck von 0,02 mm Hg in einer Kugelrohrapparatur getrocknet. Man erhält so ein braunes Öl. Ausbeute: 1,9 g (80 % der Theorie)

| Elementaranalyse für C₁₅H₂₁O₇P (MW = 344,30 g/mol): | | | |
|---|---|---|---|
| berechnet: | C 52,33 | H 6,16 | P 8,99 |
| gefunden: | C 52,17 | H 6,67 | P 9,23 |

### Beispiel 5 (61)

### 3-(3,4-Diacetoxyphenyl)-2-diethoxyphosphinyl)-propionsäureethylester(rac.)

3-(3,4-Diacetoxyphenyl)-2-(diethoxyphosphinyl)-propensäureethylester (4,2 g; 0,0098 mol) aus Beispiel 2 wird in 200 ml absolutem Ethanol über 0,5 g 10 prozentigen Pd/C-Katalysator in einer Parr-Hydrierapparatur bei einem Anfangsdruck von 3,45 bar bis zum Ende der Wasserstoffaufnahme hydriert. Der Katalysator wird abfiltriert und das Filtrat unter vermindertem Druck eingedampft. Der Rückstand kann über Kieselgel mit Ethylacetat chromatographiert werden. Nach dem Trocknen in einer Kugelrohrapparatur bei einer Temperatur von 60°C und einem Druck von 0,02 mm Hg wird ein helles Öl erhalten. Ausbeute: 3,2 g (76 % der Theorie)
MS (Cl) m/z 431; (C₁₉H₂₇O₉P; MW = 430,39 g/mol)

### Beispiel 6 (22)

### 3-(3,4-Dibenzyloxyphenyl)-2-(diethoxyphosphinyl)propionsäureisopropylester

Eine Lösung von 3-(3,4-Dibenzyloxyphenyl)-2'-(diethoxyphosphinyl)-propensäureisopropylester (2,5 g; 0,0046 mol) aus Beispiel 1 in 70 ml Ethanol wird bei 0°C mit einer Lösung von Natriumborhydrid (0,095 g; 0,0025 mol) in 10 ml Eiswasser tropfenweise versetzt. Nach dem Erwärmen auf Raumtemperatur wird 2 Stunden weitergerührt. Das Reaktionsgemisch wird mit 5N Salzsäure angesäuert und mehrfach mit Dichlormethan (je 15 ml) extrahiert. Die vereinigten organischen Phasen werden über Natriumsulfat getrocknet und unter vermindertem Druck eingedampft. Es verbleibt ein helles Öl, das nach Dünnschichtchromatographie einheitlich ist (SiO₂; CH₂Cl₂:EtOAc = 7:3).
Ausbeute: 1,8 g (72,4 % der Theorie)
MS (EI) m/z 541; (C₃₀H₃₇O₇P; MW = 540,59 g/mol)

### Beispiel 7 (51)

### Ammoniumhydrogen 2-(3,4-dibenzyloxybenzyliden)-isopropoxycarbonylmethanphosphonat

3-(3,4-Dibenzyloxyphenyl)-2-(diethoxyphosphinyt)-propensäureisopropylester (2 g; 0,0037 mol) aus Beispiel 1 wird zusammen mit Bromtrimethylsilan (0,52 ml; 0,004 mol) in Dichlormethan (70 ml) 3 Tage bei Raumtemperatur gerührt. Zur Beendigung der Reaktion wird nochmals Bromtrimethylsilan (0,52 ml; 0,004 mol) zugesetzt und weiter bei Raumtemperatur gerührt (6 Stunden). Nach Zugabe von ammoniakalischem Ethanol wird unter vermindertem Druck eingedampft. Der teilweise kristalline Rückstand wird mit Isopropanol dispergiert. Der Niederschlag wird durch Filtration abgetrennt und die Mutterlauge unter vermindertem Druck am Rotationsverdampfer auf etwa die Hälfte des Volumens eingeengt. Durch Abfiltrieren des gebildeten Niederschlages und dessen Trocknung wird die eine Festsubstanz mit dem Schmelzpunkt 187 bis 188°C erhalten. Ausbeute: 1 g (54 % der Theorie) MS (FAB), (M+Na⁺) m/z 505,2; C₂₈H₂₇O₇P+Na⁺; (MW = 505,41 g/mol)
NMR (200 MHz, DMSO): δ = 7,2 bis 7,5 (m; 10 H), 6,8 bis 7,1 (m; 4 H), 5,08 (d; 4 H; J = 16,7 Hz), 4,95 (m; 1 H), 1,15 (d; 6 H; J = 6,2 Hz)

### Beispiel 8 (8)

### 3-[3,5-Dimethoxy-4-(4-methoxybenzyloxy)phenyl]-2-(dimethoxyphosphinyl)-propensäureisopropylester

3-(3,5-Dimethoxy-4-hydroxyphenyl)-2-(dimethoxyphosphinyl)-propensäureisopropylester (4 g; 0,01 mol; Tabelle 1, Nr. 10) wird in 60 ml Acetonitril gelöst und mit Kaliumcarbonat (4,45 g; 0,032 mol) und 4-Methoxybenzylchlorid (1,4 ml; 0,011 mol) versetzt und zunächst 8 Stunden bei Raumtemperatur gerührt. Zur Beendigung der Reaktion erfolgt eine Zugabe von 0,7 ml 4-Methoxybenzylchlorid und weiteres Rühren für 4 Stunden, wonach die Umsetzung gemäß dem Ergebnis einer Dünnschichtchromatographie vollständig ist. Nach Abfiltrieren von Kaliumcarbonat wird das Filtrat unter vermindertem Druck zur Trockene eingedampft und der Rückstand chromatographiert (SiO₂; CH₂Cl:AcOEt = 8:2). So wird ein langsam kristallisierendes Öl erhalten, erstarrt zeigt es den Schmelzpunkt 68 bis 69°C.
Ausbeute: 4,4 g (88,9 % der Theorie)

| Elementaranalyse für C₂₄H₃₁O₉P (MW = 494,48 g/mol): | | | |
|---|---|---|---|
| berechnet | C 58,30 | H 6,33 | P 6,26 |
| gefunden | C 58,10 | H 6,70 | P 6,30 |

### Beispiel 9 (72)

### 2-Dimethoxyphosphinyl)-3-(3-hexyloxy-4-methoxyphenyl)-propensäureisopropylester

2-(Dimethoxyphosphinyl)-3-(3-hydroxy-4-methoxyphenyl)-propensäureisopropylester (2 g; 0,0058 mol; Tabelle 1, Nr. 26), 1-Bromhexan (0,85 ml; 0,006 mol), 30 ml Dimethylformamid (DMF) und Kaliumcarbonat (gemahlen, 0,89 g; 0,0065 mol) werden vereinigt und 6 Stunden bei Raumtemperatur gerührt. Nach Abfiltrieren der anorganischen Salze wird das Filtrat unter vermindertem Druck eingedampft. Der erhaltene ölige Rückstand wird an einer Kugelrohrapparatur bei 70°C/0,02 mm Hg von DMF-Resten befreit und dann über Kieselgel mit einem Elutionsgemisch von Dichlormethan:Essigester (8:2) chromatographiert. So wird ein helles Öl erhalten. Ausbeute: 3,6 g (72,5 % der Theorie)

| Elementaranalyse für C₂₁H₃₃O₇P (MW = 428,47 g/mol): | | | |
|---|---|---|---|
| berechnet | C 58,87 | H 7,78 | P 7,23 |
| gefunden | C 58,5 | H 7,7 | P 7,0 |

HPLC: m = 20, n = 60, R = 96,67, t = 11,549; NMR (200 MHz, CDCl₃): δ = 8,1 (d; < 0,1 H; J = 42 Hz), 7,53 (d; 1 H; J = 23 Hz), 6,8 bis 7,3 (m; 4 H), 5,2 (sept; 1 H), 3,6 bis 4,1 (m; 12 H), 1,2 bis 2,0 (m; 15 H)

### Beispiel 10 (37 und 38)

### Z und E 2-(Diethoxyphosphinyl)-3-(3,4-dimethoxyphenyl)-acrylnitril

Entsprechend Beispiel 1 wird 3,4-Dimethoxybenzaldehyd (21 g; 0,123 mol) mit Cyanmethanphosphonsäurediethylester (22,32 g; 0,123 mol), Orthotitansäuretriisopropylesterchlorid (61,5 ml; 0,26 mol) und N-Methylmorpholin (28 ml; 0,25 mol) in 300 ml Tetrahydrofuran umgesetzt und aufgearbeitet. Bei der Chromatographie auf Kieselgel mit dem Elutionsmittel Dichlormethan-Essigsäureethylester (2:1) können die beiden stereomeren Formen der Titelverbindung als weitgehend einheitliche Fraktionen abgetrennt werden.
1.) Z-Stereomer (2,6 g):

| Elementaranalyse für C₁₅H₂₀NO₅P (MW = 325,30 g/mol): | | | | |
|---|---|---|---|---|
| berechnet: | C 55,38 | H 6,21 | N 4,31 | P 9,52 |
| gefunden: | C 55,77 | H 6,54 | N 4,51 | P 9,27 |

HPLC: m = 5, n = 50, R = 81,72, t = 10,904
NMR (200 MHz, CDCl₃): δ = 6,8 bis 8,0 (m, 4 H, darin enthalten: 7,80 (d; 1 H;
J = 40 Hz)), 4,1 bis 4,3 (m; 4 H), 4,95 (s; 6 H), 1,3 (t; 6 H)
2.) Stereomerengemisch (19 g)
3.) E-Stereomer (4,5 g):

| Elementaranalyse für C₁₅H₂₀NO₅P (MW = 325,30 g/mol): | | | | |
|---|---|---|---|---|
| berechnet | C 55,38 | H 6,21 | N 4,31 | P 9,52 |
| gefunden | C 55,53 | H 6,56 | N 4,41 | P 9,57 |

HPLC: m = 5, n = 50, R = 98,83, t = 11,549
NMR (200 MHz, CDCl₃): δ = 7,90 (d; 1 H; J = 21 Hz), 6,8 bis 7,8 (m, 3 H), 4,1 bis 4,4 (m; 4 H), 3,9 (s; 6 H), 1,4 (t; 6 H)

### Beispiel 11 (19)

### {2-Acetylamino-1-[(3,4-dimethoxyphenyl)-methyl]-ethyl}phosphonsäurediethylester

2-(Diethoxyphosphinyl)-3-(3,4-dimethoxyphenyl)-acrylnitril (Tabelle 1, Nr. 37/38; Stereomerengemisch; 4 g; 0,0123 mol) wird in Essigsäureanhydrid (100 ml) gelöst und in einer Parr-Hydrierapparatur in Gegenwart von Raney-Nickel-Katalysator (0,6 g) und Natriumacetat (wasserfrei; 1,2 g; 0,015 mol) bei einem Anfangsdruck von 3,45 bar bis zum Ende der Wasserstoffaufnahme hydriert. Natriumacetat und Katalysator werden abfiltriert und das Filtrat unter vermindertem Druck (30 mbar) eingedampft. Der Rückstand wird über Kieselgel chromatographiert (Elutionsmittel: Essigester:Ethanol, 9:1). Dabei wird die Titelverbindung als einheitliche Fraktion von 1,4 g (30,5 % der Theorie) erhalten.
MS (ES) m/z 374 (M+H⁺); C₁₇H₂₈NO₆P; (MW = 373,39 g/mol)

### Beispiel 12 (45)

### 3-(3,4-Diacetoxyphenyl)-2-(diethoxyphosphinyl)-propionnitril

3-(3,4-Diacetoxyphenyl)-2-(diethoxyphosphinyl)-acrylnitril (Tabelle 1, Nr. 56; 2,5 g; 0,0066 mol) wird in 100 ml Acetanhydrid über 0,5 g 10 prozentigen Pd/C-Katalysator in einer Parr-Hydrierapparatur bei einem Anfangsdruck von 3,45 bar bis zum Ende der Wasserstoffaufnahme hydriert (16 Stunden), wobei nach Ablauf von 8 Stunden zusätzlich 0,3 g Katalysator zugegeben werden. Schließlich wird der Katalysator abfiltriert und das Filtrat unter vermindertem Druck eingedampft. Der Rückstand wird über Kieselgel chromatographiert, wobei mit dem Lösungsmittelgemisch Dichlormethan:Essigsäureethylester (7:3) eluiert wird. So erhält man die Titelverbindung als helles Öl in einer Ausbeute von 1,7 g (67,7 % der Theorie).
MS (EI) m/z 384 (M+H⁺); C₁₇H₂₂NO₇P; (MW = 383,34 g/mol)

### Beispiel 13 (6)

### 1,4-Bis-{[5-(2-dimethoxyphosphinyl-2-isopropoxycarbonylethenyl)-2-methoxyphenoxy]-methyl}-benzol

3-Hydroxy-4-methoxybenzaldehyd (3 g; 0,0197 mol), α,α'-Dibrom-p-xylol (2,3 g; 0,009 mol) und gepulvertes Kaliumcarbonat (4 g; 0,03 mol) werden in Acetonitril (20 ml) 6 Stunden bei Raumtemperatur gerührt. Danach wird das Reaktionsgemisch filtriert. Im Filtrat sind nach dem Eindampfen unter vermindertem Druck nur geringe Mengen an Produkt zu finden. Der Filterrückstand wird in Wasser aufgenommen und mehrfach mit Dichlormethan ausgeschüttelt. Die vereinigten organischen Phasen werden über Natriumsulfat getrocknet und unter vermindertem Druck eingedampft, zum Schluß bei 60° und 0,03 mbar. So wird 1,4-Bis-[(5-formyl-2-methoxyphenoxy)-methyl]-benzol in Form weißer Kristalle (2,2 g; 54,9 % der Theorie) mit dem Schmelzpunkt 180 bis 181°C erhalten.
¹H-NMR (in CDCl₃, 200 MHz): δ = 9,81 (s; 2 H), 6,9 bis 7,6 (m; 10 H), 5,19 (s; 4 H), 3,96 (s; 6 H)
Orthotitansäuretriisopropylesterchlorid (1,6 ml; 0,00984 mol) wird bei 0°C zu Tetrahydrofuran (75 ml) zugetropft. Man fügt dann bei gleicher Temperatur tropfenweise je eine Lösung von dem oben erhaltenen 1,4-Bis-[(5-formyl-2-methoxyphenoxy)-methyl]-benzol (2 g; 0,00492 mol) und von Phosphonoessigsäuretrimethylester (1,6 ml; 0,00984 mol) in 10 ml trockenem Tetrahydrofuran unter Argonatmosphäre zu. Nach halbstündigen Rühren bei 0°C wird eine Lösung von N-Methyl-morpholin (2,2 ml; 0,0196 mol) in Tetrahydrofuran (5 ml) zugesetzt. Das Reaktionsgemisch läßt man sich auf Raumtemperatur erwärmen und 8 Stunden weiterrühren. Danach wird mit Wasser (20 ml) versetzt. Die ausgefallenen Salze werden abgesaugt und das Filtrat mehrfach mit Diethylether ausgeschüttelt. Die organischen Phasen werden vereinigt, über Natriumsulfat getrocknet und unter vermindertem Druck eingedampft. Das erhaltene gelbe Öl wird über Kieselgel chromatographiert (Elutionsmittel: Essigsäureethylester). So wird die Titelverbindung als einheitliche Fraktion in einer Ausbeute von 1,5 g (38,6 % der Theorie) in Form eines hellen Öles erhalten.
MS (FAB) m/z 791,3 (M+H⁺); C₃₈H₄₈O₁₄P₂; (MW = 790,75 g/mol)
HPLC: m = 40, n = 80, R = 49,33, t = 8,717

### Beispiel 14 (5 und 4)

### 1,4-Bis-{[5-{2-diethoxyphosphinyl-2-cyanoethenyl)-2-methoxyphenoxy]-methyl}-benzol (und als Nebenprodukt: 3-[3-(Brommethyl)-benzlyoxy)-4-methoxyphenyl]-2-(diethoxyphosphinyl)-acrylnitril)

Analög Beispiel 8 läßt man 2-(Diethoxyphosphinyl)-3-(3-hydroxy-4-methoxyphenyl)-acrylnitril (Tabelle 1, Nr. 77; 6 g; 0,019 mol), α,α'-Dibrom-p-xylol (2,5 g; 0,0096 mol), Kaliumcarbonat (1,45 g; 0,011 mol), ein paar Kristalle Kaliumjodid als Katalysator und Acetonitril (60 ml) zusammen bei Raumtemperatur rühren (4 Tage). Danach fügt man weiteres Kaliumcarbonat (1,3 g; 0,009 mol) zu und läßt bei 50°C weiterrühren (1 Tag). Nach dem Filtrieren wird das Filtrat unter vermindertem Druck eingedampft und der Rückstand über Kieselgel chromatographiert (Elutionsmittel: Dichlormethan:Essigsäureethylester:Petrolether, 6:2:2). Als erste einheitliche Fraktion wird 3-[3-(Brommethyl)benzyloxy)-4-methoxyphenyl]-2-(diethoxyphosphinyl)-acrylnitril mit dem Schmelzbereich 90 bis 107°C (Tabelle 1, Nr. 4) in einer Menge von 0,7 g (7,35 % der Theorie) aufgefangen.
MS (ES) m/z 494,3 (M+H⁺); C₂₂H₂₅BrNO₅P; (MW = 494,33 g/mol)
HPLC: m = 40, n = 80, R = 81,37, t = 6,853
Nach der Eluation einer geringen Menge des Ausgangsnitrils folgt als weitere einheitliche Fraktion die Titelverbindung in einer Ausbeute von 2,7 g (38,6 % der Theorie) mit dem Schmelzpunkt 166 bis 169°C.

| Elementaranalyse für C₃₆H₄₂N₂O₁₀P₂ (MW = 724,69 g/mol): | | | | |
|---|---|---|---|---|
| berechnet | C 59,67 | H 5,85 | N 3,87 | P 8,55 |
| gefunden | C 60,0 | H 5,7 | N 3,9 | P 8,1 |

HPLC: m = 40, n = 80, R = 88,27, t = 8,635

### Beispiel 15 (33 und 34)

### 2-(Diethoxyphosphinyl)-3-(3,4-dimethoxyphenyl)-propylamin(I) und 2-(Diethoxyphosphinyl)-3-(3,4-dimethoxyphenyl)-propionnitril(II)

2-(Diethoxyphosphinyl)-3-(3,4-dimethoxyphenyl)-acrylnitril, als E- und Z-Stereomerengemisch (Tabelle 1, Nr. 37 und 38; 4 g; 0,012 mol) wird in 100 ml Ethanol über 0,6 g 10 prozentigen Pd/C-Katalysator in einer Parr-Hydrier-apparatur bei einem Anfangsdruck von 3,45 bar bis zum Ende der Wasserstoff-aufnahme hydriert. Der Katalysator wird abfiltriert und das Filtrat unter vermindertem Druck eingedampft. Den Rückstand chromatographiert man über Kieselgel mit Essigsäureethylester-Dichlormethan (9:1). Als einheitliche Fraktionen erhält man 0,7 g (17,2 % der Theorie) der Titelverbindung I.

| Elementaranalyse für C₁₅H₂₆NO₅P (MW = 331,35 g/mol): | | | |
|---|---|---|---|
| berechnet | C 54,37 | H 7,93 | N 4,23 |
| gefunden | C 54,6 | H 7,4 | N 4,1 |

IR (KBr): keine Bande bei 2240 cm⁻¹
und 1,15 g (28,6 % der Theorie) der Titelverbindung II ebenfalls als Öl.

| Elementaranalyse für C₁₅H₂₂NO₅P (MW = 327,32 g/mol): | | | |
|---|---|---|---|
| berechnet | C 55,04 | H 6,79 | N 4,28 |
| gefunden | C 54,6 | H 7,0 | N 4,5 |

MS(EI) m/z 328 (M+H⁺) IR (KBr): u.a. 2240 cm⁻¹

### Beispiel 17 (79)

### 3-(3,4-Dibenzyloxyphenyl)-2-(diethoxyphosphinyl)-propensäureisopropylester (Z-Stereomer)

Zu einer Suspension von Natriumhydrid (0,23 g; 0,0094 mol) in 70 ml Tetrahydrofuran wird bei Raumtemperatur eine Lösung von Phosphonoessigsäuretriethylester (2,1 g; 0,00094 mol) in 15 ml Tetrahydrofuran unter Argonatmosphäre bei Raumtemperatur zugetropft und so lange gerührt, bis eine klare Lösung entstanden ist. Die Reaktionslösung wird darauf 1 Stunde unter Rückfluß erhitzt, auf -78°C abgekühlt und mit Orthotitansäuretriisopropylesterchlorid (2,25 ml; 0,0094 mol) versetzt. Man läßt auf Raumtemperatur kommen und rührt 1,5 Stunden nach.
3,4-Dibenzyloxybenzaldehyd (3 g; 0,0094 mol) wird in 15 ml Tetrahydrofuran gelöst und ins obige Gemisch getropft. Nach vierstündigen Rühren wird das Reaktionsgemisch in verdünnte Salzsäure gegossen und mehrere Male mit Diethylether extrahiert. Die vereinigten organischen Phasen werden über Natriumsulfat getrocknet und unter vermindertem Druck eingedampft. Der ölige Rückstand wird an einer Kugelrohrapparatur bei 60°/0,02 mm Hg getrocknet. Die anschließende Chromatographie auf Kieselgel mit Dichlormethan/ Essigsäureethylester (4:1) ergab zwei Hauptfraktionen:
1.) 0,1 g eines hellen Öl (überwiegend E-Stereomer, Tabelle 1, Nr. 62
2.)2 g der Titelverbindung als helles Öl (39,5 % der Theorie).

| Elementaranalyse für C₃₀H₃₅O₇P (MW = 538,58 g/mol): | | | |
|---|---|---|---|
| berechnet | C 66,90 | H 6,54 | P 5,75 |
| gefunden | C 66,3 | H 6,3 | P 5,5 |

HPLC: m = 50, n = 80, R = 90,34, t = 5,152
NMR (200 MHz, CDCl₃): δ = 8,0 (d; 0,9 H; J = 40 Hz), 6,7 bis 7,6
(m; > 13 H incl. Anteil E-Stereomer und CDCl₃), 5,05 bis 5,25 (m; 5 H), 3,9 bis 4,3 (m; 4 H), 1,1 bis 1,4 (m; 12 H)

### Beispiel 18 (93)

### 3-(3-Acetoxy-4-methoxyphenyl)-2-(dimethoxyphosphinyl)-propensäureisopropylester

2-(Dimethoxyphosphinyl)-3-(3-hydroxy-4-methoxyphenyl)-propensäureisopropylester (Tabelle 1, Nr. 26; 2 g; 0,0058 mol) wird in 15 ml Acetanhydrid mit 2 Tropfen Pyridin zunächst 10 Minuten bei 10°C, dann 6 Stunden bei Raumtemperatur gerührt. Zur Hydrolyse des überschüssigen Acetanhydrids wird mit 15 ml Wasser versetzt und gerührt. Das Reaktionsgemisch wird mehrfach mit Dichlormethan ausgeschüttelt. Die vereinigten organischen Phasen werden über Natriumsulfat getrocknet und unter vermindertem Druck eingedampft. Das verbleibende Öl wird an einer Kugelrohrapparatur bei 60°C/0,02 mm Hg getrocknet. So werden 1,7 g (75,9 % der Theorie) erhalten.
MS (DCI) m/z 495,2 (M+H⁺)
HPLC: m = 5, n = 50, R = 92,00, t = 11,417

### Beispiel 19 (94)

### 3-(4-Diethoxyphosphinylmethoxy-3-methoxyphenyl)-2-(dimethoxyphosphinyl)-propensäureisopropylester

2-(Dimethoxyphosphinyl)-3-(4-hydroxy-3-methoxyphenyl)-propensäureisopropylester (Tabelle 1, Nr. 69; 2 g; 0,0058 mol) und Natriumhydrid (0,144 g; 0,006 mol) werden in 10 ml Dimethylsulfoxid 20 Minuten bei Raumtemperatur gerührt. Danach wird eine Lösung von 4-Chlorphenylsulfonyloxymethylphosphonsäurediethylester [Org. Synth., 64: 80, 1985] (2,23 g; 0,0065 mol) in 10 ml Dimethylsulfoxid zugesetzt. Das Reaktionsgemisch wird 10 Stunden bei Raumtemperatur gerührt und dann unter vermindertem Druck eingedampft. Das verbleibende Öl wird über Kieselgel mit Essigsäureethylester:Ethanol (9,5:0,5) chromatographiert. Es werden 0,4 g (16 % der Theorie) eines hellen Öls erhalten.
MS (DCI) m/z 495,2 (M+H⁺)
HPLC: m = 5, n = 50, R = 94,21, t = 11,346

### Beispiel 20

### 3-(4-Carboxymethoxy-3-methoxyphenyl)-2-(dimethoxyphosphinyl)-propensäureisopropylester (E-Stereomer)

3-(4-tert.-Butoxycarbonylmethoxy-3-methoxyphenyl)-2-(dimethoxyphosphinyl)-propensäureisopropylester (Tabelle 1, Nr. 91; 0,5 g; 0,011 mol) wird in 10 ml 5 N Salzsäure 4 Stunden bei 60°C gerührt und danach auf Raumtemperatur abgekühlt. Die ausgefallenen Kristalle werden abgesaugt, mehrfach mit Wasser gewaschen und getrocknet. So werden 0,27 g (61,5 % der Theorie) mit dem Schmelzpunkt 156-158°C erhalten.
MS (FAB) m/z 403,2 (M + H⁺)
HPLC: m = 15, n = 70, R = 92,44, t = 6,677

### Beispiel 21 (110)

### 3-(3,4-Dimethoxyphenyl)-2-(dimethoxyphosphinyl)-propensäure-2-(4-morpholinyl) ethylester

3-(3,4-Dimethoxyphenyl)-2-(dimethoxyphosphinyl)-propensäure (Tabelle 1, Nr. 107; 2,3 g; 0,0069 mol) und in Toluol (30 ml) suspendiert und mit einer Lösung von Oxalylchlorid (0,6 ml; ,007 mol) in 2 ml Toluol versetzt gefolgt von 3 Tropfen Dimethylformamid. Die Carbonsäure geht in Lösung. Es wird 2 Stunden weitergerührt und unten vermindertem Druck eingedampft. Das erhaltene gelbe Öl zeigt unter anderem eine IR-Bande bei 1775 cm⁻¹ aus und wird als Rohprodukt weiter umgesetzt. Ausbeute: 2,2 g (roh) Das so erhaltene Carbonsäurechlorid wird in 70 ml Acetonitril gelöst, mit N-(2-Hydroxyethyl)-morpholin versetzt und 10 Stunden gerührt. Die Reaktionslösung wird unter vermindertem Druck eingedampft. Man nimmt den Rückstand mit 50 ml Dichlormethan auf und wäscht mit gesättigter Natriumbicarbonatlösung. Nach dem Trocknen über Natriumsulfat wird unter vermindertem Druck eingedampft und das verbleibende Öl durch Chromatographie über Kieselgel (Essigester:Ethanol = 8:2) gereinigt. So werden 0,6 g (20 % der Theorie) eines zähen Öles erhalten.
MS (FAB) m/z 430,2 (M + H⁺)
HPLC: m = 0, n = 70, R = 84,02, t = 7,107

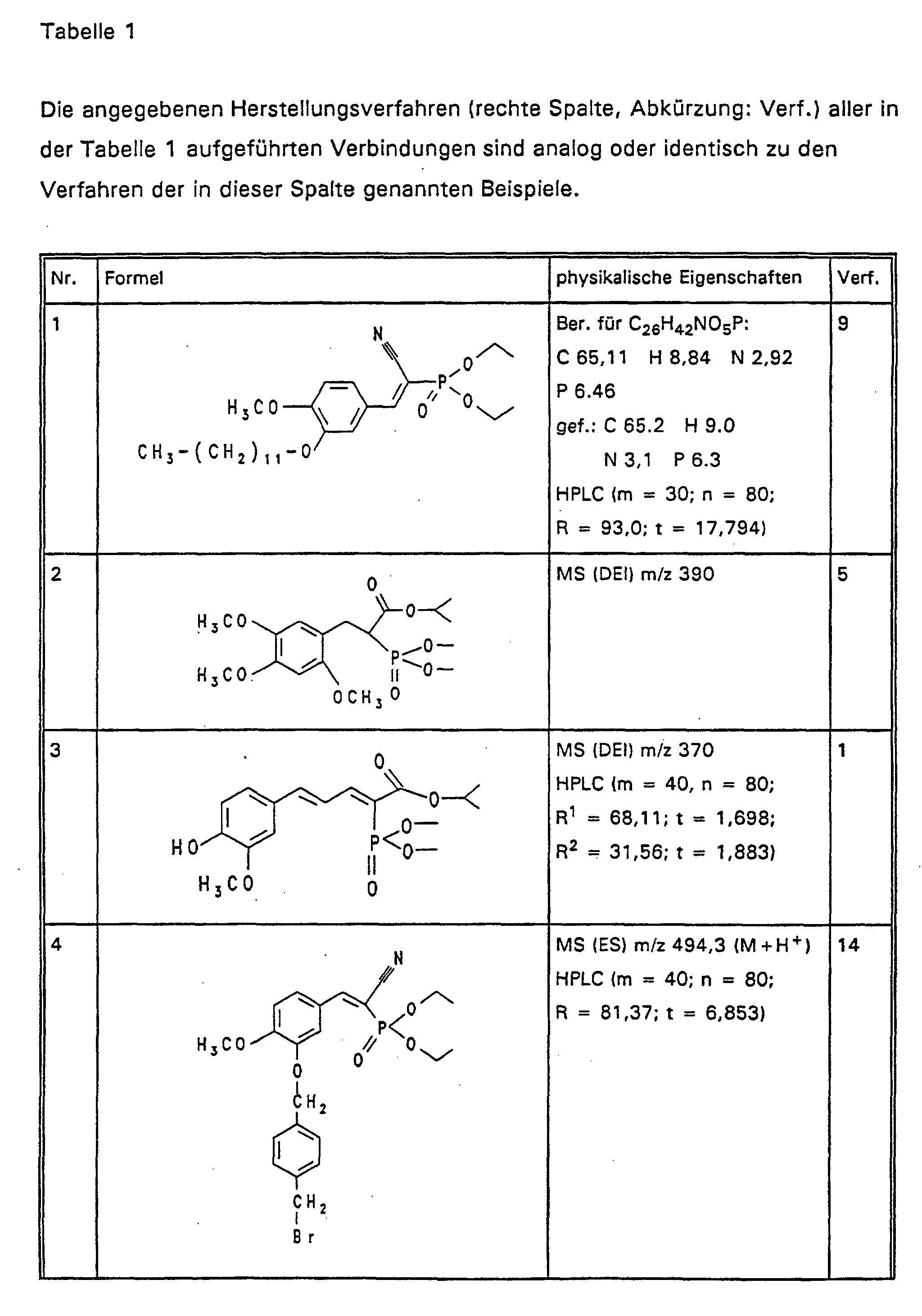

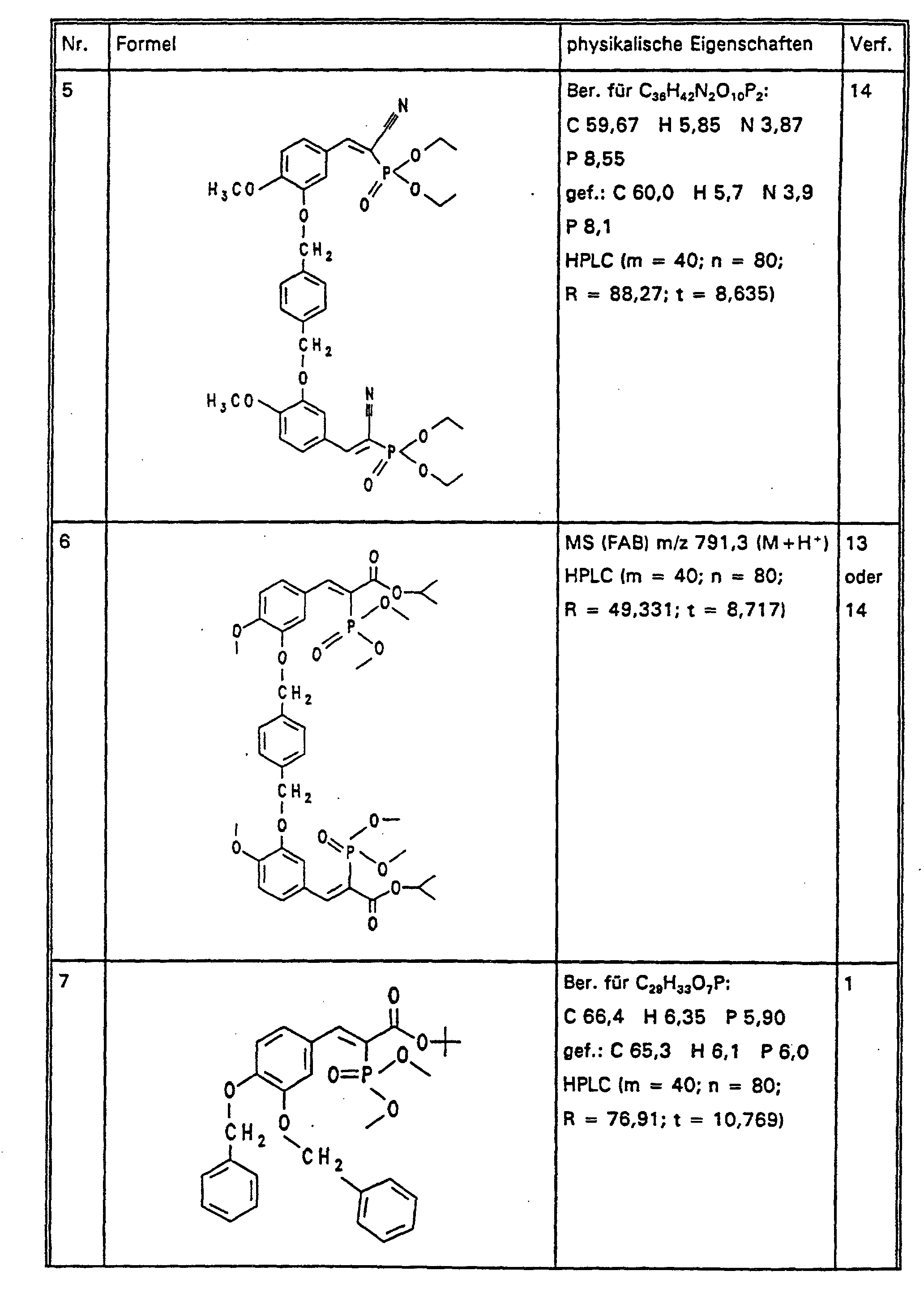

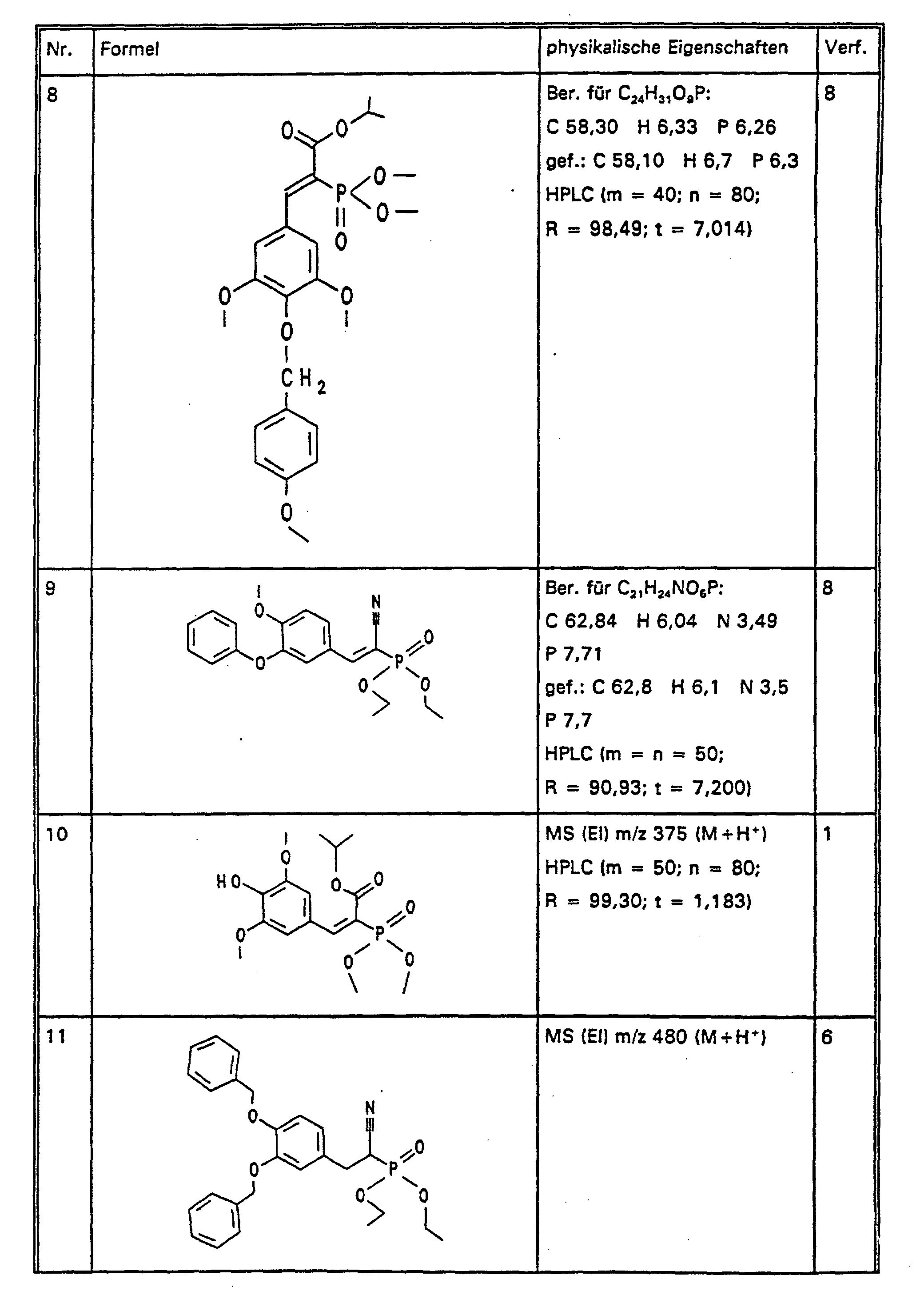

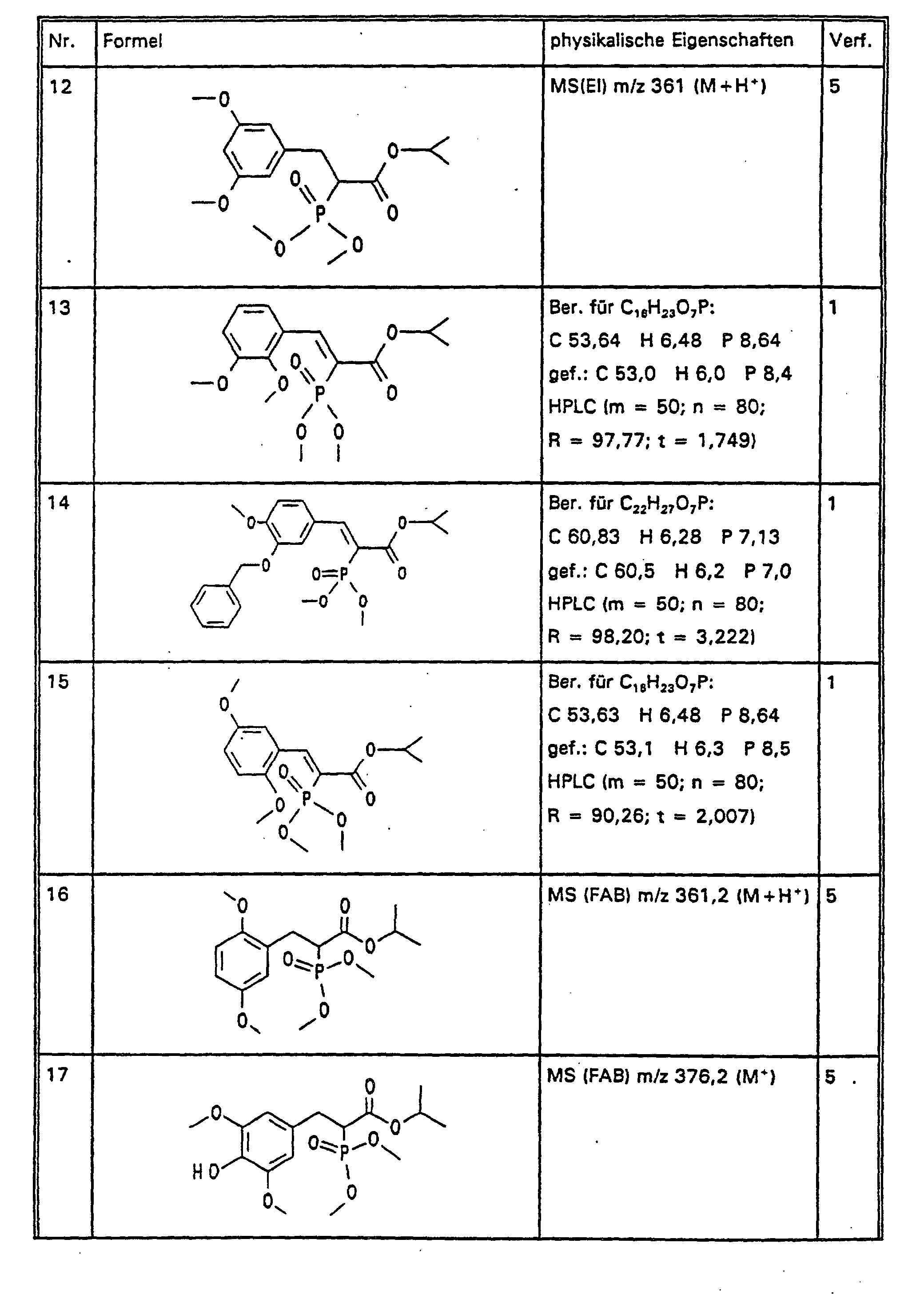

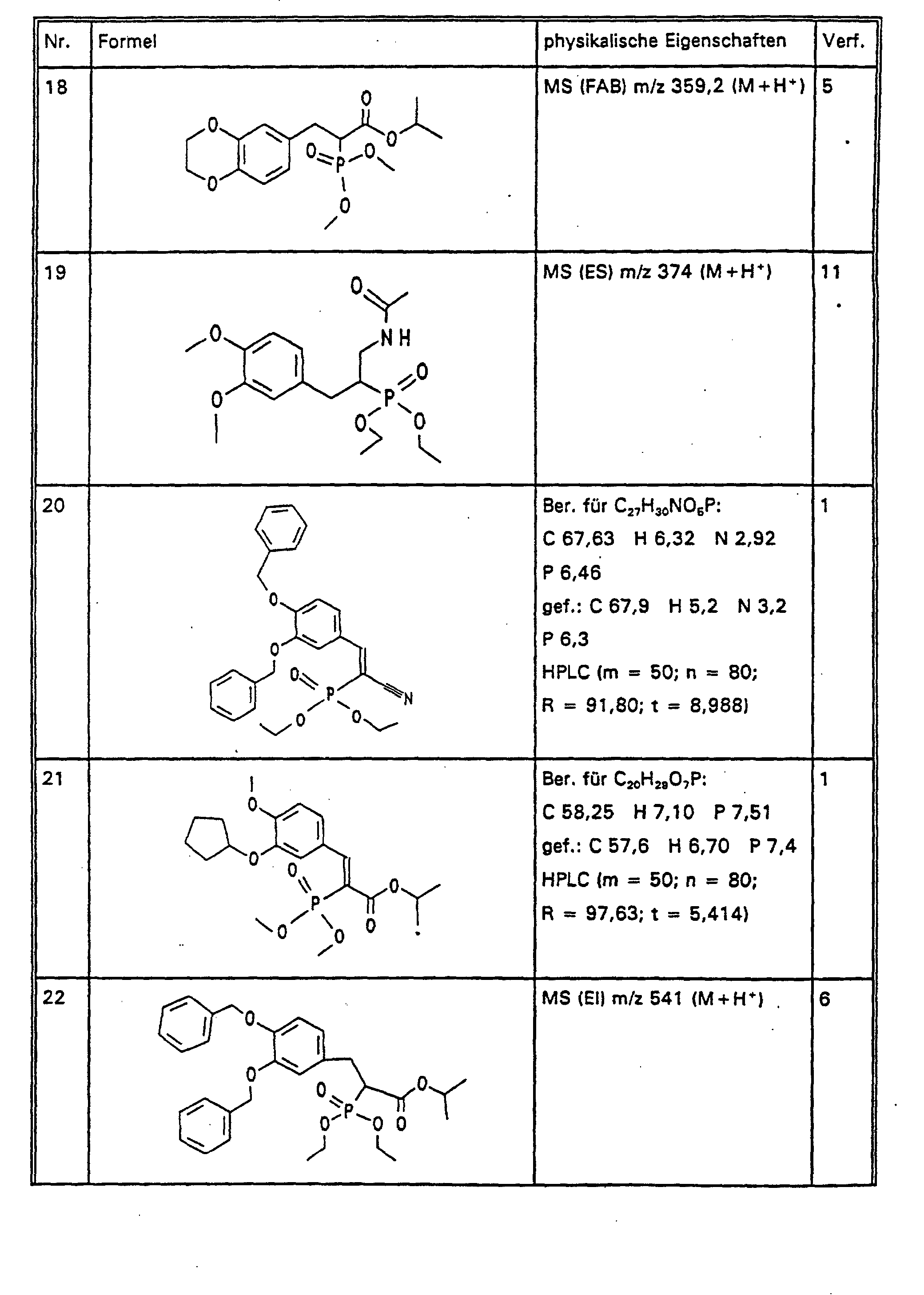

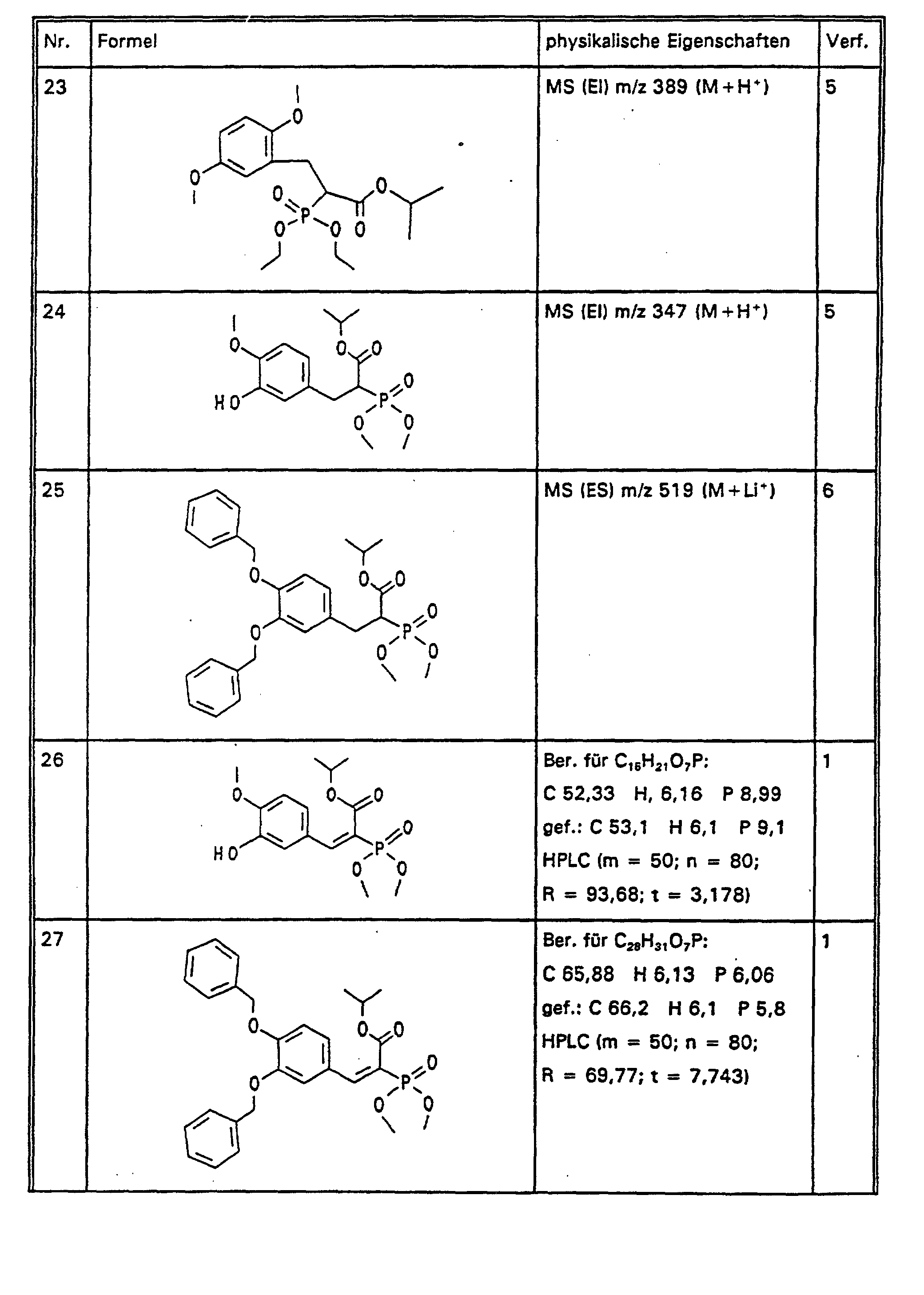

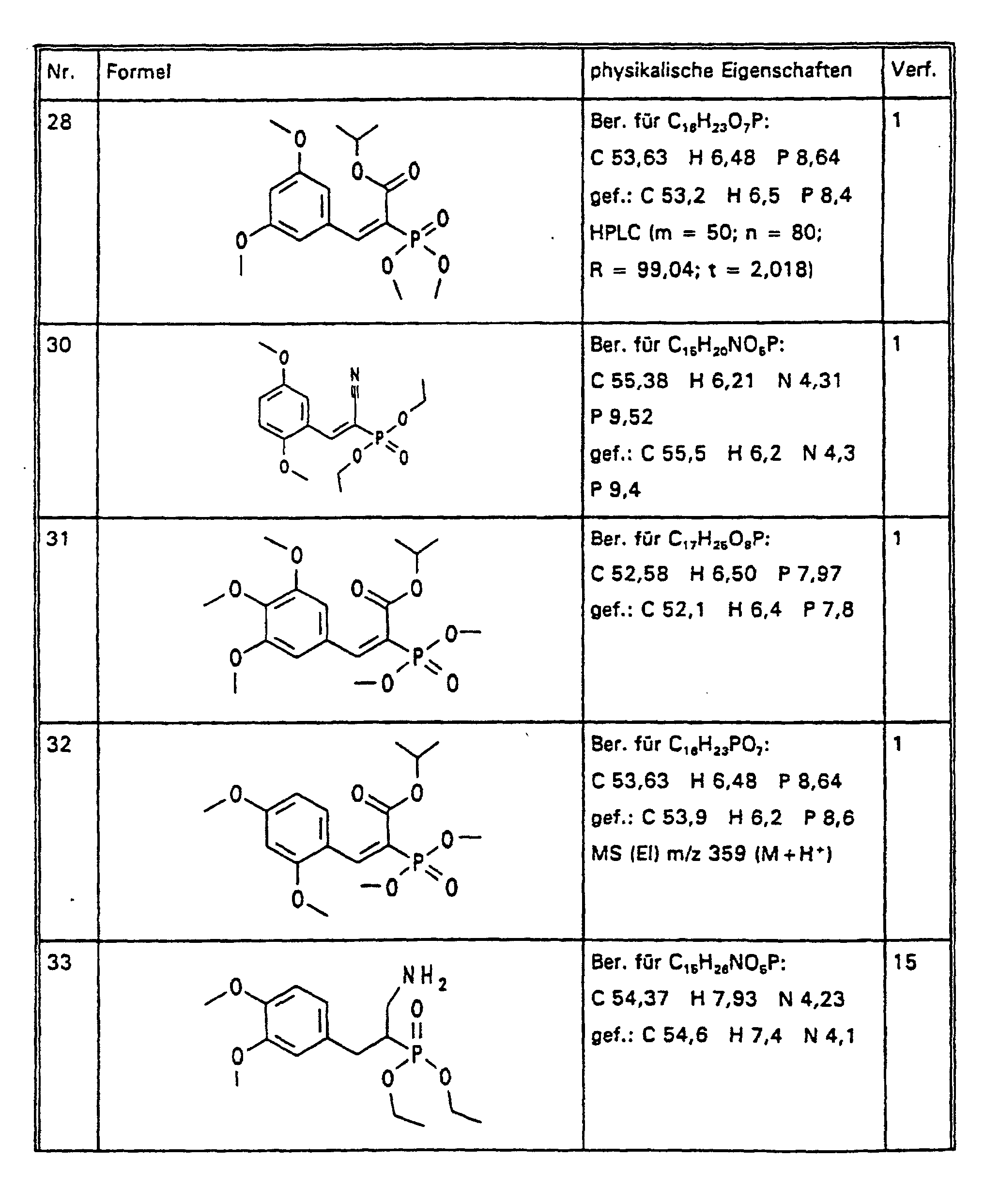

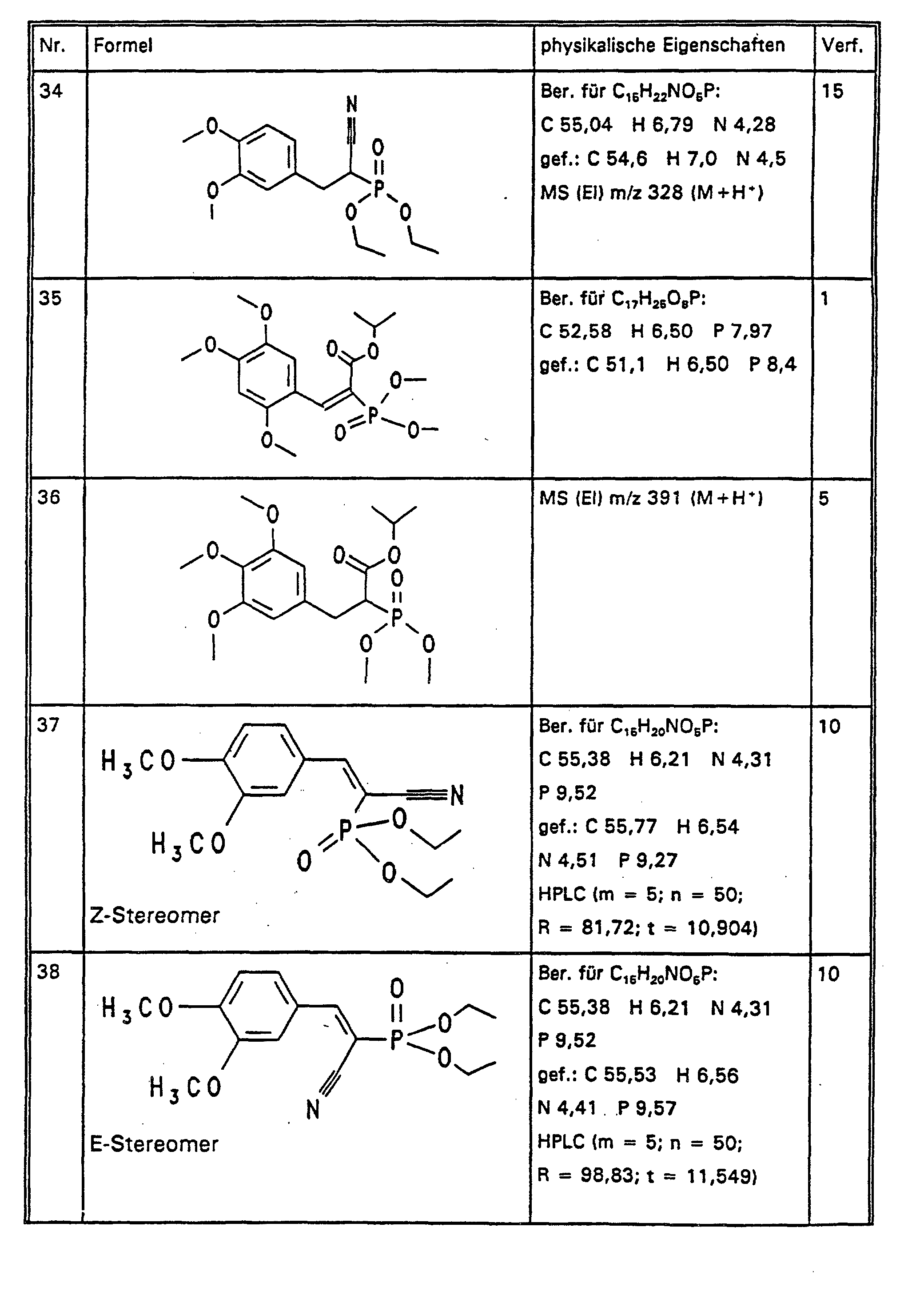

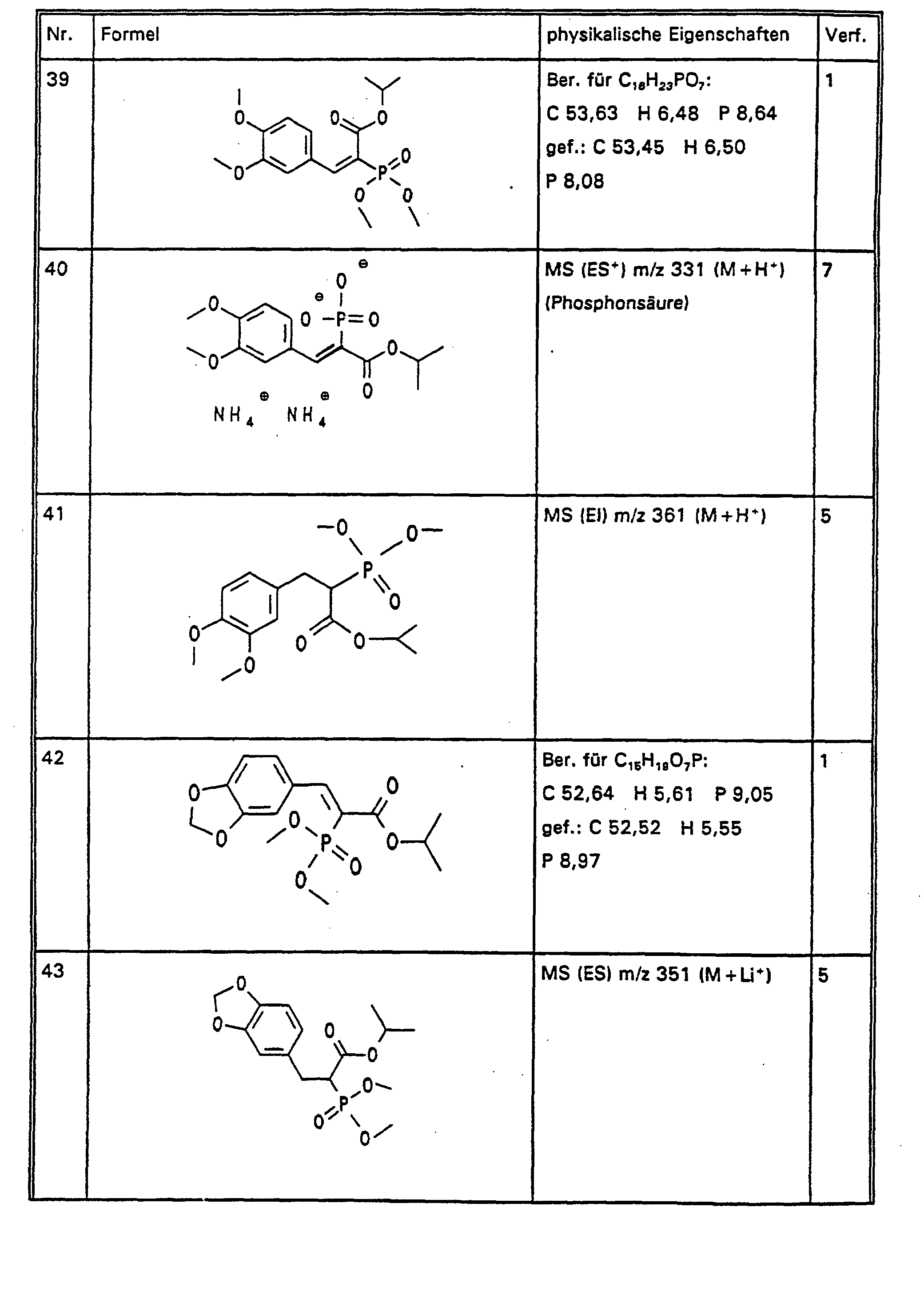

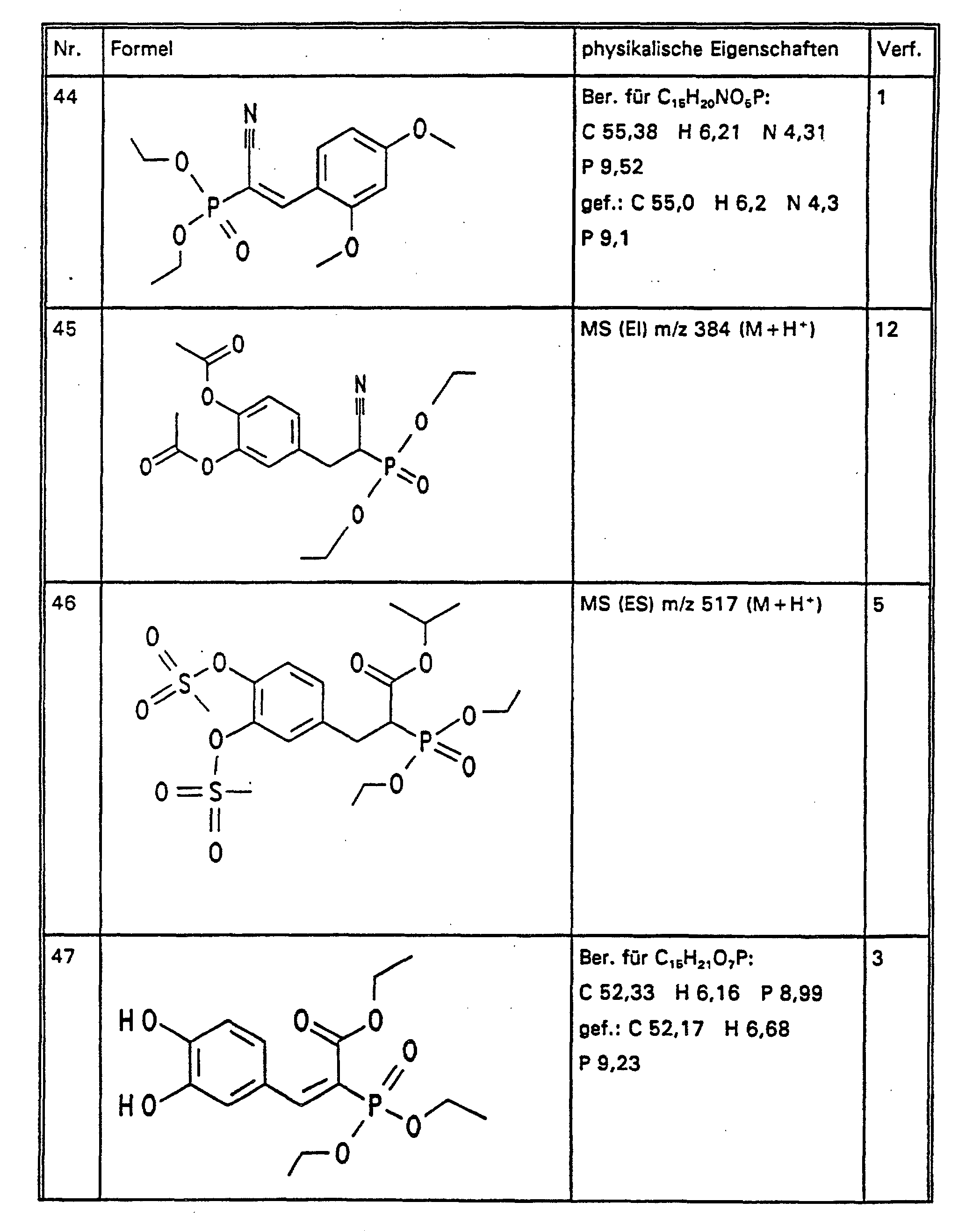

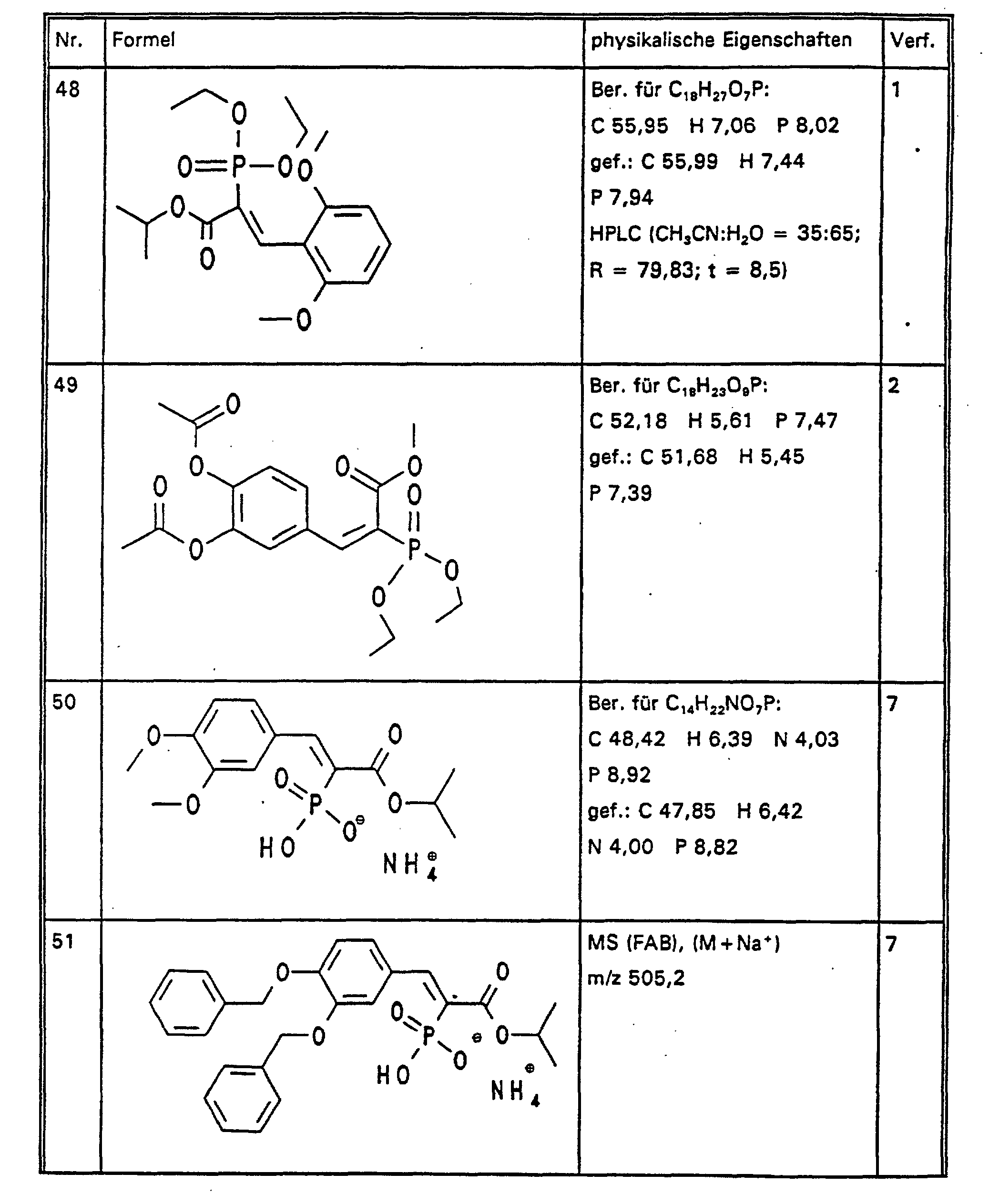

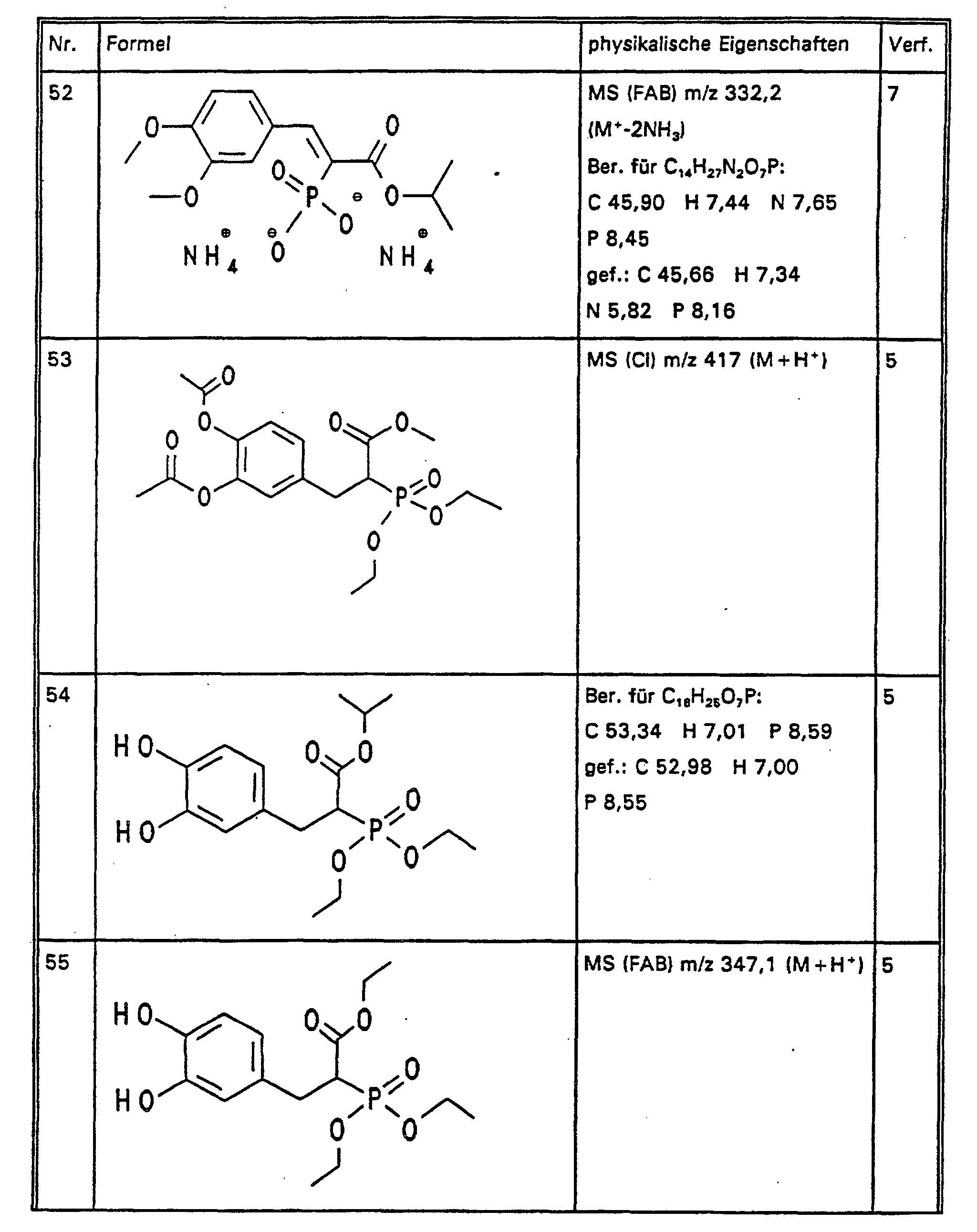

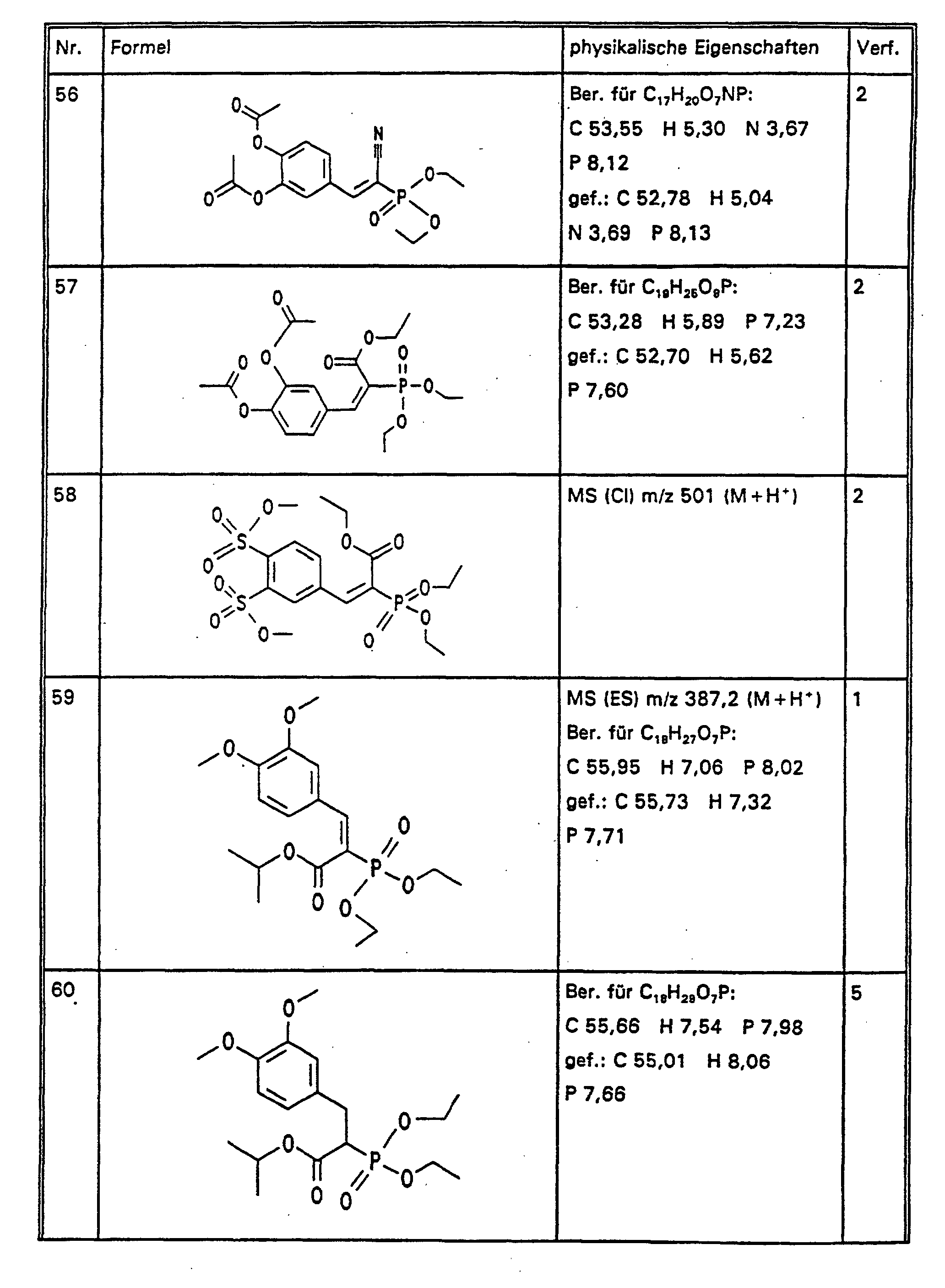

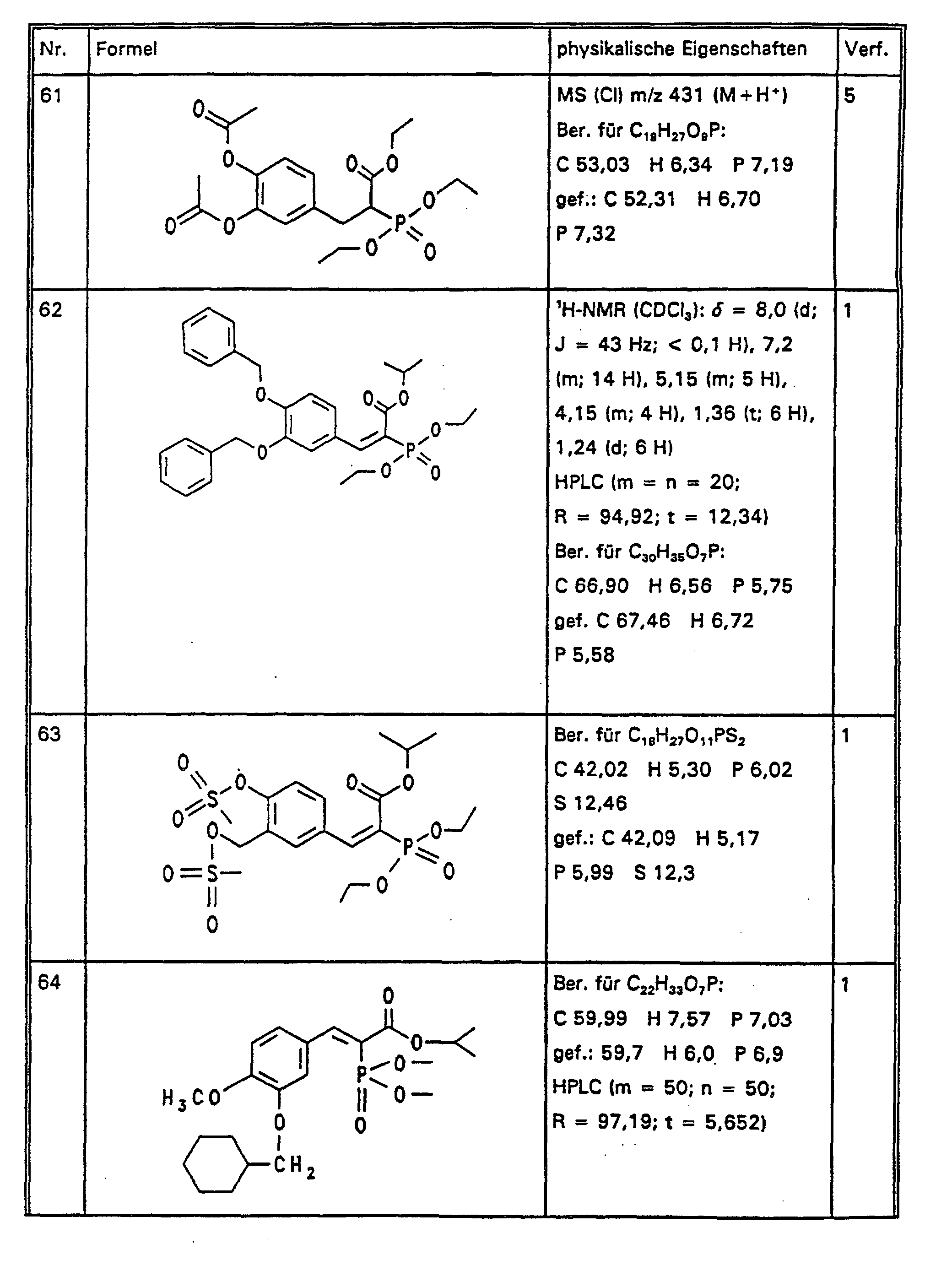

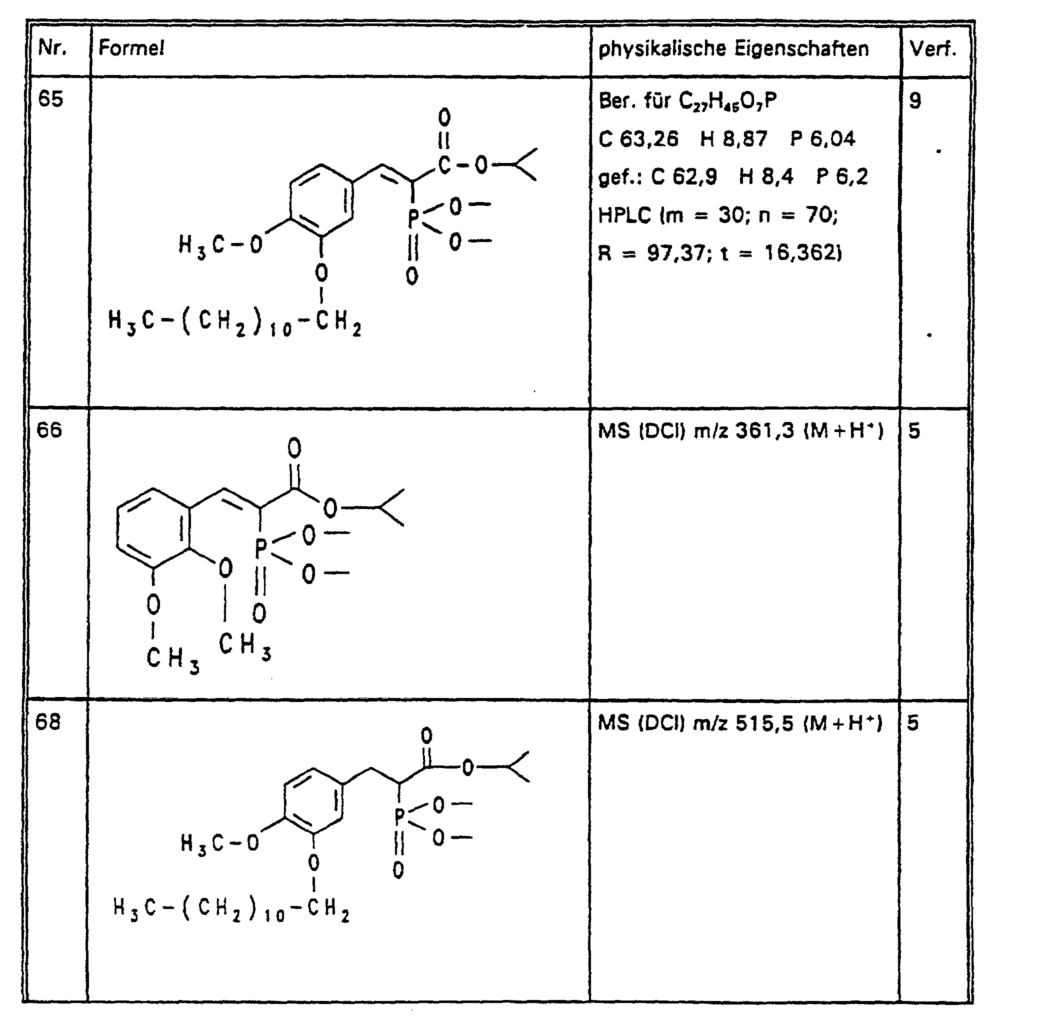

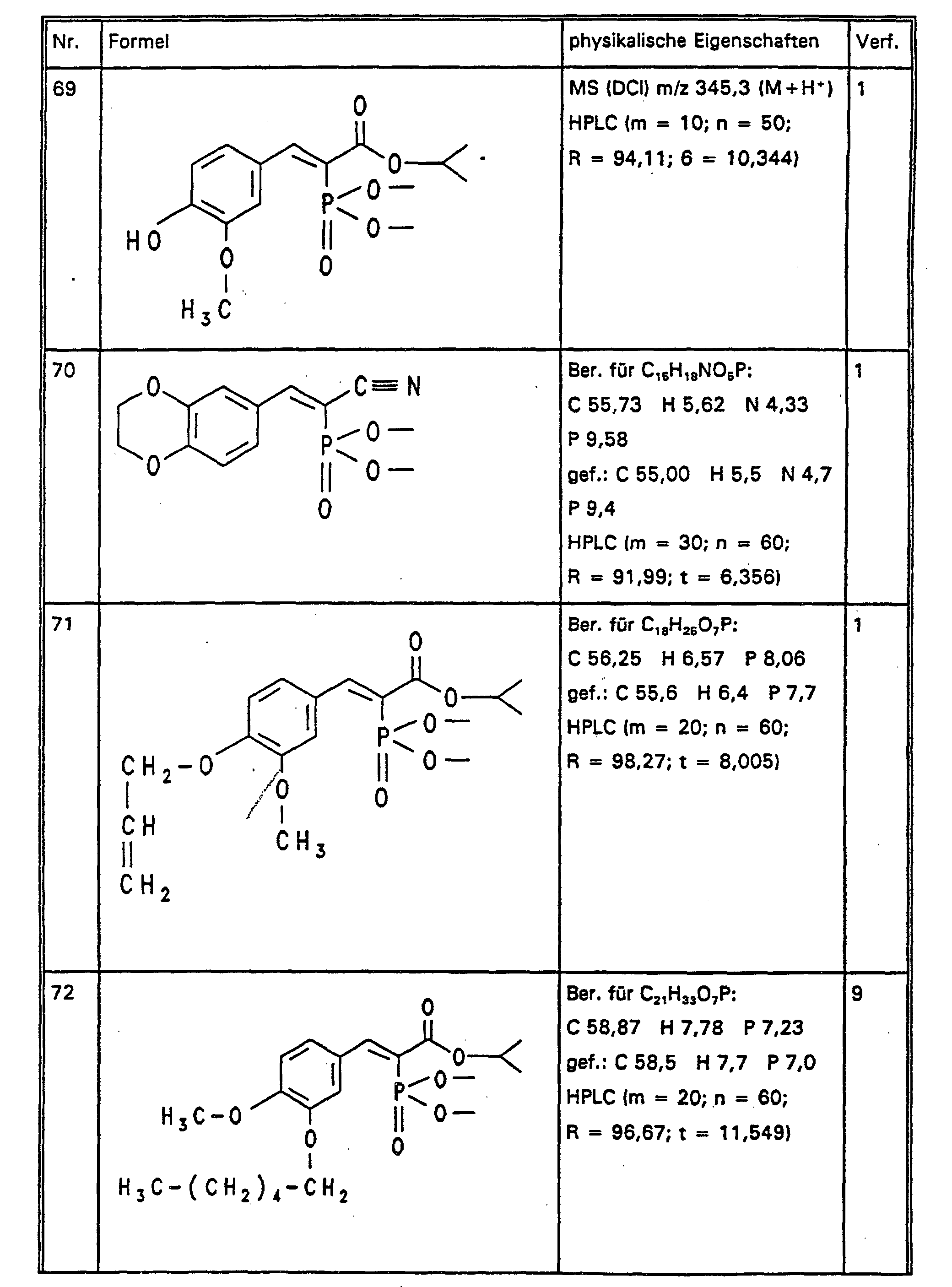

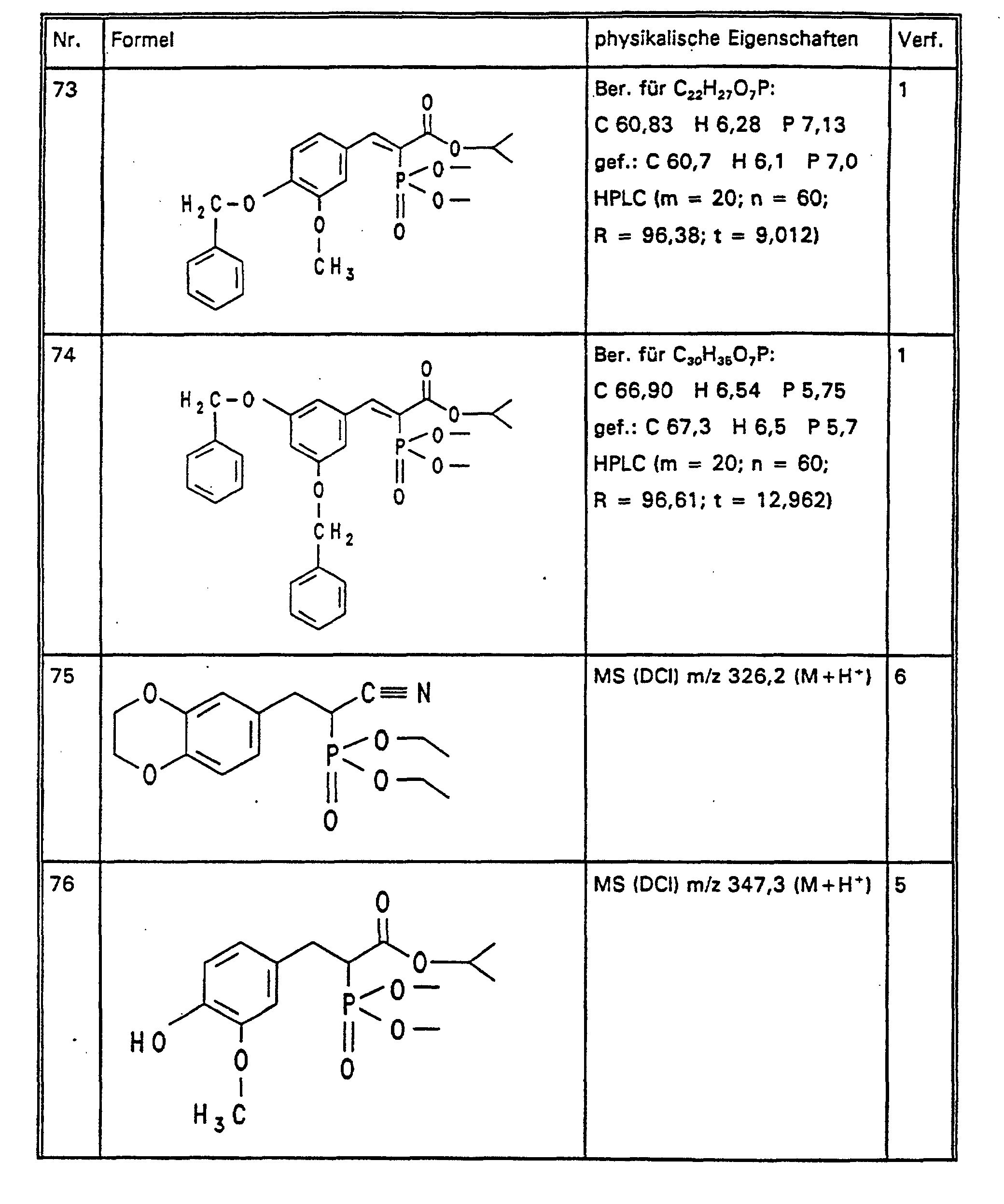

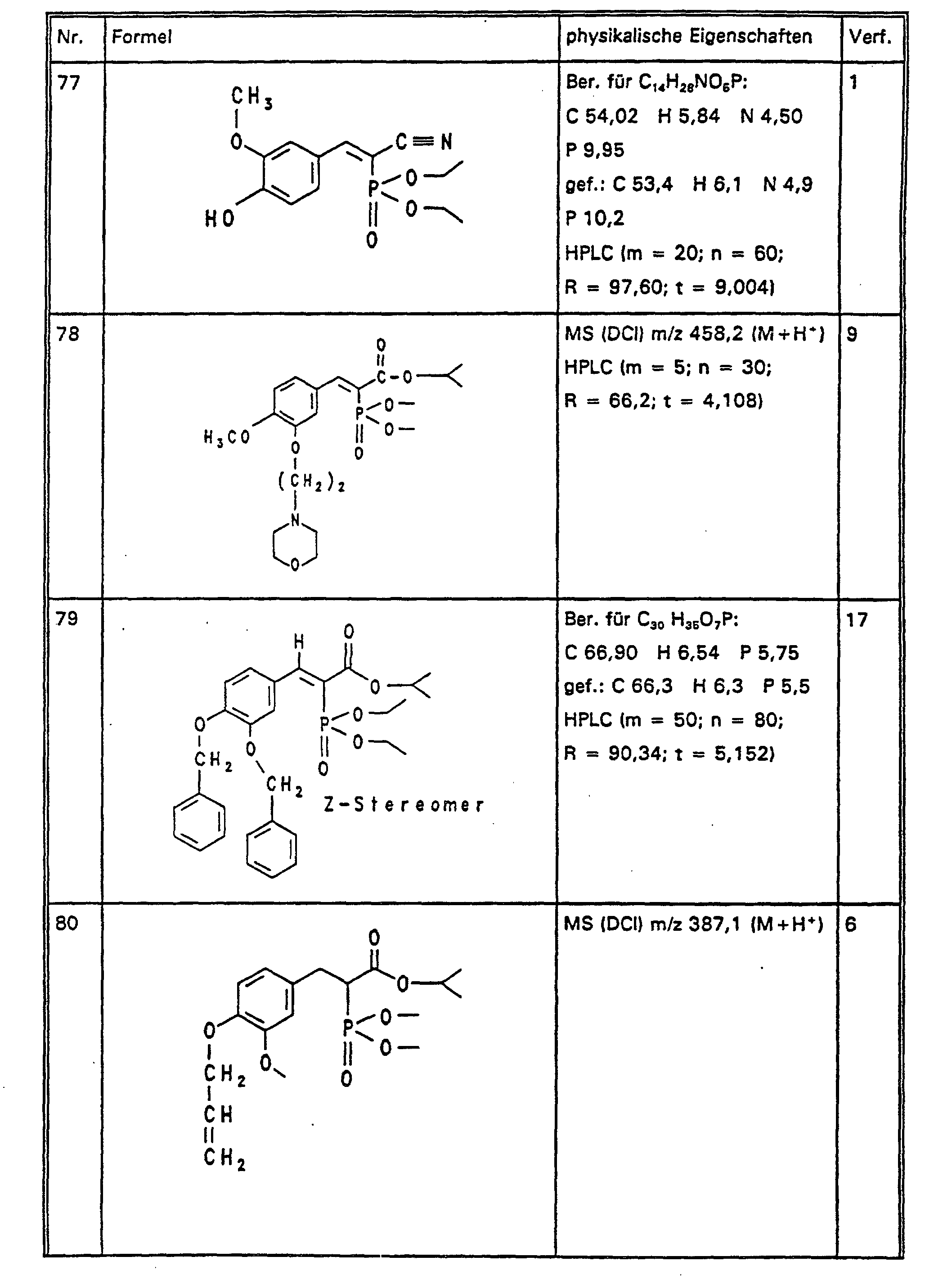

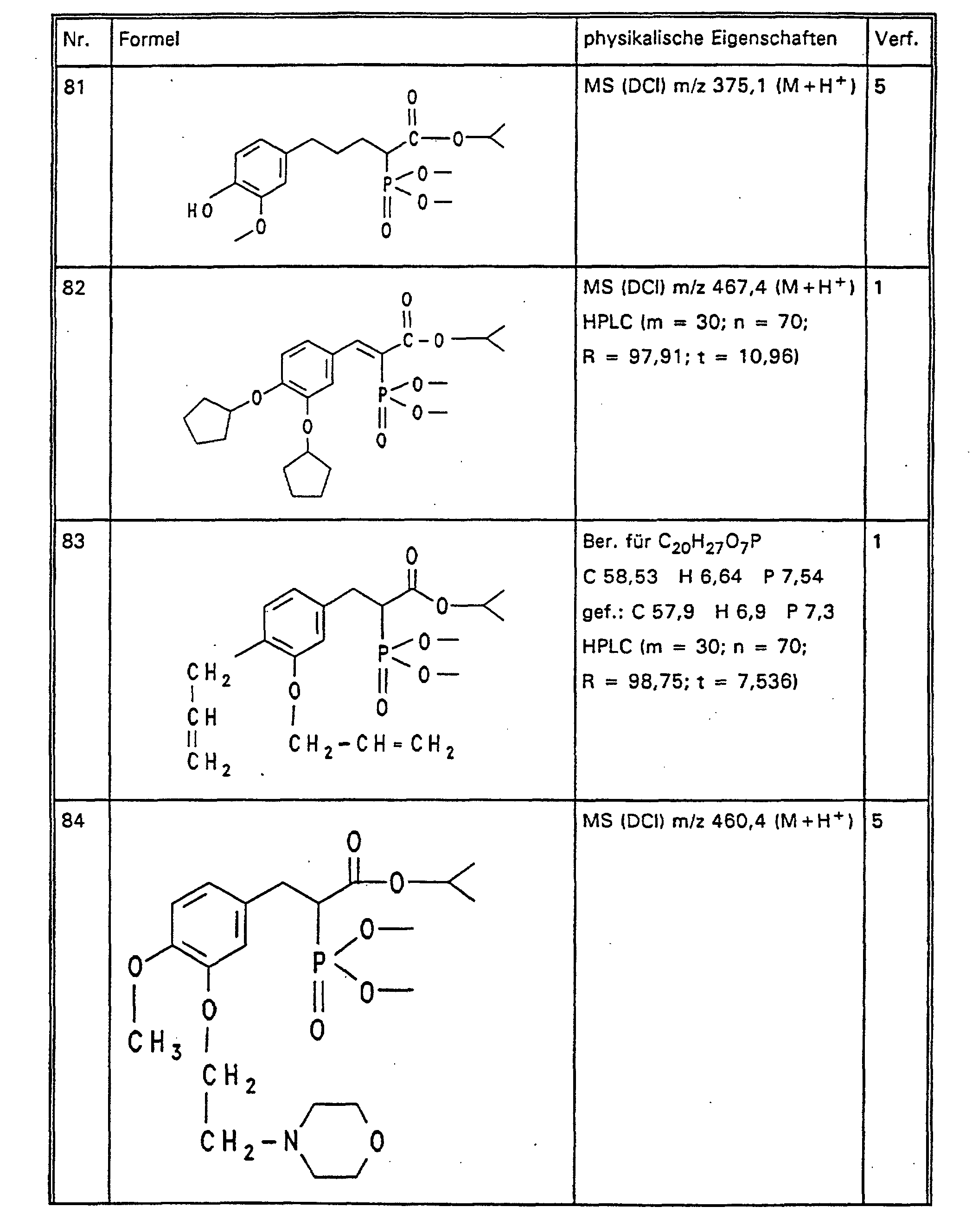

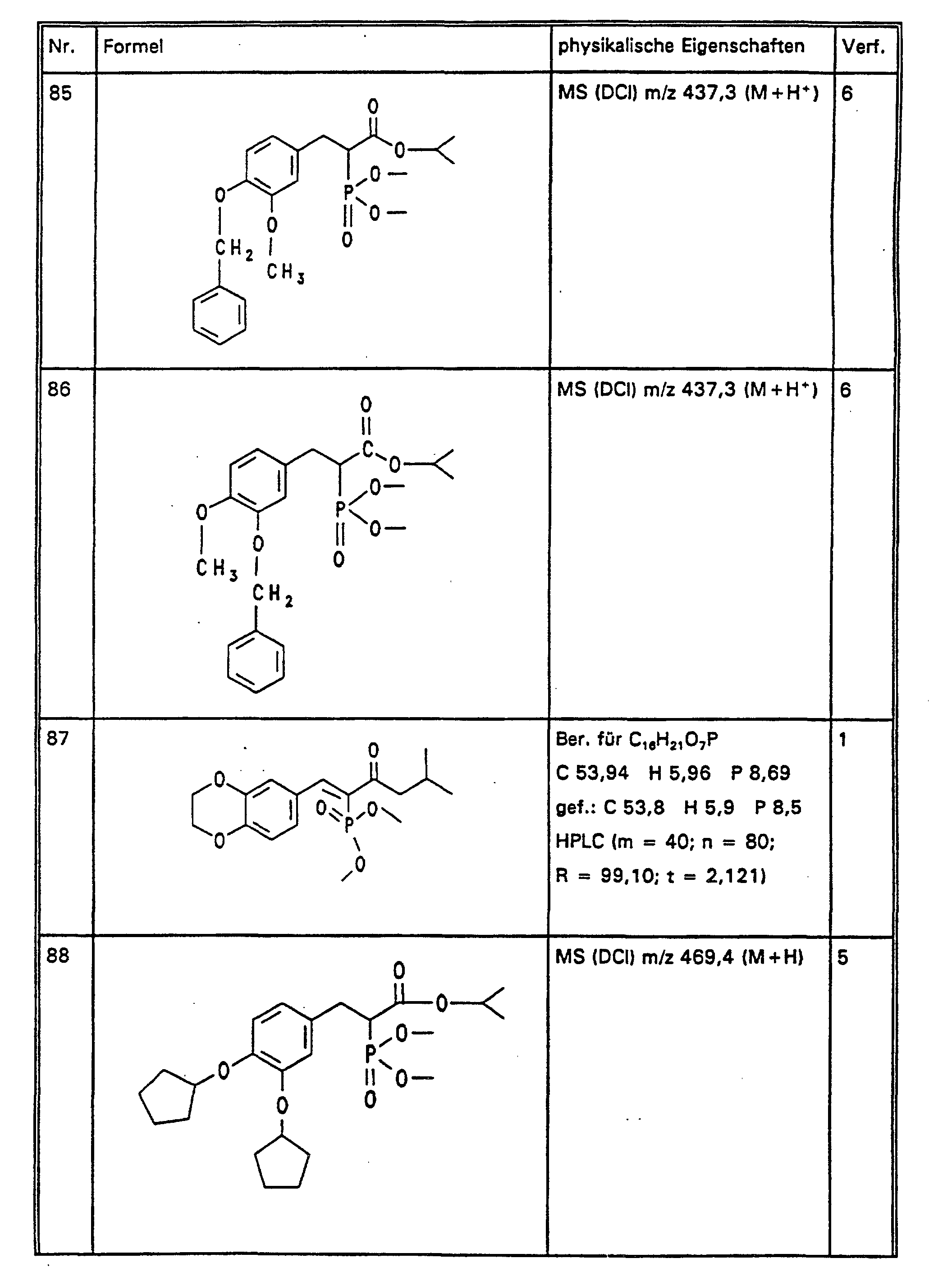

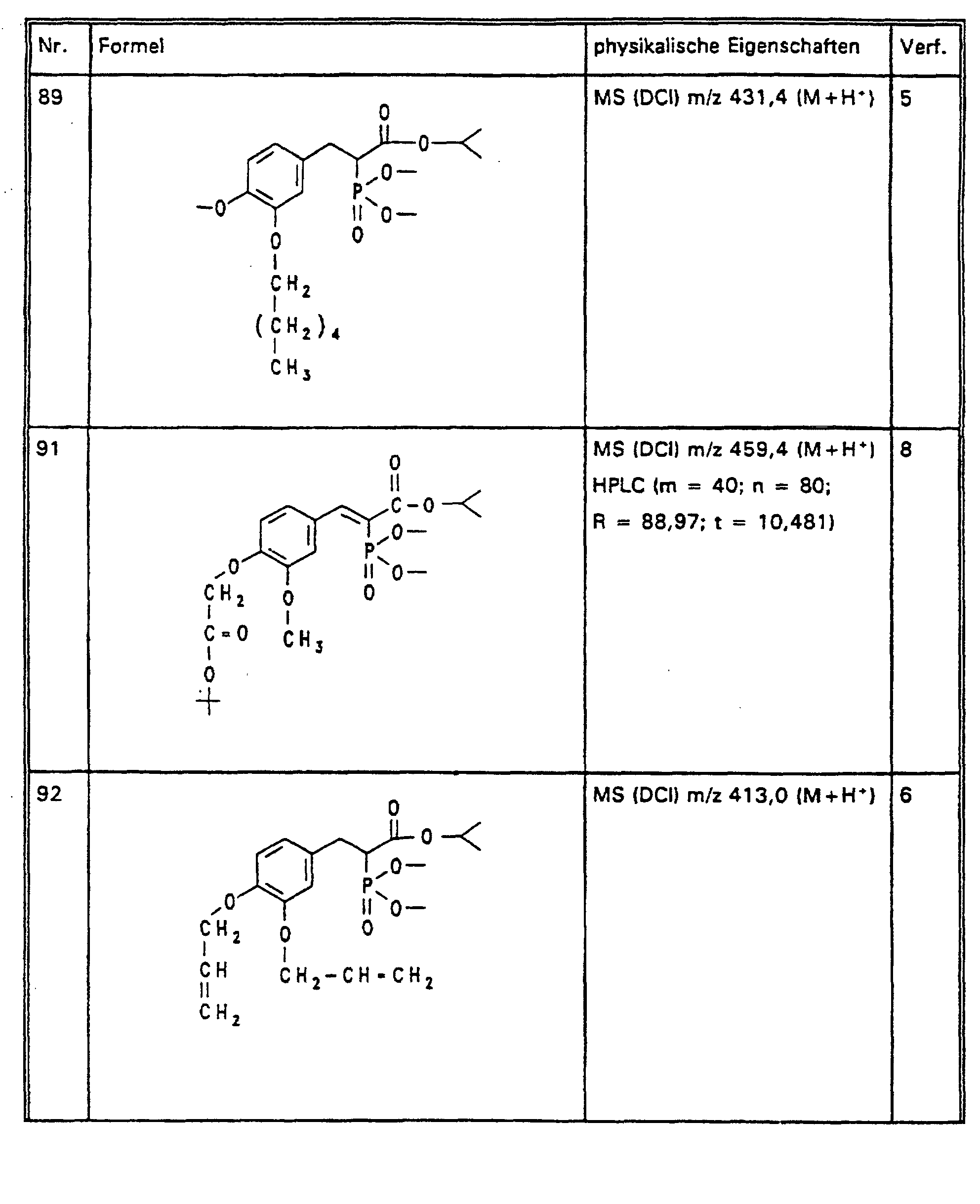

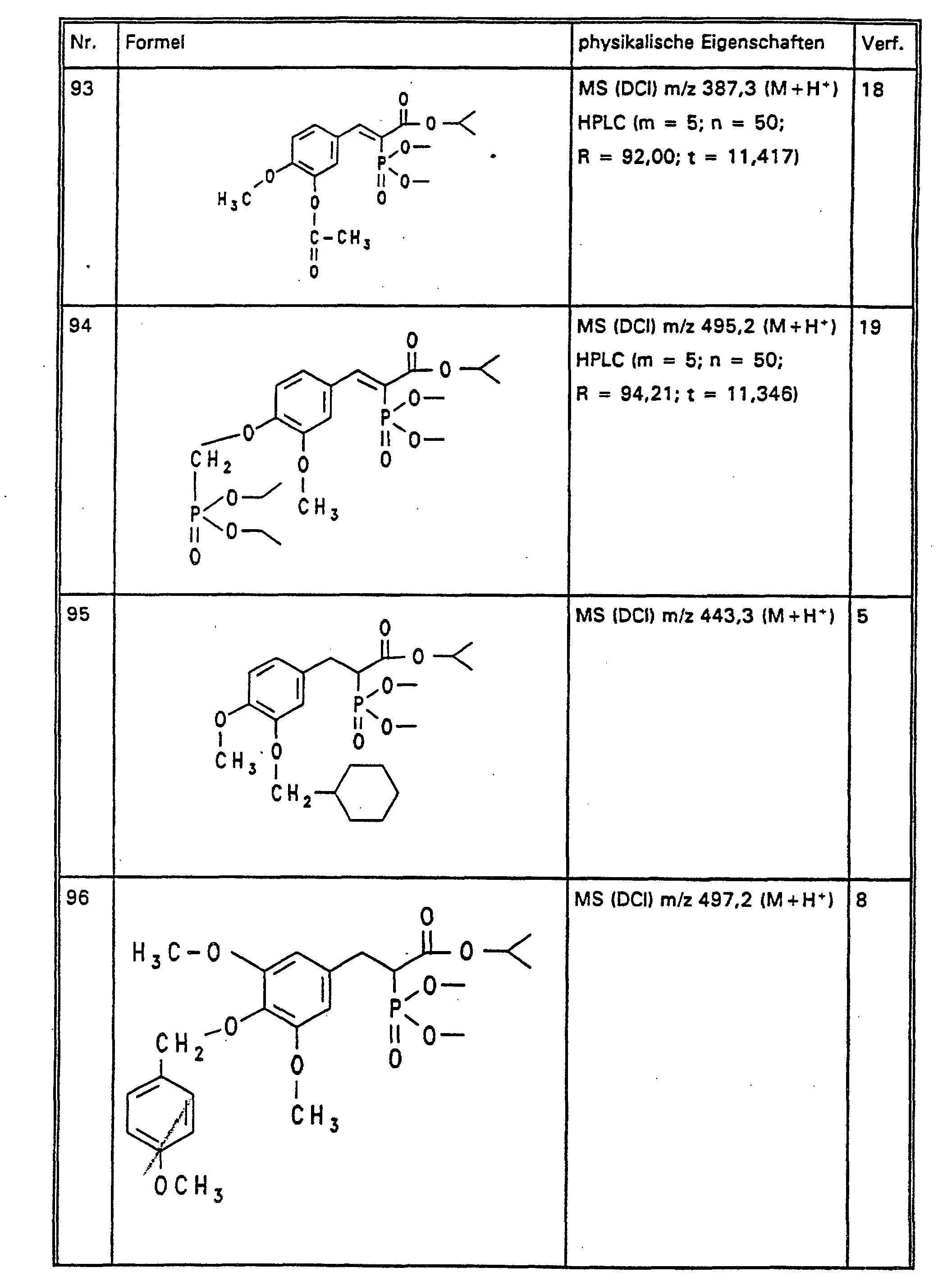

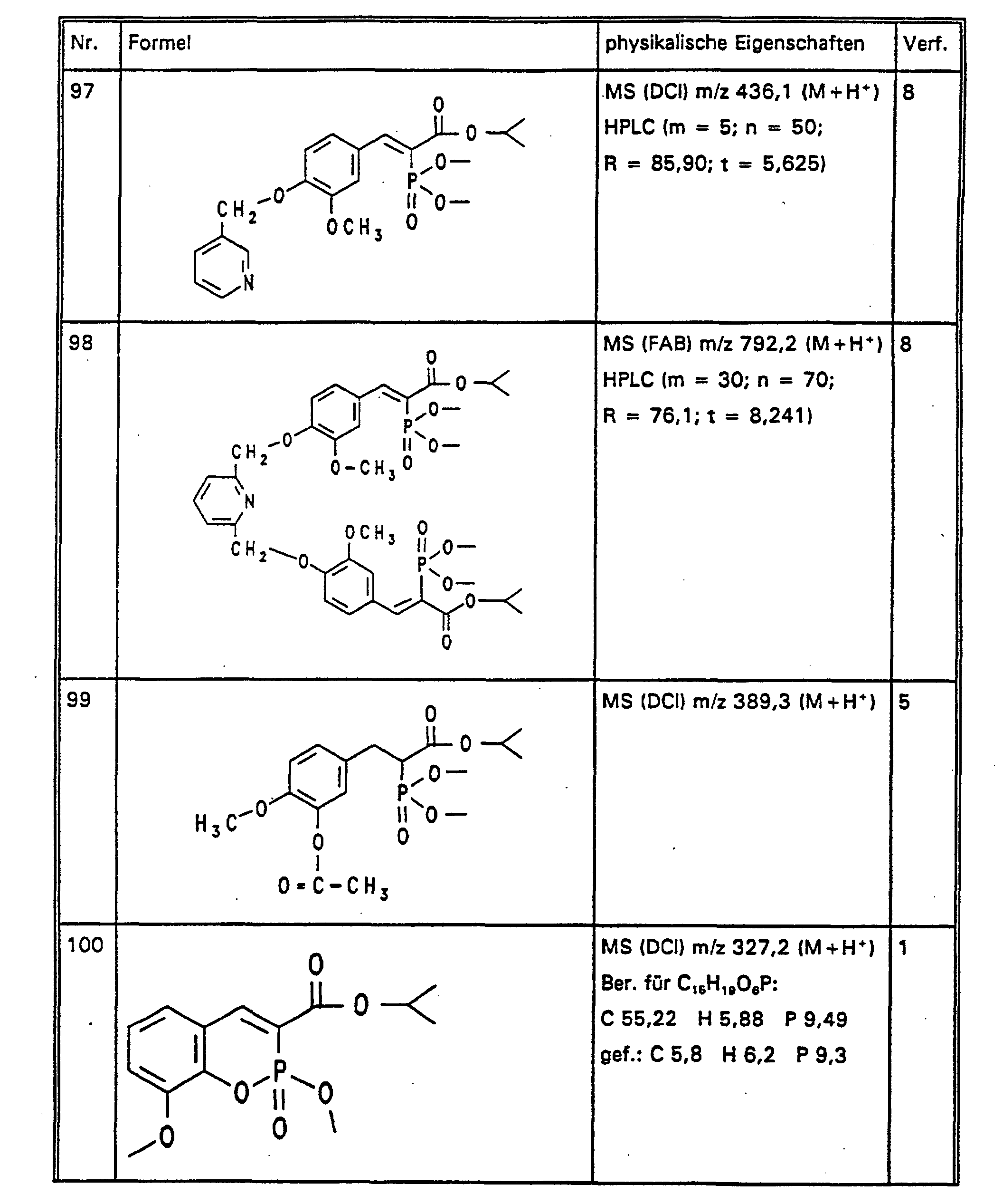

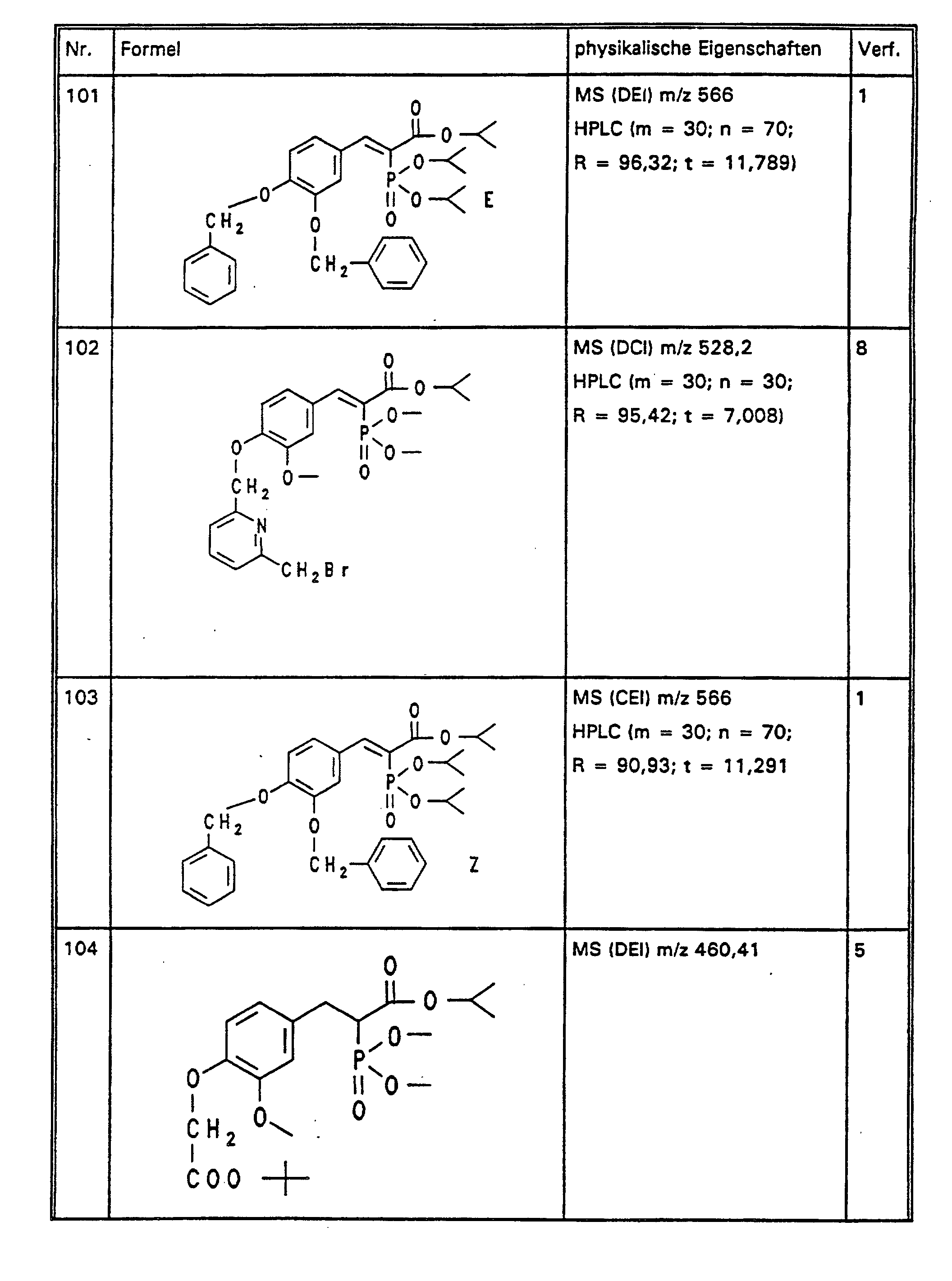

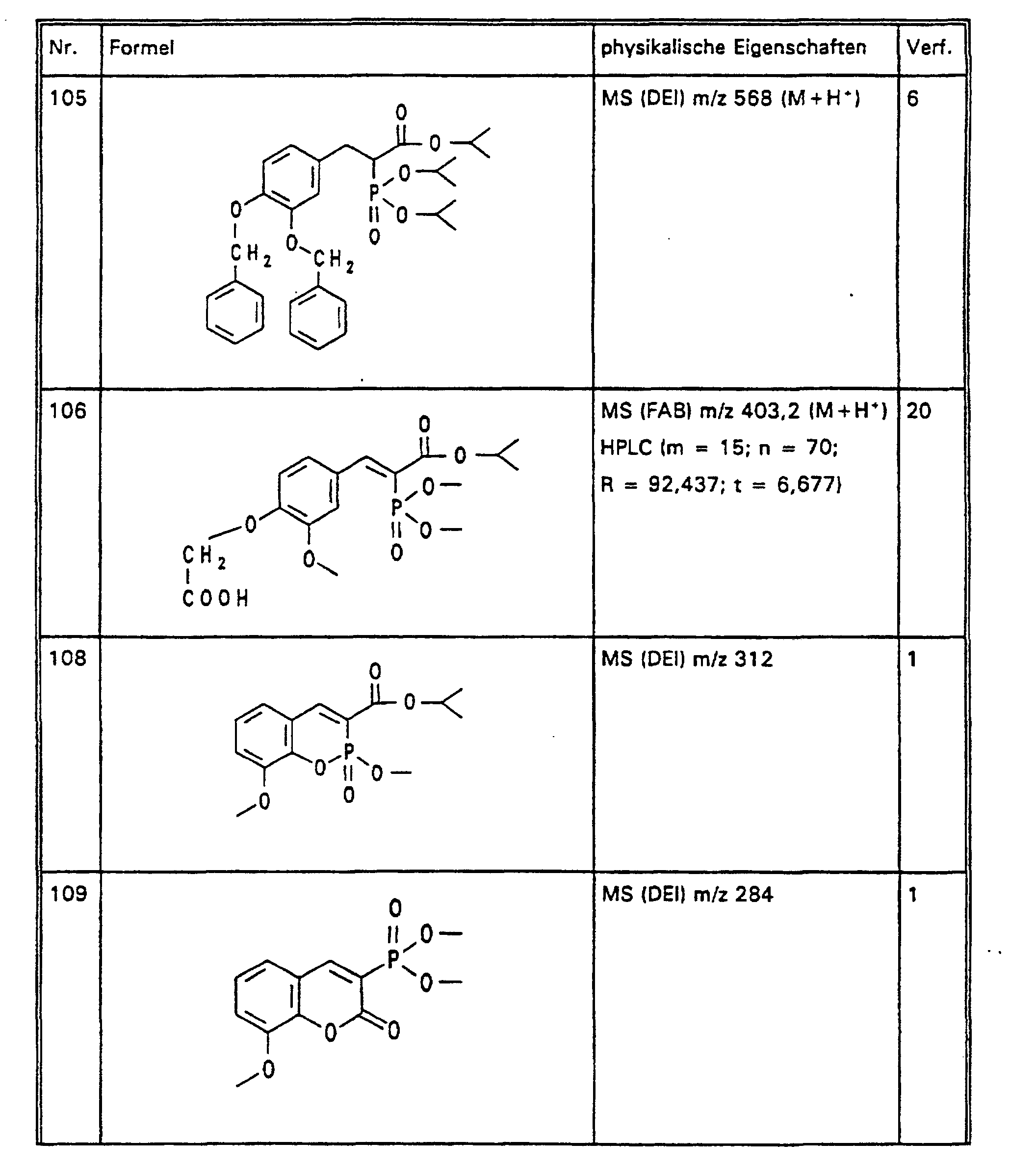

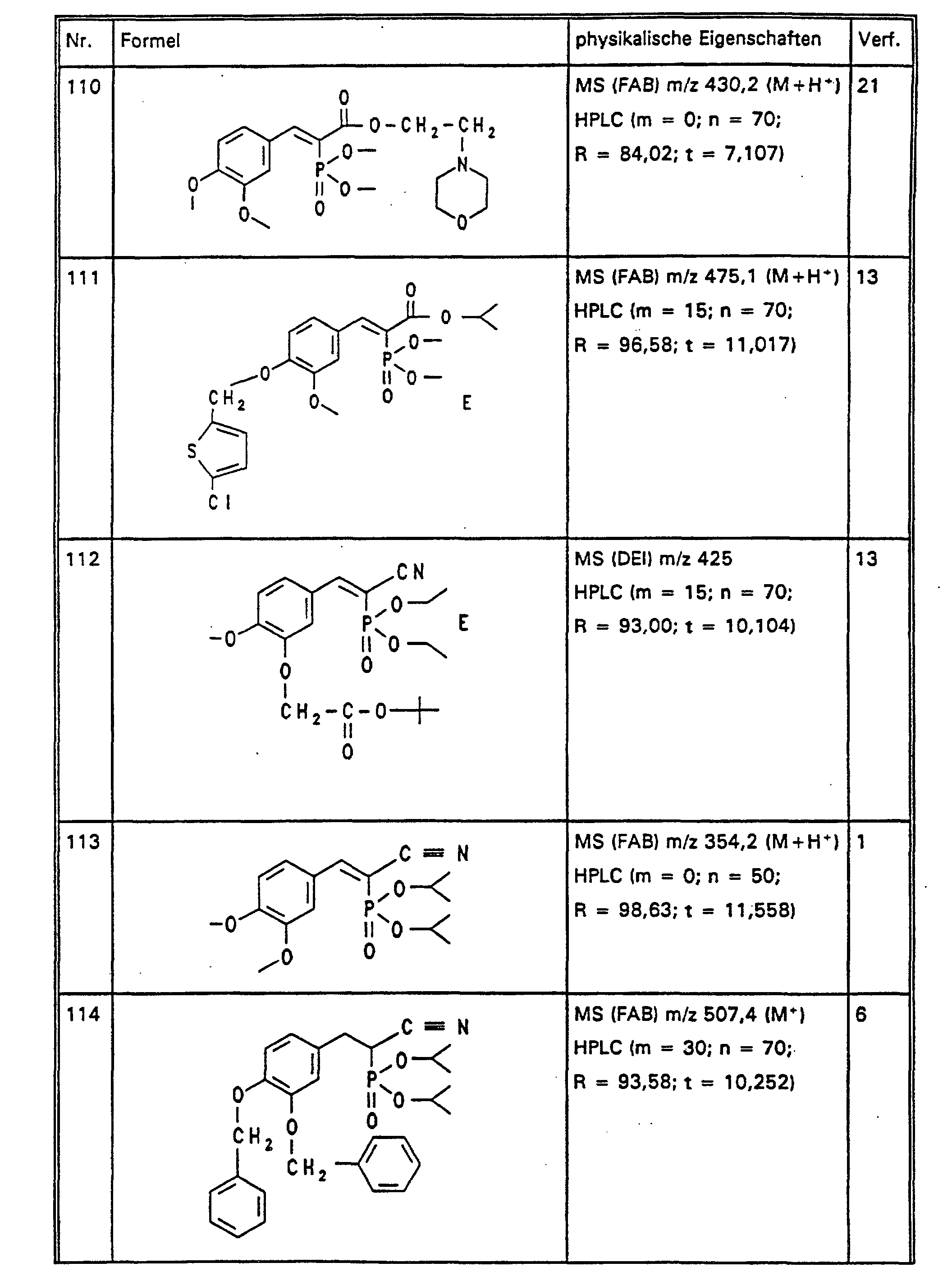

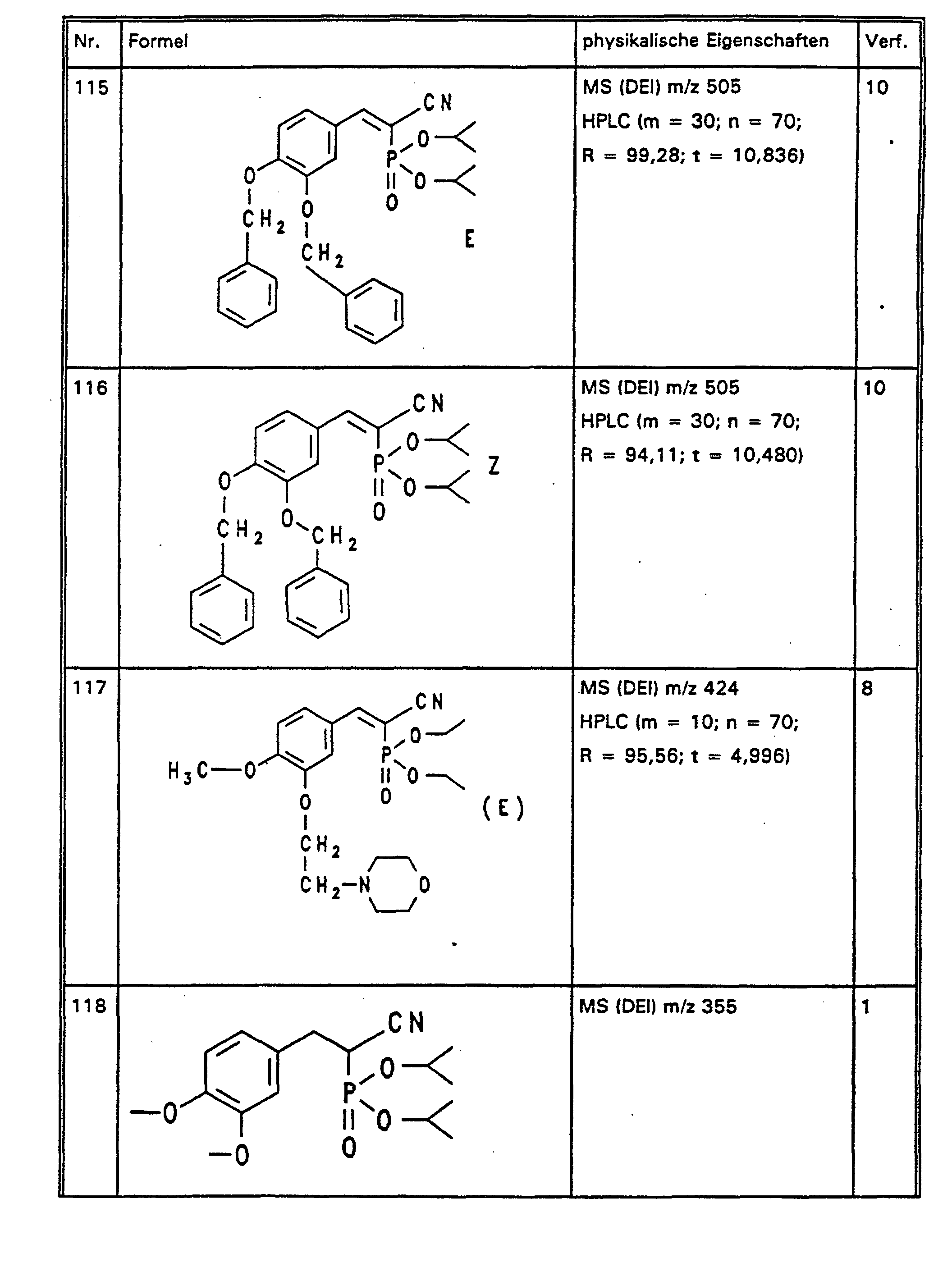

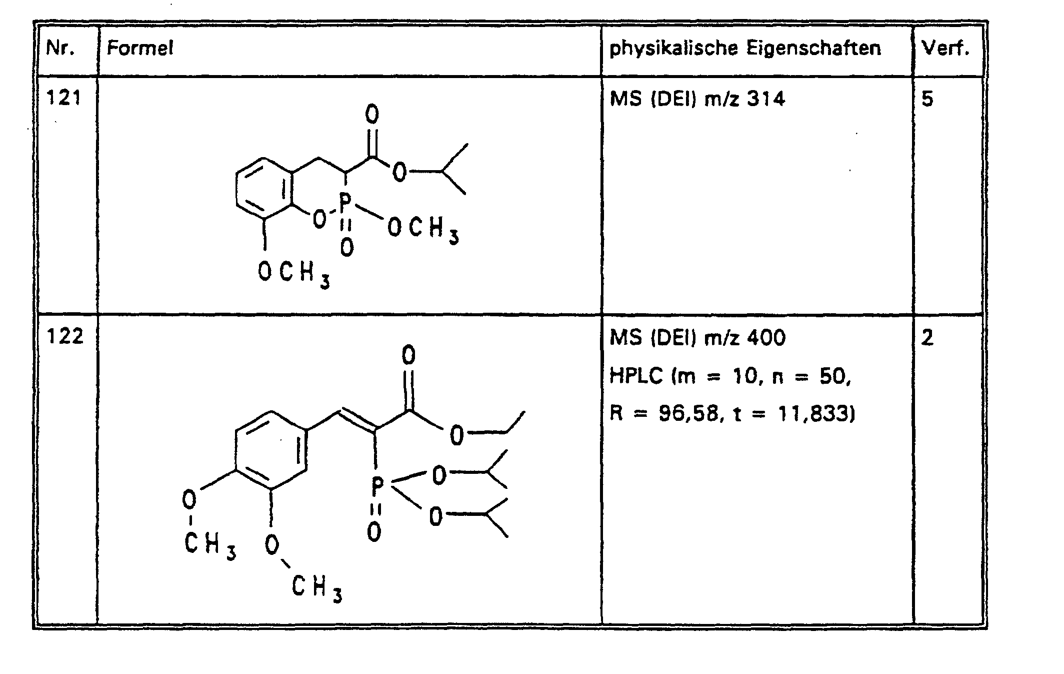

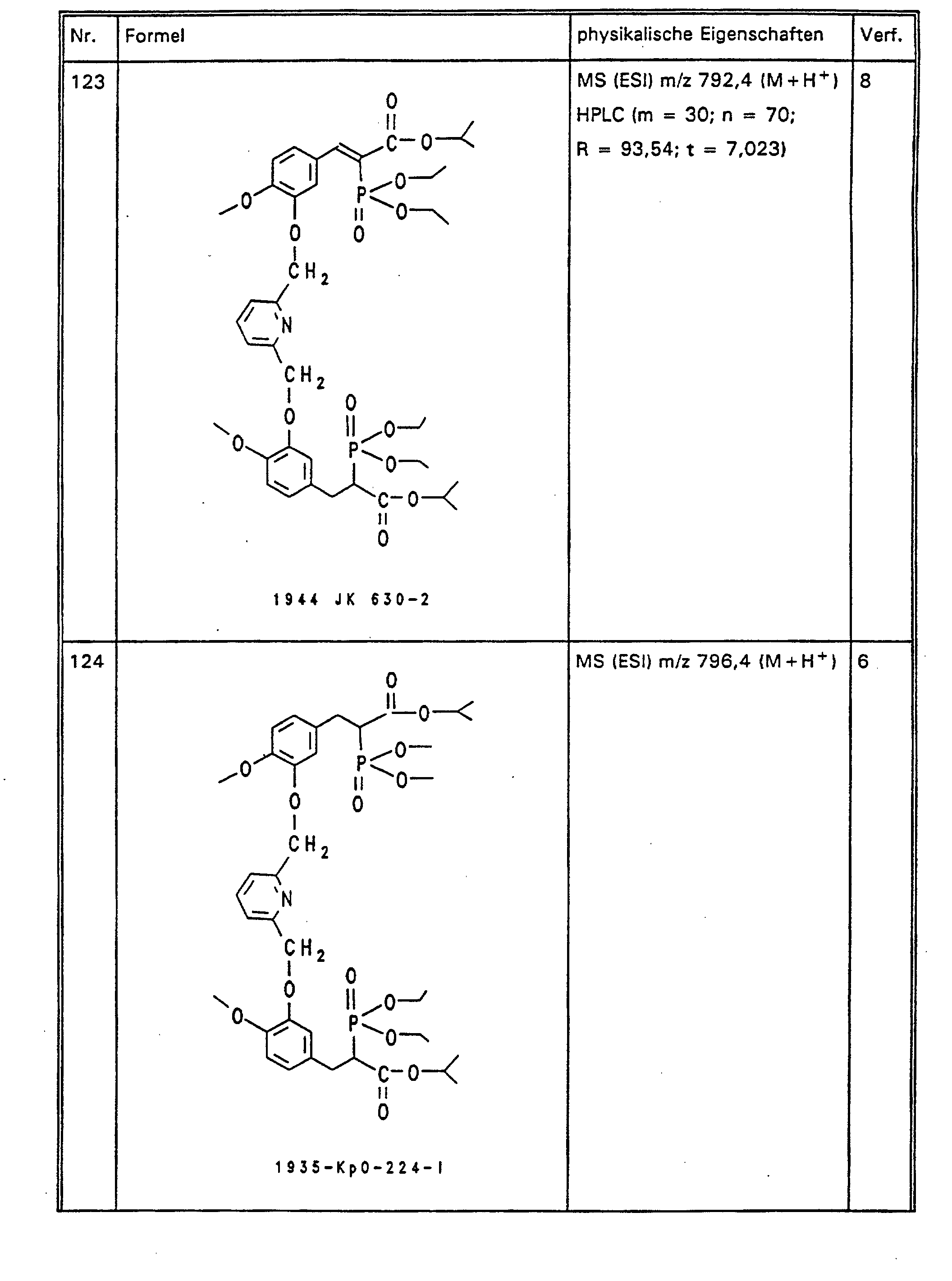

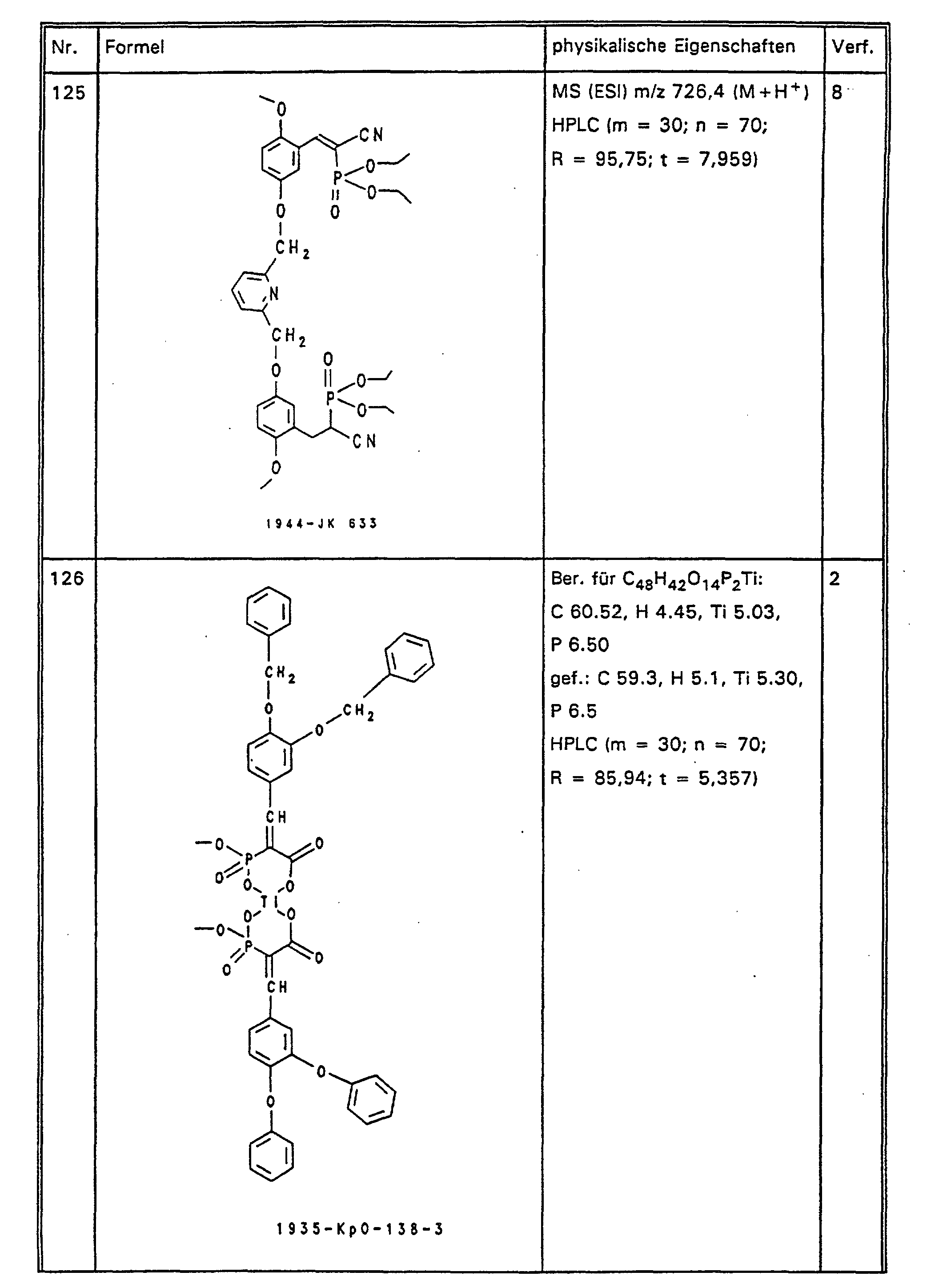

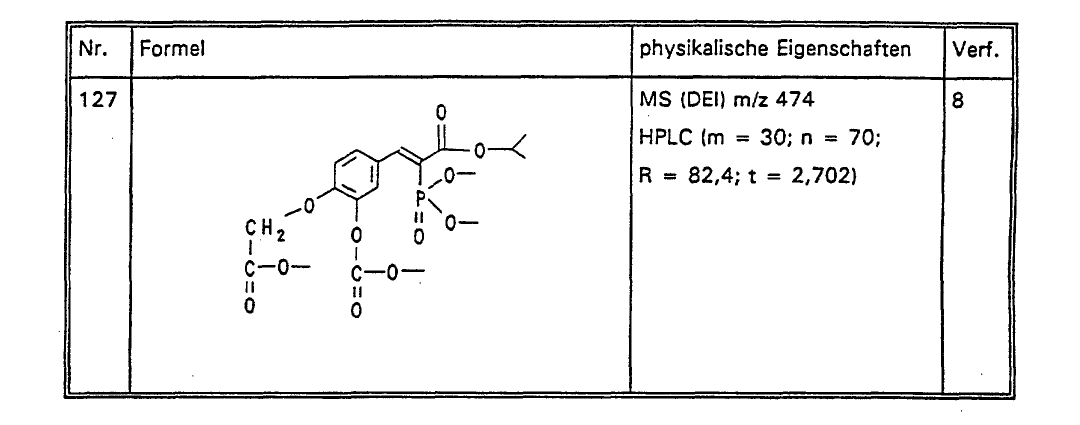

### Pharmakologische Prüfungen

### 1. Wirksamkeit in der Chondrolyse, Prüfung in der Chondrozyten-Kultur

Zellen: Den Fußgelenken frisch geschlachteter Rinder wird der hyaline Knorpel entnommen, mit Pronase (Boehringer Mannheim) und Collagenase (Sigma), die Matrix enzymatisch abgebaut, und die Chondrozyten in 1 prozentiger "low melting Agarose" in 24er Multiwell-Schalen in einer Zelldichte von 4 x 10⁶ pro well ausplattiert.

Medium: Komplettes Medium enthält HAM's F12 (Biochrom KG, Berlin) und 10 % fötales Kälberserum (Boehringer Mannheim), die Testsubstanz wird in Medium gelöst, üblicherweise in einer Konzentration von 10⁻⁵ M zugegeben und bei jedem Mediumwechsel erneut zugefügt.

Versuchsdurchführung: Die Behandlung erfolgt vom dritten bis zehnten Tag der Primärkultur, am 9. Tag wird 20 *µ*Ci/ml (7,4 x 10⁵ Bq) Na₂³⁵SO₄ für 24 h dem Medium zugefügt. Die Extraktion der Proteoglykane aus der Agarose-Schicht wird mit 8 M Guanidinium-Hydrochlorid und in Gegenwart von Proteinase-Inhibitoren (Sigma) unter Schütteln bei 4°C über 24 Stunden durchgeführt. Der Überstand wird nach Zentrifugation über eine PD 10 ® Sephadex G 25 Säule in freies und inkorporiertes Sulfat getrennt, dessen Aktivität nach Aliquotierung im β-Szintillationszähler gemessen wird.

Auswertung: Der Parameter für die Matrixproduktion der Chondrozyten ist die Menge synthetisierter Proteoglykane, gemessen als Sulfatinkorporation in Zerfälle pro Minute (cpm). Aus vier Wells pro Gruppe wird der Mittelwert errechnet. Dieser wird durch den Mittelwert der Interleukin I (IL-I) behandelten Kontrolle geteilt, und ergibt somit einen Stimulationsfaktor, der bei Stimulation der Matrixsynthese größer 1 ist, bei Hemmung derselben durch die Substanzwirkung kleiner 1, und bei unveränderter Matrixsynthese gleich 1 ist. Als Standard wurde Diacerein eingesetzt, das unter dem Handelsnamen Artrodar in Italien als Arthrosemittel eingesetzt wird.
Ergebnisse: Die Ergebnisse sind in Tabelle 2 aufgeführt.

**Tabelle 2:**

| Einfluß auf die IL-1-induzierte Chondrolyse in der Agarose-Kultur | |
|---|---|
| Beispiel Nr. Standard (Diacerein) | Stimulationsfaktor der Proteoglykan-Synthese 1,1 |
| 10 | 3,5 |
| 12 | 4,1 |
| 13 | 3,3 |
| 14 | 3,8 |
| 15 | 3,4 |
| 21 | 1,3 |
| 24 | 1,6 |
| 25 | 2,1 |
| 26 | 2,1 |
| 27 | 1,9 |
| 28 | 3,0 |
| 35 | 1,3 |
| 39 | 1,2 |
| 40 | 1,2 |
| 41 | 1,2 |
| 47 | 2,4 |
| 49 | 1,4 |
| 50 | 1,4 |
| 51 | 1,3 |
| 52 | 1,4 |
| 53 | 2,1 |
| 55 | 1,2 |
| 60 | 2,5 |
| 61 | 2,4 |
| 62 | 2,5 |
| 63 | 2,5 |
| 87 | 1,4 |

### 2. Inhibierung von Matrix Metalloproteasen (MMP)

Die erfindungsgemäßen Verbindungen zeigen deutliche inhibitorische Wirkungen auf proteolytische Enzyme, die sogenannten Matrix Metalloproteasen. Dies ist von großer Bedeutung, weil diese dem Fachmann an sich bekannten Enzyme am proteolytischen Abbau der intakten Knorpelmatrix entscheidend beteiligt sind.

Zellkultur: Kaninchen Synoviocyten (HIG-82; ATCC, Rockville, Maryland, USA) werden in Nährmedium HAM'S F12 (Sigma, Deisenhofen, Deutschland, Katalog Nr. N-6760) mit 10 % fötalem Kälberserum (Sigma, Deisenhofen, Deutschland, Katalog Nr. F-2442), zusammen mit Penicillin 100 U/ml sowie Streptomycin 100 *µ*g/ml, kultiviert. Nach konfluentem Zellwachstum wird die MMP Expression in serumfreiem HAM'S F12-Medium durch Zugabe von 0,3 *µ*mol/l Phorbol-12-Myristat-13-Acetat induziert. Nach 20 Stunden Inkubationszeit bei 37°C wird der Überstand abgenommen.

Aktivierung der MMP: Der Überstand wird mit Trypsin (5 *µ*g/ml) aktiviert. Nach 15 Minuten bei 37°C wird die Aktivierung durch Zugabe von 1 mmol/l Phenylmethylsulfonylfluorid (PMSF) beendet und für weitere 10 Minuten inkubiert. Das Gesamtvolumen des Ansatzes beträgt 210 *µ*l.

Messung der MMP Aktivität (C. G. Knight et al.: FEBS Lett. 296: 263, 1992): 20 *µ*l des obengenannten Überstandes werden 1:10 verdünnt, und mit 240 *µ*l Puffer (0,1 M Tris/HCl pH 7,5; 0,1 M NaCI; 0,01 M CaCl₂; 0,05 % Brij) gemischt. Die Testsubstanz wird in der angegebenen Konzentration (s. Tabelle) zugegeben. Nach einer Inkubationszeit von 15 Minuten wird die Reaktion durch Zugabe von 20 *µ*mol/l Fluoreszenz-Substrat ((7-Methoxycoumarin-4-yl)acetyl-Pro-Leu-Gly-Leu-[3-(2',4'-dinitrophenyl)-L-2,3-diaminopropionyl]-Ala-Arg-NH₂ (Bachem, Heidelberg, Deutschland, Katalog Nr. M-1895) gestartet. Die Reaktion wird nach 30 Minuten durch Zugabe von 10 mmol/l EDTA beendet. Das Gesamtvolumen des Ansatzes beträgt 320 *µ*l. Als Meßparameter dienen die Fluoreszenzintensitäten bei λₑₘ: 328 nm und λₑₓ: 393 nm. Um die mögliche Eigenfluoreszenz der Testsubstanzen zu berücksichtigen, werden die Fluoreszenzintensitäten von Parallel-Messungen ohne Substrat von den Messwerten mit Substrat abgezogen. Alle Schritte erfolgen bei 20°C. Im Kontrollexperiment ohne Hemmstoff wird die Fluoreszenz entsprechend 0 % Hemmung eingesetzt, während völlige Auslöschung der Fluoreszenz 100 % der Hemmung bedeutet. Ergebnisse: Die Ergebnisse sind in Tabelle 3 aufgeführt.

**Tabelle 3:**

| Hemmung der MMP | | |
|---|---|---|
| Beispiel | Hemmung (%) | Konzentration (*µ*M) |
| 14 | 50 | 100 |
| 14 | 19 | 30 |
| 15 | 26 | 100 |
| 15 | 4 | 30 |
| 21 | 51 | 100 |
| 21 | 19 | 30 |
| 26 | 51 | 100 |
| 26 | 24 | 30 |
| 26 | 15 | 10 |
| 27 | 48 | 100 |
| 27 | 24 | 30 |
| 28 | 9 | 100 |
| 31 | 35 | 100 |
| 31 | 12 | 30 |
| 31 | 8 | 10 |
| 32 | 52 | 100 |
| 32 | 22 | 30 |
| 32 | 12 | 10 |
| 35 | 52 | 100 |
| 35 | 21 | 30 |
| 39 | 47 | 100 |
| 39 | 19 | 30 |
| 39 | 16 | 10 |
| 40 | 13 | 100 |
| 47 | 55 | 100 |
| 47 | 24 | 30 |
| 47 | 13 | 10 |
| 48 | 26 | 100 |
| 48 | 9 | 30 |
| 48 | 7 | 10 |
| 49 | 28 | 100 |
| 49 | 8 | 30 |
| 50 | 14 | 100 |
| 51 | 14 | 100 |

### 3. Hemmung der mikrosomalen Lipidperoxidation

Beim unerwünschten Abbau der Knorpelmatrix sind in beträchtlichem Umfang auch oxidative Abbauprozesse involviert. Die erfindungsgemäßen Verbindungen zeigen eine starke Hemmwirkung auf biologische Oxidationsprozesse und sind deshalb zur Inhibierung des oxidativen Knorpelabbaus besonders geeignet.

Gewinnung von Rattenlebermikrosomen: Alle Schritte werden bei 0°C durchgeführt. Die Leber einer Ratte wird, um alles Hämoglobin zu entfernen, mit 0,9 % NaCI-Lösung gründlich gespült. Die grob zerkleinerte Leber wird dann in 10 mM Tris/HCl pH 7,4; 250 mM Saccharose (10 ml Puffer/g Leber) gepottert. Zunächst wird, um Zelltrümmer zu entfernen, 5 Minuten bei 600 x g zentrifugiert. Der Überstand wird dann 10 Minuten bei 12000 x g zentrifugiert und wird mit festem CaCl₂ (117,6 mg/100 ml) auf eine Konzentration von 8 mM eingestellt. Durch Zentrifugation bei 25000 x g (15 Minuten) wird ein Mikrosomenniederschlag gewonnen. Dieser Niederschlag wird im gleichen Volumen Puffer (10 mM Tris/HCl pH 7,4; 150 mM KCI) homogenisiert und erneut 15 Minuten bei 25000 x g zentrifugiert.

Peroxidation von Rattenlebermikrosomen: Ein Testansatz besteht aus 20 *µ*l Mikrosomen (100 mg/ml), vgl. oben, gelöst in einem Puffer (250 mM Tris/HCl pH 6,6; 750 mM KCl), 10 *µ*l 50 mM MgCl₂, 10 *µ*l 200 mM Isocitronensäure, 10 *µ*l 4 mM NADP (3,028 mg/ml, in Wasser), 10 *µ*l 25 mM Niacinamid, 10 *µ*l Isocitratdehydrogenase (Boehringer, 1:100 verdünnt) und 20 *µ*l Wasser bzw. Testsubstanz. Gestartet wird die Reaktion mit 10 *µ*l 0,25 mM FeSO₄. Die Inkubation dauert 10 Minuten und erfolgt bei 37°C. Gestoppt wird mit 500 *µ*l eiskalter 20 prozentiger Trichloressigsäure. Gebildetes Malondialdehyd wird durch Zugabe von 500 *µ*l 0,67 prozentiger Thiobarbitursäure und 30 min Inkubation bei 90°C in eine pinkfarbene Verbindung überführt, die bei 532 nm photometrisch gemessen wird. Die Extinktion im Kontrollansatz ohne Testsubstanz wird auf 0 % Hemmung gesetzt, während das völlige Verschwinden des Signals 100 % Hemmung bedeutet. Ergebnisse: Die Ergebnisse sind in Tabelle 4 aufgeführt.

**Tabelle 4**

| Mikrosomale Lipidperoxidation | |
|---|---|
| Beispiel | IC₅₀ (µmol/l) |
| 47 | 2,10 |

### 4. Freisetzung von Interleukinen (z.B. IL-1β) aus humanen mononuklearen Zellen

Die erfindungsgemäßen Verbindungen haben eine starke Hemmwirkung auf die Freisetzung von Interleukinen aus Humanzellen. Dies ist von großer pharmakologischer Bedeutung, weil Interleukine den unerwünschten Abbau der Knorpelmatrix auslösen können.

Gewinnung mononuklearer Zellen aus Humanblut: 10 ml Humanblut, stabilisiert mit 1 ml 3,8 prozentiger Natriumcitrat-Lösung, werden mit 10 ml PM16 (Serva, Heidelberg) verdünnt und mit 15 ml Lymphoprep (Dr. Molter GmbH, Heidelberg) unterschichtet. Die Proben werden 40 Minuten bei 400 x g (Minifuge 2, Heraeus, Osterode) bei Raumtemperatur zentrifugiert. Die mononuklearen Zellen sind als weißer Ring an der Grenze Lymphoprep/Plasma sichtbar. Dieser Ring wird mit einer Spritze vorsichtig entnommen, mit dem gleichen Volumen PM16 verdünnt und 10 Minuten bei 400 x g zentrifugiert. Der Niederschlag wird mit 10 ml RPMI1640 (+300 mg/l L-Glutamin, Gibco, Eggenstein) gewaschen. Nach suspendieren der Zellen in ∼1 ml RPMI1640 (+300 mg/l L-Glutamin, +25 mM HEPES, + 100 *µ*g/ml Streptomycin, + 100 *µ*g/ml Penicillin) wird die Zelldichte mit einem Coulter Counter JT (Coulter Diagnostics) bestimmt und auf 5•10⁶/ml eingestellt. 90 % der erhaltenen Zellen sind Lymphocyten und 10 % Monocyten.

Hemmung der Freisetzung von Interleukin-1β: 230 *µ*l mononuklearer Zellen werden mit 10 *µ* l Testsubstanz (10 *µ*M in Dimethylsulfoxid (DMSO)/Wasser = 1/10) und 10 *µ*l einer Lipopolysaccharid-Lösung (500 *µ*g gelöst in 1 ml DMSO und vor Testbeginn 1/10 mit Wasser verdünnt, von Salmonella abortus equi, Sigma, Deisenhofen) 20 bis 22 Stunden bei 37°C, 5 % CO₂ inkubiert. Die Proben werden in einem Eisbad auf 0°C abgekühlt und in einer Sigma-Zentrifuge zentrifugiert (2 Minuten; 2000 Umdrehungen pro Minute (rpm)). Aliquote des Überstandes werden mit einem kommerziell erhältlichen Elisa-Test (Biermann, Bad Nauheim) bestimmt. Ergebnisse: Die Ergebnisse sind in Tabelle 5 aufgeführt.

**Tabelle 5**

| Hemmung der Interleukin Freisetzung | |
|---|---|
| Beispiel | % Hemmung |
| 26 | 59 |
| 27 | 99 |
| 31 | 97 |
| 32 | 80 |
| 35 | 83 |
| 36 | 15 |
| 39 | 63 |
| 41 | 11 |
| 42 | 77 |
| 47 | 80 |
| 63 | 92 |

Alle Wirkstoffe wurden in einer Konzentration von 10 *µ*mol/l getestet. Es wurde jeweils die IL-1β-Freisetzung geprüft.

### 5. Wirksamkeit als Antagonist der Kontraktion der isolierten Trachea des Meerschweinchens

Die erfindungsgemäßen Verbindungen werden als Antagonisten der Kontraktion verschiedener Organteile, hier der isolierten Trachea des Meerschweinchens verwendet. Als Agonisten wurden KCl, PGF₂ₐ, Calciumionophor A23187 und Substanz P eingesetzt:
a) KCl
   Präparate mit unspezifischer Wirkung zeigen im allgemeinen gegenüber allen Agonisten eine spasmolytische Wirkung. Als unspezifische, rezeptorunabhängige Kontraktion steht stellvertretend hier KCI, da KCI-induzierte Kontraktionen überwiegend physikalisch, also durch Erhöhung der K+-Konzentration im Außenmilieu entstehen und das Ruhepotential der Zelle verändern.
b) PGF2a
   PGF2a entfaltet seine kontraktile Wirkung über eigene Rezeptoren. Hemmung von Kontraktionen, welche durch dieses Cyclooxigenaseprodukt induziert werden, sind nur durch kompetitive Antagonisierung am Rezeptor oder des nachgeschalteten Signalweges möglich.
c) Calciumionophor A23187 (Calbiochem, Bad Soden) bewirkt durch erhöhte Aufnahme von Calcium-lonen, in der Zelle eine Aktivierung aller calciumabhängigen Signalkaskaden. Insbesondere Lipoxigenasehemmer und generell antientzündlich wirkende Substanzen zeigen in diesem Modell Effekte.
d) Substanz P Substanz P als Transmitterstoff zwischen Immunsystem und Nervensystem zeigt eine kontraktile Wirkung gegenüber glatter Muskulatur. Die Antagonisierung ist einerseits durch spezifische Rezeptorantagonisten, andererseits auch durch Inhibition des nachgeschalteten Signalwegs möglich. Insbesondere Präparate, welche die intrazelluläre Konzentration von cyclischen Nukleotiden direkt oder indirekt erhöhen, zeigen hier spasmolytische Eigenschaften.

### Präparation der Organteile und Versuchsdurchführung:

Das Meerschweinchen wird durch finale Lachgasnarkose getötet. Die Trachea wird in ganzer Länge herauspräpariert und in 15 einzelne Knorpelringe geschnitten. Jeweils fünf Ringe werden zu einer Kette zusammengebunden und im Organbad unter 3 g Vorlast fixiert. Nach 45 Minuten Äquilibrierungszeit wird mit dem Agonisten eine Kontraktion erzeugt. Auf dem Plateau vom Maximum wird der Antagonist (Testpräparat) kumulativ addiert. Die Auswertung erfolgt in % Kraftänderung bezogen auf das Kontraktionsmaximum. Die Versuchsdurchführung erfolgt bei 37°C, als physiologische Nährlösung wird eine mit 95 Vol.-% O₂, 5 Vol.-% CO₂ durchperlte, modifizierte Krebs-Henseleit-Lösung benutzt.
- Versuchstiere;: Meerschweinchen Albino (102), Gewicht: 200 bis 300 g, männliches oder weibliches Geschlecht

Zusammensetzung des Organbads (Nährlösung):

| Modifizierte Krebs-Henseleit-Lösung und Calciumionophor | | für die Agonisten KCl, PgF₂ₐ für den Agonisten SP | |
|---|---|---|---|
| NaCl | 6,9 g | NaCl | 7,9 g |
| KH₂PO₄ | 0,14 g | KH₂PO₄ | 0,18 g |
| NaHCO₃ | 2,1 g | NaHCO₃ | 1,37 g |
| Glucose | 2,0 g | Glucose | 1,53 g |
| KCl | 0,35 g | KCl | 0,25 g |
| CaCl₂ | 0,28 g | CaCl₂ | 0,27 g |
| MgSO₄ | 0,14 g | auf 1 l Aqua bidest. | |
| auf 1 l Bidestilliertes Wasser (Aqua bidest.) | | | |

Vehikel: Aqua bidest., Ethanol oder Isopropanol; Applikation: ins Organbad Anzahl der Applikation: Kumulativ, bei SP Einzeldosen

Ergebnisse: Es zeigt sich eine antagonistische Wirkung der erfindungsgemäßen Verbindungen in bezug auf KCI, das Prostaglandin PgF_{2α}, Calciumionophor (A 23187) und Substanz P. Die Ergebnisse sind in Tabelle 6 aufgeführt.

**Tabelle 6:**

| Antikontraktile Wirkung (Trachea) | | | | |
|---|---|---|---|---|
| Beispiel | KCl | PgF_{2α} | Ca-lonophor | Substanz P |
| 11 | >10 | >10 | | 10-30 |
| 30 | >10 | 6-10 | 3-6 | 10-30 |
| 34 | >10 | >10 | | 1-10 |
| 37 | ca. 10 | ca. 3 | ca. 1 | ca. 3 |
| 38 | >10 | 1-3 | ca. 3 | 3-10 |
| 44 | >10 | ca. 6 | | 10-30 |
| 52 | >10 | >10 | 6-10 | |

Alle Zahlenwerte sind in der Einheit *µ*g/ml angegeben und beziehen sich auf den Konzentrationsbereich an getesteter, erfindungsgemäßer Verbindung (siehe Beispielnummern in Tabelle 1), der nötig ist, um eine Dilatation zu bewirken, die dem ED₅₀-Bereich entspricht.

### 6. Wirksamkeit als Antagonist der Kontraktion isolierter Lungenstreifen des Meerschweinchens

Der durchgeführte Test basiert auf dem gleichen Prinzip wie der in der pharmakologischen Prüfung 5 angegebene Test; hier werden jedoch Lungenstreifen verwendet.

### Präparation der Organteile und Versuchsdurchführung:

Das Meerschweinchen wird in einer Lachgasnarkose getötet. Der gesamte Lungentrakt wird von der Trachea ausgehend herausgeschnitten. Die Lungenlappen werden zirkulär geschnitten, so daß ca. 3 mm breite Streifen entstehen. Die Streifen der Oberlappen werden geteilt und um insgesamt sechs etwa gleich große Streifen zu erhalten. Die Streifen werden unter eine Vorlast von 4 g in die Organbäder eingehängt. Als Nährlösung werden modifizierte Krebs-Henseleit-Lösungen verwandt. Bei Verwendung von "Platelet activating factor" (PAF) als Agonist entspricht die Zusammensetzung dieser Lösung der für den Agonisten SP in "Pharmakologischen Test 5"; für den Agonisten LTD₄ wird eine Krebs-Henseleit-Lösung wie im Falle des Agonisten KCI (Pharm. Test 5) verwendet. Zur Durchperlung des Bades wird 95 Vol.-% O₂, 5 Vol.-% CO₂ benutzt, die Badtemperatur beträgt 37°C. Die Versuchsdurchführung erfolgt in einer therapeutisch-kumulativen Methodik, mit den Dosierungsabstufungen 1, 3, 6 und 10 *µ*g/ml.

Versuchstiere, Organbad, Vehikel, Art der Applikation entsprechen dem in der pharmakologischen Prüfung 5 gesagten.

Ergebnisse: Es zeigt sich eine antagonistische Wirkung der erfindungsgemäßen Verbindungen in bezug auf "Leukotrien D₄" (LTD₄) und das Membranlipid "Platelet activating factor". Die Ergebnisse sind in Tabelle 7 aufgeführt.

**Tabelle 7**

| Antikontraktile Wirkung (Lunge) | | |
|---|---|---|
| Beispiel | LTD₄ | PAF |
| 1 | >10 | 1-3 |
| 4 | 3-6 | 1-3 |
| 9 | 1-3 | 1-3 |
| 11 | | 6-10 |
| 30 | ca. 10 | 1-3 |
| 37 | 0,1-0,3 | 1-3 |
| 38 | ca. 1 | <0,1 |
| 39 | ca. 10 | |
| 40 | >10 | 1-3 |
| 41 | >10 | 1-3 |
| 42 | >10 | 3-6 |
| 46 | ca. 10 | 1-3 |
| 56 | 3-6 | 1-3 |
| 61 | >10 | ca. 3 |
| 70 | ca. 6 | |

Alle Zahlenwerte sind in der Einheit *µ*g/ml angegeben und beziehen sich auf den Konzentrationsbereich an getesteter, erfindungsgemäßer Verbindung (siehe Beispielnummern in Tabelle 1), der nötig ist, um eine Kontraktion zu bewirken, die dem IC₅₀-Wert entspricht.

### 7. Adjuvans-Arthritis

Die Untersuchungen erfolgten wie in EP 0 432 740 beschrieben. Nach 18 Tagen 2 mal-täglicher intraperetonealer Applikation von 12,6 mg der Verbindung 38 pro kg von Wistar-Lewis-Ratten ergibt sich eine 98 % Hemmung der Pfotenvolumenzunahme im Vergleich mit einer unbehandelten Kontrollgruppe.

### 8. Freisetzung von Tumor Nekrose Faktor α (TNFα) und Substanz P aus RAW 264.7-Zellen

Kulturmedium: DMEM + 10 % FCS + Penicillin/Streptomycin (50 U/50 *µ*g/ml)
Kulturbedingungen: 37°C, 10 % CO₂
Kulturschalen: 24 well-Schalen

- Je 106 Zellen/ml/well werden 24 Stunden (h) inkubiert
- Zugabe von 10 *µ*l Testsubstanz ( = 100 *µ*M im Test, gelöst in Aqua bidest.)
- 1 h Inkubation
- Zugabe von 50 *µ*l Lipopolysaccharid (LPS) aus E. coli (= 10 pg/ml im Test, gelöst in Kulturmedium)
- 2,5 h und 24 h Inkubation

Die Bestimmung von Tumor Nekrose Faktor α erfolgt mit einem Mouse TNFα-ELISA-Kit Factortest X der Fa. Genzyme (Rüsselsheim, Deutschland), Bestellnr. 80-2802-00.

Verbindung 37 zeigt nach 2,5 Stunden (h) Inkubation 55 % Hemmung der TNFα-Bildung im Vergleich mit einer unbehandelten Kontrolle; Verbindung 38 zeigt nach 4 h Inkubation 16 % Hemmung.

Die Freisetzung von Substanz P erfolgt wie für TNFα beschrieben, allerdings erfolgt die Stimulation der Zellen durch 50 ng LPS für 4 Stunden. Die Bestimmung von Substanz P erfolgt durch einen Substance P RIA-Testkit der Fa. Peninsula Laboratories (Belmont, USA) Testnr. RIK-7451.

Die unbehandelten Zellen bilden 20 pg/ml Substanz P, während die Verbindung 37 und 38 die Bildung von Substanz P zu 100 % hemmen.

### 9. Hemmung der Phosphodiesterase III-Aktivität

Der Test wird mit der Phosphodiesterase der Fa. Boehringer Mannheim (Mannheim, Deutschland), Bestellnr. 108 243 nach der Methode von T. SAEKI, I. SAITO, Biochem. Pharm. 46, Nr. 5, (1986), Seiten 833-839 durchgeführt.

100 *µ*M der Verbindung 37 bewirkt im Test eine 39 %ige Hemmung der Enzymaktivität.

## Patentansprüche

1. Verbindung der Formel (I) 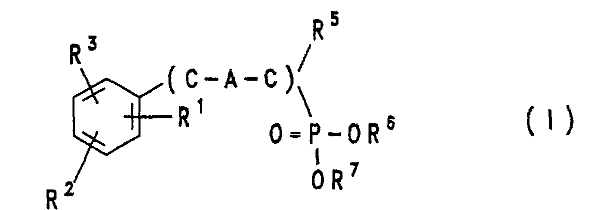 und/oder ein physiologisch verträgliches Salz der Verbindung der Formel (I) und/oder eine stereoisomere Form der Verbindung der Formel (I), wobei mindestens zwei der Reste R¹, R² und R³ vorhanden sind und unabhängig voneinander für
1) OH,
2) (C₁-C₁₂)-Alkoxy,
3) -O-(C₁-C₁₂)-Alkyl-COOH,
4) -O-(C₁-C₁₂)-Alkyl-C(O)-O-(C₁-C₁₂)-Alkyl,
5) (C₃-C₁₂)-Cycloalkoxy,
6) (C₃-C₆)-Alkenyloxy,
7) (C₅-C₇)-Cycloalkyl-(C₁-C₃)-alkoxy,
8) Heteroaryl-(C₁-C₃)-alkoxy, wobei die Heteroatome N, S und/oder O sind,
9) Heterocycloalkyl-(C₁-C₃)-alkoxy, wobei die Heteroatome N, S und/oder O sind, der Heterocycloalkylrest unsubstituiert oder ein- bis dreifach mit (C₁-C₃)-Alkyl substituiert ist und die Heterocycloalkylgruppe fünf- oder sechsgliedrig ist,
10) Phenyl-(C₁-C₃)-alkoxy,
11) Benzyloxy, ein- bis dreifach substituiert durch Halogenmethyl oder (C₁-C₃)-Alkoxy,
12) Phenoxy, ein- bis dreifach substituiert durch (C₁-C₃)-Alkoxy, stehen,
13) zwei der Reste R¹, R² oder R³, welche zwei direkt benachbarte C-Atome des aromatischen Rings substituieren, bilden zusammen einen Methylendioxy- oder Ethylendioxyrest am aromatischen Ring,
14) einen Rest der Formeln II, III oder IV 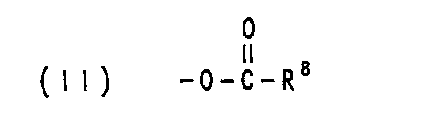 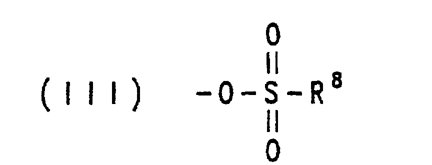 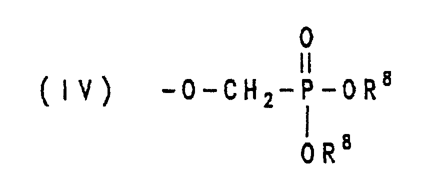 wobei R⁸ (C₁-C₄)-Alkyl oder Wasserstoffatom bedeutet,
15) eine Gruppe der Formel V 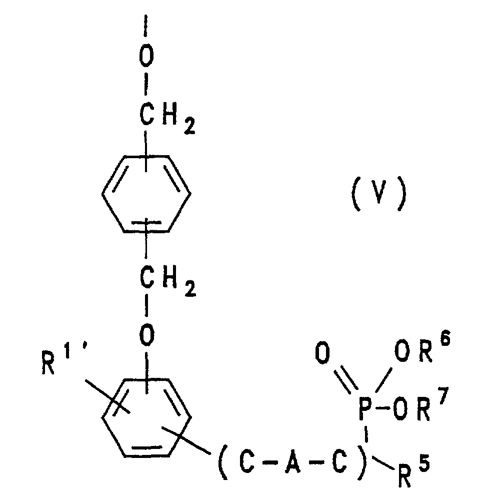 stehen, wobei
R^{1'} wie für R¹ von 1) bis 12) definiert ist und (C-A-C), R⁵, R⁶ und
R⁷ wie unten definiert sind, oder
16) R¹ und R⁷ eine kovalente Bindung sind und dadurch eine Verbindung der Formel la bilden 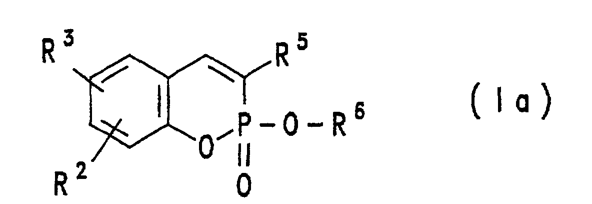 wobei, R² und R³ wie oben und R⁵ und R⁶ wie unten definiert sind, oder
17) R¹ und R⁵ eine kovalente Bindung sind und dadurch eine Verbindung der Formel Ib bilden 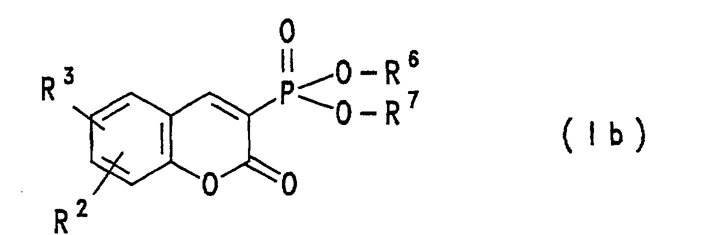 wobei, R² und R³ wie oben und R⁶ und R⁷ wie unten definiert sind, und
R⁵
1) CN,
2) CH₂NHR⁹ bedeutet, wobei R⁹ Wasserstoffatom oder -C(O)-(C₁-C₃)-Alkyl bedeutet, oder
3) für einen Rest der Formel VI 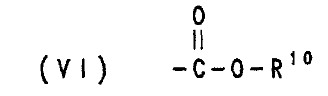 steht, wobei
R¹⁰
1) (C₁-C₆)-Alkyl, unsubstituiert oder ein- bis vierfach substituiert durch 1.1 -COOH, 1.2 -C(O)-O-(C₁-C₃)-Alkyl oder 1.3, 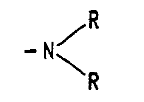 wobei R
1.3.1 Wasserstoffatom, 1.3.2 (C₁-C₃)-Alkyl bedeutet oder
1.3.3 zusammen mit dem N-Atom an das es gebunden ist einen Morpholinring bildet, oder
2) Trialkylsilyl bedeutet,
R⁶ und R⁷ unabhängig voneinander
1) Wasserstoffatom oder
2) (C₁-C₆)-Alkyl bedeuten, (C-A-C) für
1) (CH=CH-CH=C),
2) (CH₂-CH₂-CH₂-CH),
3) (CH₂-CH₂-CH),
4) (-CH₂-CH) oder
5) (-CH=C) steht,
mit der Maßgabe, daß
wenn der Rest R⁵ eine CN-Gruppe bedeutet, höchstens einer der Reste R¹, R² oder R³ für einen Hydroxylrest steht, oder
wenn der Rest R¹⁰ Methyl, Ethyl oder t-Butyl bedeutet, die Reste R¹, R² oder R³ nicht für Methoxy stehen, sowie
die Verbindungen 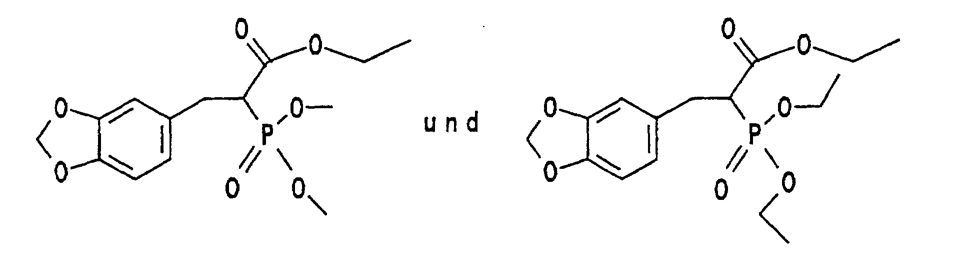
ausgenommen sind.

2. Verbindung der Formel I gemäß Anspruch 1, wobei mindestens zwei der Reste R¹, R² und R³ vorhanden sind und unabhängig voneinander für
1) OH,
2) (C₁-C₆)-Alkoxy,
3) -O-(C₁-C₆)-Alkyl-COOH,
4) -O-(C₁-C₆)-Alkyl-C(O)-O-(C₁-C₆)-Alkyl,
5) (C₅-C₇)-Cycloalkoxy,
6) (C₃-C₆)-Alkenyloxy,
7) (C₅-C₇)-Cycloalkyl-(C₁-C₂)-alkoxy,
8) Heteroaryl-(C₁-C₂)-alkoxy, wobei die Heteroatome N und/oder O sind,
9) Heterocycloalkyl-(C₁-C₂)-alkoxy, wobei die Heteroatome N und/oder O sind, der Heterocycloalkylrest unsubstituiert oder ein- bis dreifach mit (C₁-C₃)-Alkyl substituiert ist und die Heterocycloalkylgruppe fünf- oder sechsgliedrig ist,
10) Phenyl-(C₁-C₂)-alkoxy,
11) Benzyloxy, ein- bis dreifach substituiert durch Halogenmethyl oder (C₁-C₃)-Alkoxy oder
12) Phenoxy, ein- bis dreifach substituiert durch (C₁-C₃)-Alkoxy, bedeuten,
13) zwei der Reste R¹, R² oder R³ gleichzeitig für eine Gruppe der Formel (II) 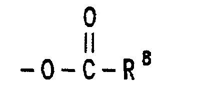 , wobei R⁸ (C₁-C₄)-Alkyl bedeutet,
stehen, und
R⁵
1)CN,
2) CH₂NHR⁹ bedeutet, wobei R⁹ Wasserstoffatom oder
-C(O)-(C₁-C₃)-Alkyl bedeutet, oder
3) für einen Rest der Formel VI 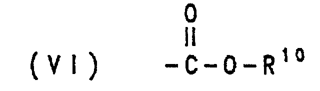 steht, wobei
R¹⁰
1) (C₁-C₆)-Alkyl, unsubstituiert oder einfach substituiert durch 1) -COOH, 2) -C(O)-(C₁-C₃)-Alkyl oder 3) 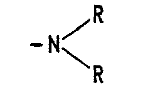 , wobei R für (C₁-C₃)-Alkyl steht, oder
2) Trialkylsilyl bedeutet,
R⁶ und R⁷ unabhängig voneinander Wasserstoffatom oder (C₁-C₄)-Alkyl bedeuten und
(C-A-C) für 1) (-CH₂-CH) oder 2) (-CH=C) steht.

3. Verbindung der Formel I gemäß Ansprüche 1 oder 2, wobei
R¹ Methoxy,
R² Methoxy oder Benzyloxy,
R³ Methoxy oder Benzyloxy bedeutet, und
R⁵
1)CN,
2) CH₂NHR⁹ bedeutet, wobei R⁹ Wasserstoffatom oder -C(O)-(C₁-C₃)-Alkyl bedeutet, oder
3) für einen Rest der Formel VI steht, wobei
R¹⁰ (C₁-C₄)-Alkyl, substituiert durch
1)-COOH, oder
2) 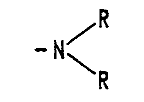 , wobei R für Wasserstoffatom und/oder (C₁-C₃)-Alkyl steht, bedeutet,
R⁶ und R⁷ Wasserstoffatom oder (C₁-C₄)-Alkyl bedeuten und
(C-A-C) für einen (-CH=C) Rest steht.

4. Verbindung der Formel I gemäß einem oder mehreren der Ansprüche 1 bis 3, wobei
R¹ Wasserstoffatom und
R² und R³ beide Methoxy bedeuten, und
R⁵ für eine Gruppe der Formel VI steht, wobei
R¹⁰ Isopropyl bedeutet,
R⁶ und R⁷ gleichzeitig Ethyl oder Methyl bedeuten und
(C-A-C) für einen (-CH₂-CH) Rest steht.

5. Verbindung der Formel I gemäß einem oder mehreren der Ansprüche 1 bis 4, wobei R¹ Wasserstoffatom und
R² und R³ beide Benzyloxy bedeuten, und
R⁵ für eine Gruppe der Formel VI steht, wobei
R¹⁰ Isopropyl bedeutet,
R⁶ und R⁷ Methyl oder Ethyl bedeuten und
(C-A-C) für einen (-CH=C) Rest steht,

6. Verfahren zur Herstellung der Verbindung der Formel 1, **dadurch gekennzeichnet, daß** man
a) eine Verbindung der Formel VII 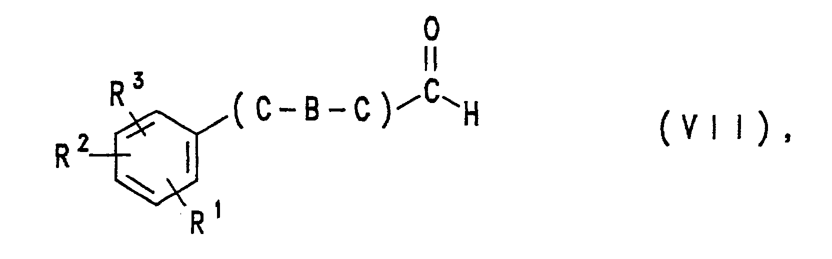 wobei R¹, R² und R³ wie in Formel I definiert sind und (C-B-C) für eine kovalente Bindung, (-CH=CH-), (-CH₂-CH₂-CH₂), (-CH₂-CH₂) oder (-CH₂-) steht, mit einer Verbindung der Formel VIII oder mit einem Salz einer Verbindung der Formel VIII 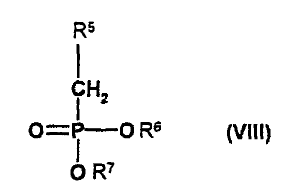 , wobei R⁵, R⁶ und R⁷ wie in Formel I definiert sind, in Anwesenheit von Tetrahydrofuran und Titantetrachlorid oder in Anwesenheit von Tetrahydrofuran und einem Orthotitansäuretriester der Formel IX
(IX) (R¹¹)₃TiCl,
wobei R¹¹ für -O-(C₁-C₆)-Alkyl steht, umsetzt, oder
b) eine nach Verfahren a) hergestellte Verbindung der Formel I, die aufgrund ihrer chemischen Struktur in enantiomeren oder stereoisomeren Formen auftritt, durch Salzbildung mit enantiomerenreinen Säuren oder Basen, Chromatographie an chiralen Stationärphasen oder Derivatisierung mittels chiraler enantiomerenreiner Verbindungen wie Aminosäuren, Trennung der somit erhaltenen Diastereomeren, und Abspaltung der chiralen Hilfsgruppe in die reinen Enantiomeren auftrennt oder die Stereoisomeren durch Chromatographie auftrennt, oder
c) eine nach a) hergestellte Verbindung der Formel I, wobei mindestens ein R¹, R² oder R³ ein Rest der Formel II oder III ist, zum entsprechenden Phenol verseift, oder
d) die Umsetzung gemäß Verfahren c) in Gegenwart von Natriumhydrogencarbonat durchführt,
e) eine nach a) hergestellte Verbindung der Formel I, wobei R⁵ für einen Rest der Formel VI steht und/oder mindestens ein R¹, R² oder R³ ein Rest -O-(C₁-C₁₂)-Alkyl-C(O)-O-(C₁-C₁₂)-Alkyl ist, zur Carbonsäure verseift, oder
f) die Umsetzung gemäß Verfahren e) in Gegenwart von einer ethanolischen Kaliumhydroxidlösung oder einer Salzsäurelösung, durchführt, oder
g) eine nach a) hergestellte Verbindung der Formel I, enthaltend eine oder zwei Doppelbindungen mit α.) Wasserstoff und einem Pd/C-Katalysator oder Raney-Nickel oder β.) Natriumborhydrid hydriert, oder
h) einen nach a) hergestellten Phosphonsäuremonoalkyl- oder Phosphonsäuredialkylester der Formel I zum Phosphonsäurehalbester oder zur Phosphonsäure verseift, wobei die Phosphonsäureesterspaltung in Gegenwart von Bromtrimethylsilan in Dichlormethan durchführt wird, oder
i) die nach Verfahren a), b), c), e), g), h), k) oder I) hergestellte Verbindung der Formel I entweder in freier Form isoliert oder im Falle des Vorliegens von sauren oder basischen Gruppen gegebenenfalls in physiologisch verträgliche kristalline Salze umwandelt, oder
k) eine Verbindung der Formel I, wobei mindestens einer der Reste R¹, R² oder R³ ein Hydroxylrest ist mit einer Verbindung der Formel XI
(XI) X-R¹³,
wobei X Halogen oder
gegebenfalls substituierte Phenylsulfonyloxy ist und
R¹³wie für R¹ 2) bis 12) in Formel I definiert ist, wobei X das O-Atom von R¹ ersetzt, welches mit dem Phenylrest verknüpft ist, nach Behandlung mit Natriumhydrid oder in Gegenwart von Kaliumcarbonat in Acetonitril, Dimethylformamid oder cyclischen Ketonen umsetzt oder mit R¹³COCl oder 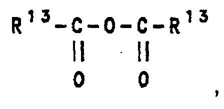 wobei R¹³ wie R⁸ definiert ist, gegebenenfalls katalysiert durch Stickstoffbasen wie Pyridin, zu den entsprechenden Phenolethern oder Phenolestern umsetzt,
l) eine nach Verfahren a) hergestellte Verbindung der Formel I, wobei R⁵ für CN steht, in Gegenwart von Wasserstoff und einem Pd/C-Katalysator oder Raney-Nickel, gemäß dem unter C)α.) beschriebenen Verfahren, hydriert, oder
m) eine nach Verfahren l) erhaltene Aminoverbindung mit einem (C₁-C₃)-Alkylcarbonsäureanhydrid, zum entsprechenden Carbonsäureamid umsetzt, oder
n) eine nach Verfahren a) hergestellte Verbindung der Formel I, wobei R⁵ für - COOH steht, durch Veresterung in den entsprechenden Carbonsäureester umsetzt, wobei die Carbonsäuren mittels Oxalylchlorid in das Carbonsäurechlorid überführt und dieses mit R¹⁰-OH umgesetzt wird.

7. Arzneimittel, enthaltend eine wirksame Menge mindestens einer Verbindung der Formel (I) 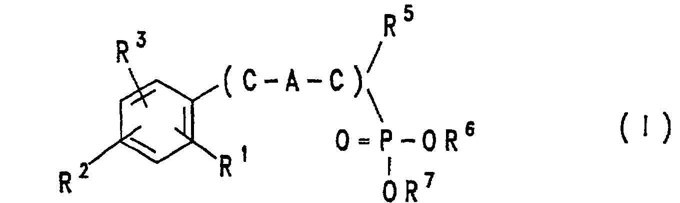 und/oder ein physiologisch verträgliches Salz der Verbindung der Formel (I) und/oder eine stereoisomere Form der Verbindung der Formel (I), wobei mindestens zwei der Reste R¹, R² und R³ vorhanden sind und unabhängig voneinander für
1) OH,
2) (C₁-C₁₂)-Alkoxy,
3) -O-(C₁-C₁₂)-Alkyl-COOH,
4) -O-(C₁-C₁₂)-Alkyl-C(O)-O-(C₁-C₁₂)-Alkyl,
5) (C₃-C₁₂)-Cycloalkoxy,
6) (C₃-C₆)-Alkenyloxy,
7) (C₅-C₇)-Cycloalkyl-(C₁-C₃)-alkoxy,
8) Heteroaryl-(C₁-C₃)-alkoxy, wobei die Heteroatome N, S und/oder O sind,
9) Heterocycloalkyl-(C₁-C₃)-alkoxy, wobei die Heteroatome N, S und/oder O sind, der Heterocycloalkylrest unsubstituiert oder ein- bis dreifach mit (C₁-C₃)-Alkyl substituiert ist und die Heterocycloalkylgruppe fünf- oder sechsgliedrig ist,
10) Phenyl-(C₁-C₃)-alkoxy,
11) Benzyloxy, ein- bis dreifach substituiert durch Halogenmethyl oder (C₁-C₃)-Alkoxy,
12) Phenoxy, ein- bis dreifach substituiert durch (C₁-C₃)-Alkoxy, stehen,
13) zwei der Reste R¹, R² oder R³, welche zwei direkt benachbarte C-Atome des aromatischen Rings substituieren, bilden zusammen einen Methylendioxy- oder Ethylendioxyrest am aromatischen Ring,
14) einen Rest der Formeln II, III oder IV 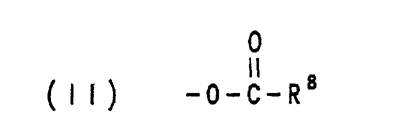 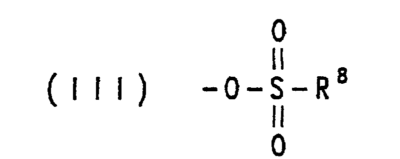 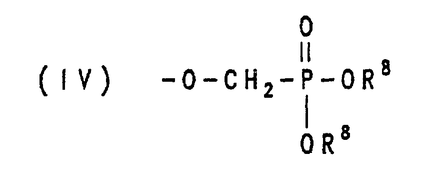 stehen, wobei
R⁸ (C₁-C₄)-Alkyl oder Wasserstoffatom bedeutet,
15) für eine Gruppe der Formel V 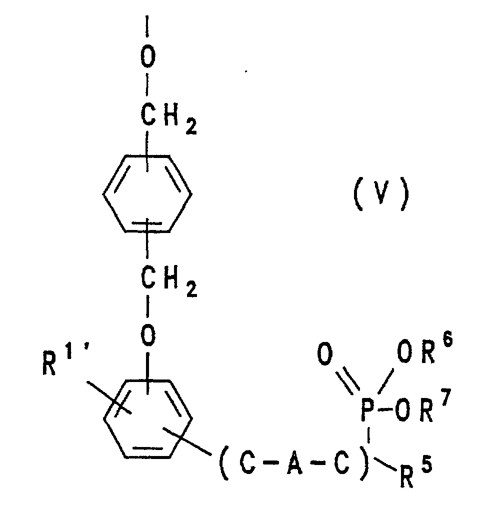 stehen, wobei
R^{1'} wie für R¹ von 1) bis 12) definiert ist und (C-A-C), R⁵, R⁶ und R⁷ wie unten definiert sind, oder
16) R¹ und R⁷ bilden eine Verbindung der Formel la 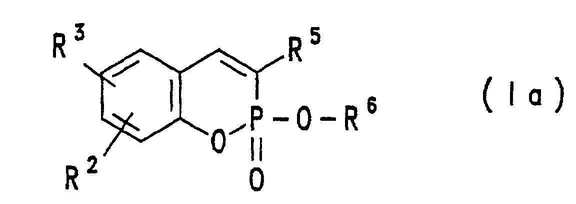 wobei, R² und R³ wie oben und R⁵ und R⁶ wie unten definiert sind, oder
17) R¹ und R⁵ bilden eine Verbindung der Formel Ib 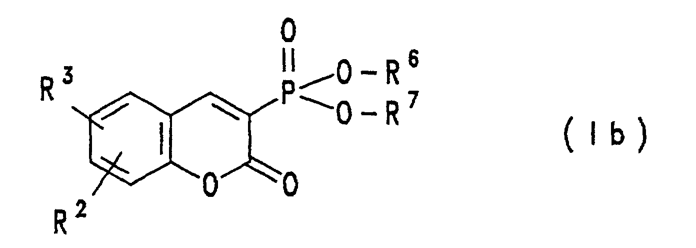 wobei, R² und R³ wie oben und R⁶ und R⁷ wie unten definiert sind, und
R⁵
1)CN,
2) CH₂NHR⁹ bedeutet, wobei R⁹ Wasserstoffatom oder -C(O)-(C₁-C₃)-Alkyl bedeutet, oder
3) für einen Rest der Formel VI 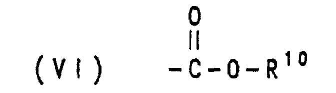 steht, wobei
R¹⁰
1) (C₁-C₆)-Alkyl, unsubstituiert oder ein- bis vierfach substituiert durch 1.1 -COOH, 1.2 -C(O)-O-(C₁-C₃)-Alkyl oder 1.3 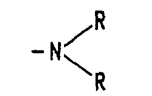 , wobei R
1.3.1 Wasserstoffatom, 1.3.2 (C₁-C₃)-Alkyl bedeutet oder 1.3.3 zusammen mit dem N-Atom an das es gebunden ist einen Morpholinring bildet, oder
2) Trialkylsilyl bedeutet,
R⁶ und R⁷ unabhängig voneinander
1) Wasserstoffatom oder
2) (C₁-C₆)-Alkyl bedeuten, und
(C-A-C) für
1) (CH=CH-CH=CH-CH),
2) (CH₂-CH₂-CH₂-CH₂-CH),
3) (CH₂-CH₂-CH₂-CH),
4) (CH₂-CH₂-CH),
5) (-CH₂-CH) oder
6) (-CH=C) steht,
mit der Maßgabe, daß
die Verbindung der Formel I, wobei (C-A-C) für (C=CH) steht, R⁵ für eine CN-Gruppe steht, R⁶ und R⁷ gleich sind und für Ethyl stehen, R¹ und R² gleichzeitig -OH bedeuten und in meta und para-Stellung zu (C-A-C) gebunden sind und R³
Wasserstoffatom bedeutet, ausgenommen ist.

8. Verwendung von mindestens einer Verbindung der Formel (I) nach einem oder mehreren der Ansprüche 1 bis 5, zur Herstellung von Arzneimitteln zur Behandlung und Prophylaxe von degenerativen Gelenkserkrankungen, von Erkrankungen des rheumatischen Formenkreises, die von einem Knorpelabbau begleitet werden wie die chronische Polyarthritis, das Gelenktrauma sowie der Knorpelschwund infolge längerer Immobilisation des Gelenks, von Entzündungen, septischen Schock, Erkrankungen mit gestörter Leukozyten-Adhäsion, Erkrankungen, die durch eine erhöhte Konzentration an Tumor Nekrose Faktor alpha bedingt sind wie Cachexie oder Morbus Crohn.

9. Verfahren zur Herstellung eines Arzneimittels nach Anspruch 7, **dadurch gekennzeichnet, daß** man mindestens eine Verbindung der Formel (I) und/oder eine nach dem Verfahren gemäß Anspruch 6 erhaltene Verbindung mit physiologisch annehmbaren Hilfs- und Trägerstoffen und gegebenenfalls weiteren Zusatzstoffen und/oder anderen Wirkstoffen in eine geeignete Darreichungsform bringt.

## Claims

1. A compound of the formula (I) 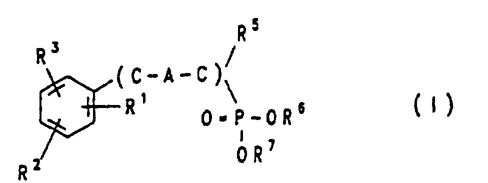 and/or a physiologically tolerated salt of the compound of the formula (I) and/or a stereoisomeric form of the compound of the formula (I), where at least two of the radicals R¹, R² and R³ are present and are, independently of one another,
1) OH,
2) (C₁-C₁₂)-alkoxy,
3) -O-(C₁-C₁₂)-alkyl-COOH,
4) -O-(C₁-C₁₂)-alkyl-C(O)-O-(C₁-C₁₂)-alkyl,
5) (C₃-C₁₂)-cycloalkoxy,
6) (C₃-C₆)-alkenyloxy,
7) (C₅-C₇) -cycloalkyl-(C₁-C₃)-alkoxy,
8) heteroaryl-(C₁-C₃)-alkoxy where the heteroatoms are N, S and/or O,
9) heterocycloalkyl-(C₁-C₃)-alkoxy where the heteroatoms are N, S and/or O, the heterocycloalkyl radical is unsubstituted or substituted once to three times by (C₁-C₃)-alkyl, and the heterocycloalkyl group has five or six members,
10) phenyl-(C₁-C₃)-alkoxy,
11) benzyloxy substituted once to three times by halomethyl or (C₁-C₃)-alkoxy,
12) phenoxy substituted once to three times by (C₁-C₃)-alkoxy,
13) two of the radicals R¹, R² or R³ which are substituents on two directly adjacent carbon atoms of the aromatic ring together form a methylenedioxy or ethylenedioxy radical on the aromatic ring,
14) a radical of the formula II, III or IV 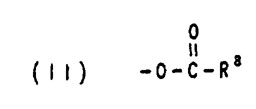 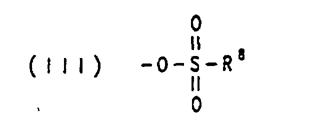 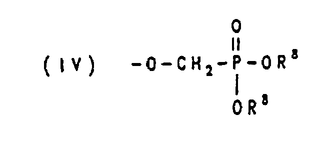 where R⁸ is (C₁-C₄)-alkyl, or hydrogen atom
15) a group of the formula V 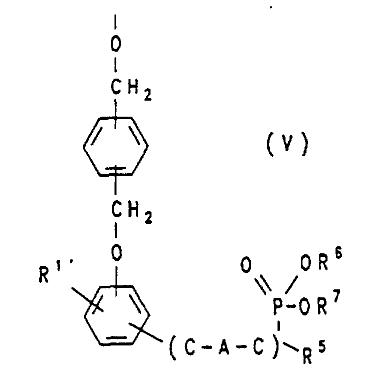 where
R¹ is defined as for R¹ from 1) to 12), and (C-A-C), R⁵, R⁶ and R⁷ are as defined below, or
16) R¹ and R⁷ are a covalent bond and thus form a compound of the formula Ia 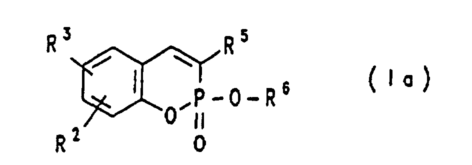 where R² and R³ are as defined above and R⁵ and R⁶ are as defined below, or
17) R¹ and R⁵ are a covalent bond and thus form a compound of the formula Ib 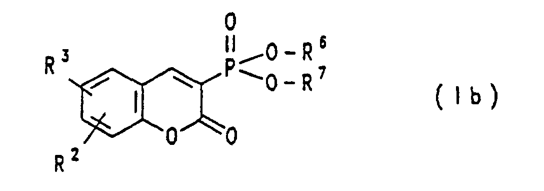 where R² and R³ are as defined above and R⁶ and R⁷ are as defined below, and
R⁵ is
1) CN,
2) CH₂NHR⁹ where R⁹ is hydrogen atom or -C(O)-(C₁-C₃)-alkyl, or
3) a radical of the formula VI 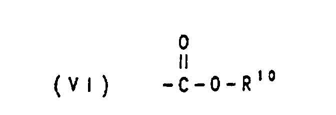 where
R¹⁰ is
1) (C₁-C₆)-alkyl, unsubstituted or substituted once to four times by 1.1 -COOH, 1.2 -C(O)-O-(C₁-C₃)-alkyl or 1.3 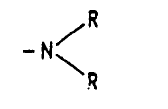 where R is
1.3.1 hydrogen atom, 1.3.2 (C₁-C₃)-alkyl or 1.3.3 forms, together with the nitrogen atom to which it is bonded, a morpholine ring, or
2) trialkylsilyl,
R⁶ and R⁷ are, independently of one another,
1) hydrogen atom or
2) (C₁-C₆)-alkyl,
(C-A-C) is
1) (CH=CH-CH=C),
2) (CH₂-CH₂-CH₂-CH),
3) (CH₂-CH₂-CH),
4) (-CH₂-CH) or
5) (-CH=C)
with the proviso that
when the radical R⁵ is a CN group, not more than one of the radicals R¹, R² or R³ is a hydroxyl radical, or when the radical R¹⁰ is methyl, ethyl or t-butyl, the radicals R¹, R² or R³ are not methoxy, and the compounds 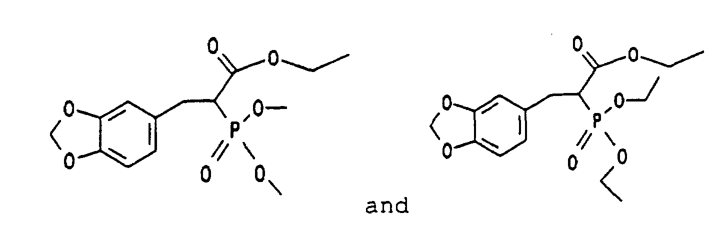 are excepted.

2. A compound of the formula I as claimed in claim 1, where at least two of the radicals R¹, R² and R³ are present and are, independently of one another,
1) OH
2) (C₁-C₆)-alkoxy,
3) -O-(C₁-C₆)-alkyl-COOH,
4) -O- (C₁-C₆)-alkyl-C (O)-O-(C₁-C₆)-alkyl,
5) (C₅-C₇)-cycloalkoxy,
6) (C₃-C₆)-alkenyloxy,
7) (C₅-C₇)-cycloalkyl-(C₁-C₂)-alkoxy,
8) heteroaryl-(C₁-C₂)-alkoxy where the heteroatoms are N and/or O,
9) heterocycloalkyl-(C₁-C₂)-alkoxy where the heteroatoms are N and/or O, the heterocycloalkyl radical is unsubstituted or substituted once to three times by (C₁-C₃)-alkyl, and the heterocycloalkyl group has five or six members,
10) phenyl-(C₁-C₂)-alkoxy,
11) benzyloxy substituted once to three times by halomethyl or (C₁-C₃)-alkoxy,
12) phenoxy substituted once to three times by (C₁-C₃)-alkoxy,
13) two of the radicals R¹, R² or R³ are both a group of the formula (II) 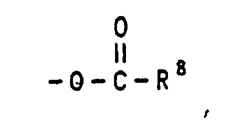 where R⁸ is (C₁-C₄)-alkyl, and
R⁵ is
1) CN,
2) CH₂NHR⁹ where R⁹ is hydrogen atom or -C(O)-(C₁-C₃)-alkyl, or
3) a radical of the formula VI 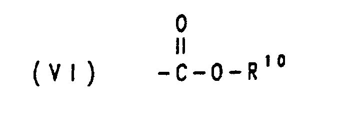 where
R¹⁰ is
1) (C₁-C₆)-alkyl, unsubstituted or substituted once by 1) -COOH, 2) -C(O)-(C₁-C₃)-alkyl or 3) 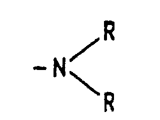 where R is (C₁-C₃)-alkyl, or
2) trialkylsilyl,
R⁶ and R⁷ are, independently of one another, hydrogen atom or (C₁-C₄)-alkyl, and
(C-A-C) is 1) (-CH₂-CH) or 2) (-CH=C).

3. A compound of the formula I as claimed in claim 1 or 2, where
R¹ is methoxy,
R² is methoxy or benzyloxy,
R³ is methoxy or benzyloxy, and
R⁵ is
1) CN,
2) CH₂NHR⁹ where R⁹ is hydrogen atom or -C(O)-(C₁-C₃)-alkyl or
3) a radical of the formula VI, where R¹⁰ is (C₁-C₄)-alkyl substituted by
1) -COOH or
2) 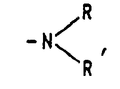 where R is hydrogen atom and/or (C₁-C₃)-alkyl,
R⁶ and R⁷ are hydrogen atom or (C₁-C₄)-alkyl, and
(C-A-C) is a (-CH=C) radical.

4. A compound of the formula I as claimed in one or more of claims 1 to 3, where
R¹ is hydrogen atom and
R² and R³ are both methoxy, and
R⁵ is a group of the formula VI where
R¹⁰ is isopropyl,
R⁶ and R⁷ are both ethyl or methyl, and (C-A-C) is a (-CH₂-CH) radical.

5. A compound of the formula I as claimed in one or more of claims 1 to 4, where R¹ is hydrogen atom and
R2 and R3 are both benzyloxy, and
R⁵ is a group of the formula VI where
R¹⁰ is isopropyl,
R⁶ and R⁷ are methyl or ethyl, and (C-A-C) is a (-CH=C) radical.

6. A process for preparing the compound of the formula I, which comprises
a) reacting a compound of the formula VII 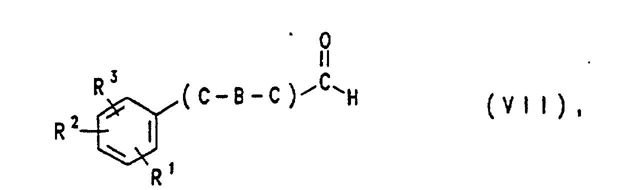 where R¹, R² and R³ are as defined in formula I, and (C-B-C) is a covalent bond, (-CH=CH-), (-CH₂-CH₂-CH₂), (-CH₂-CH₂) or (-CH₂-), with a compound of the formula VIII or with a salt of a compound of the formula VIII 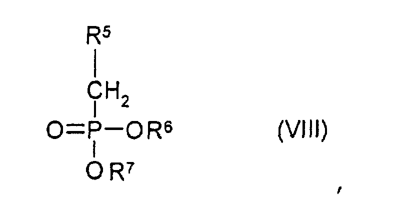 where R⁵, R⁶ and R⁷ are defined as in formula I, in the presence of tetrahydrofuran and titanium tetrachloride or in the presence of tetrahydrofuran and an orthotitanic triester of the formula IX
(IX) (R¹¹)₃TiCl,
where R¹¹ is -O-(C₁-C₆)-alkyl, or
b) fractionating a compound of the formula I which has been prepared by process a) and which, by reason of its chemical structure, occurs in enantiomeric or stereoisomeric forms, by salt formation with enantiomerically pure acids or bases, chromatography on chiral stationary phases or derivatization using chiral enantiomerically pure compounds such as amino acids, separation of the diastereomers obtained in this way, and elimination of the chiral auxiliary group, into the pure enantiomers, or fractionating the stereoisomers by chromatography, or
c) hydrolyzing a compound of the formula I which has been prepared as in a), where at least one R¹, R² or R³ is a radical of the formula II or III, to the corresponding phenol, or
d) carrying out the reaction of process c) in the presence of sodium bicarbonate,
e) hydrolyzing a compound of the formula I which has been prepared as in a), where R⁵ is a radical of the formula VI and/or at least one R¹, R² or R³ is a radical -O- (C₁-C₁₂) -alkyl-C (O)-O-(C₁-C₁₂)-alkyl, to the carboxylic acid, or
f) carrying out the reaction of process e) in the presence of an ethanolic potassium hydroxide solution or of a hydrochloric acid solution, or
g) hydrogenating a compound of the formula I which has been prepared as in a) and contains one or two double bonds with α.) hydrogen and a Pd/C catalyst or Raney nickel or β.) sodium borohydride, or
h) hydrolyzing a monoalkyl or dialkyl phosphonate of the formula I which has been prepared as in a) to the phosphonic monoester or to the phosphonic acid, carrying out the cleavage of the phosphonic ester in the presence of bromotrimethylsilane in dichloromethane, or
i) either isolating the compound of the formula I which has been prepared by process a), b), c), e), g), h), k) or 1) in free form or, if acidic or basic groups are present, converting it into physiologically tolerated crystalline salts, or
k) reacting a compound of the formula I where at least one of the radicals R¹, R² or R³ is a hydroxyl radical with a compound of the formula XI
(XI) X-R¹³,
where
X is halogen or optionally substituted phenyl-sulfonyloxy, and
R¹³ is defined as for R¹ 2) to 12) in formula I, X replacing the oxygen atom of R¹ which is linked to the phenyl radical
after treatment with sodium hydride or in the presence of potassium carbonate in acetonitrile, dimethylformamide or cyclic ketones, or with R¹³COCl or 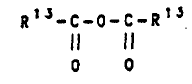 where R¹³ is defined as R⁸, where appropriate with catalysis by nitrogen bases such as pyridine, to give the corresponding phenol ethers or phenol esters,
l) hydrogenating a compound of the formula I which has been prepared by process a), where R⁵ is CN, in the presence of hydrogen and a Pd/C catalyst or Raney nickel by the process described under C)α.) or
m) reacting an amino compound which has been obtained by process 1) with a (C₁-C₃)-alkyl-carboxylic anhydride to give the corresponding carboxamide, or
n) converting a compound of the formula I which has been prepared by process a), where R⁵ is -COOH, by esterification into the corresponding carboxylic ester, where the carboxylic acids are converted with oxalyl chloride into the carbonyl chloride, and the latter is reacted with R¹⁰-OH.

7. A pharmaceutical containing an effective amount of at least one compound of the formula (I) 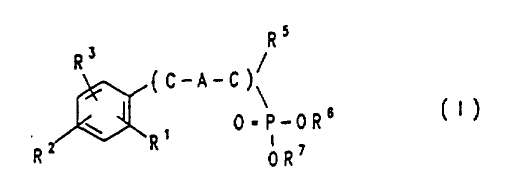 and/or a physiologically tolerated salt of the compound of the formula (I) and/or a stereoisomeric form of the compound of the formula (I), where at least two of the radicals R¹, R² and R³ are present and are, independently of one another,
1) OH,
2) (C₁-C₁₂)-alkoxy,
3) -O-(C₁-C₁₂)-alkyl-COOH,
4) -O-(C₁-C₁₂)-alkyl-C (O)-O-(C₁-C₁₂)-alkyl,
5) (C₃-C₁₂)-cycloalkoxy,
6) (C₃-C₆)-alkenyloxy,
7) (C₅-C₇)-cycloalkyl-(C₁-C₃)-alkoxy,
8) heteroaryl-(C₁-C₃)-alkoxy where the heteroatoms are N, S and/or O,
9) heterocycloalkyl-(C₁-C₃)-alkoxy where the heteroatoms are N, S and/or O, the heterocycloalkyl radical is unsubstituted or substituted once to three times by (C₁-C₃)-alkyl, and the heterocycloalkyl group has five or six members,
10) phenyl-(C₁-C₃)-alkoxy,
11) benzyloxy substituted once to three times by halomethyl or (C₁-C₃)-alkoxy,
12) phenoxy substituted once to three times by (C₁-C₃)-alkoxy,
13) two of the radicals R¹, R² or R³ which are substituents on two directly adjacent carbon atoms of the aromatic ring together form a methylenedioxy or ethylenedioxy radical on the aromatic ring,
14) a radical of the formula II, III or IV 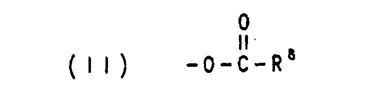 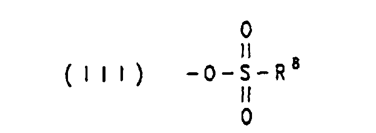 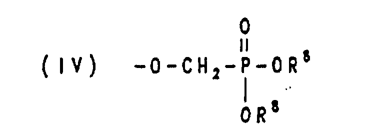 where
R⁸ is (C₁-C₄)-alkyl, or hydrogen atom
15) a group of the formula V 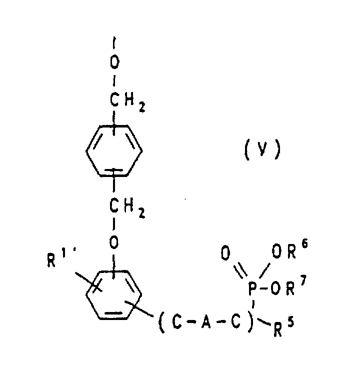 where
R^{1'} is defined as for R¹ from 1) to 12),
and (C-A-C), R⁵, R⁶ and R⁷ are as defined below, or
16) R¹ and R⁷ form a compound of the formula Ia 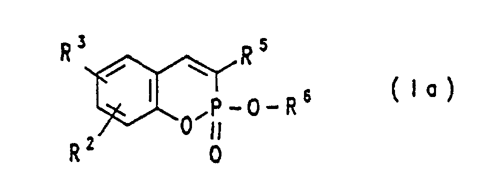 where R² and R³ are as defined above and R⁵ and R⁶ are as defined below, or
17) R¹ and R⁵ form a compound of the formula Ib 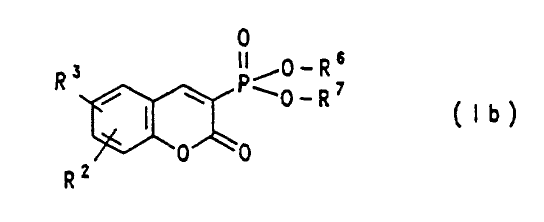 where R² and R³ are as defined above and R⁶ and R⁷ are as defined below, and
R⁵ is
1) CN,
2) CH₂NHR⁹ where R⁹ is hydrogen atom or -C(O)-(C₁-C₃)-alkyl, or
3) a radical of the formula VI 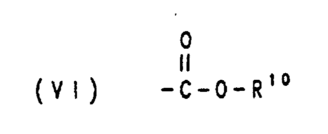 where
R¹⁰ is
1) (C₁-C₆)-alkyl, unsubstituted or substituted once to four times by 1.1 -COOH, 1.2 -C(O)-O-(C₁-C₃)-alkyl or 1.3 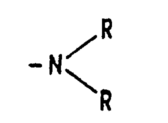 where R is 1.3.1 hydrogen atom, 1.3.2 (C₁-C₃)-alkyl or 1.3.3 forms, together with the nitrogen atom to which it is bonded, a morpholine ring, or
2) trialkylsilyl,
R⁶ and R⁷ are, independently of one another,
1) hydrogen atom or
2) (C₁-C₆)-alkyl, and
(C-A-C) is
1) (CH=CH-CH=CH-CH),
2) (CH₂-CH₂-CH₂-CH₂-CH),
3) (CH₂-CH₂-CH₂-CH),
4) (CH₂-CH₂-CH),
5) (-CH₂-CH) or
6) (-CH=C),
with the proviso that
the compound of the formula I where (C-A-C) is (C=CH), R⁵ is a CN group, R⁶ and R⁷ are identical and are ethyl, R¹ and R² are both -OH and are bonded in the meta and para position to (C-A-C), and R³ is hydrogen atom is excepted.

8. The use of at least one compound of the formula (I) as claimed in one or more of claims 1 to 5 for producing pharmaceuticals for the treatment and prophylaxis of degenerative joint disorders, of rheumatic disorders accompanied by cartilage breakdown, such as rheumatoid arthritis, joint trauma and chondrolysis as a consequence of prolonged immobilization of the joint, of inflammations, septic shock, disorders with impaired leukocyte adhesion, disorders caused by an elevated concentration of tumor necrosis factor alpha, such as cachexia or Crohn's disease.

9. A process for producing a pharmaceutical as claimed in claim 7, which comprises converting at least one compound of the formula (I) and/or a compound obtained by the process as claimed in claim 6 into a suitable dosage form with physiologically acceptable ancillary substances and vehicles and, where appropriate, further additives and/or other active substances.

## Revendications

1. Composé de formule (I) 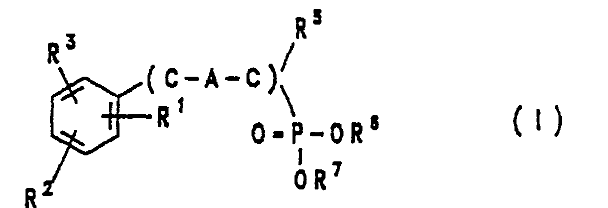 et/ou un sel physiologiquement acceptable du composé de formule (I) et/ou une forme stéréoisomère du composé de formule (I), dans lequel au moins deux des groupes R¹, R² et R³ sont présents et représentent indépendamment un groupe
1) OH
2) alcoxy en C₁ à C₁₂,
3) -O-(alkyl en C₁ à C₁₂)-COOH,
4) -O-(alkyl en C₁ à C₁₂)-C(O)-O-(alkyle en C₁ à C₁₂),
5) cycloalcoxy en C₃ à C₁₂,
6) alcényloxy en C₃ à C₆,
7) cycloalkyl en C₅ à C₇-alcoxy en C₁ à C₃,
8) hétéroaryl-alcoxy en C₁ à C₃, dans lequel les hétéroatomes sont N, S et/ou O
9) hétérocycloalkyl-alcoxy en C₁ à C₃, dans lequel les hétéroatomes sont N, S et/ou O, le groupe hétérocycloalkyle est non substitué ou une à trois fois substitué avec un groupe alkyle en C₁ à C₃ et le groupe hétérocycloalkyle est à cinq ou six chaînons,
10) phényl-alcoxy en C₁ à C₃,
11) benzyloxy, une à trois fois substitué par un groupe halogénométhyle ou alcoxy en C₁ à C₃,
12) phénoxy, une à trois fois substitué par un groupe alcoxy en C₁ à C₃,
13) deux des groupes R¹, R² ou R³, qui sont des substituants de deux atomes de carbone directement voisins du cycle aromatique, forment ensemble un groupe méthylènedioxy ou éthylènedioxy sur le cycle aromatique,
14) représentent un groupe des formules II, III ou IV 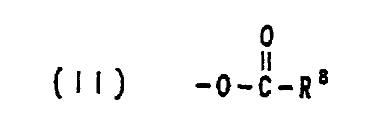 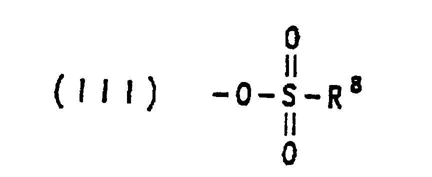 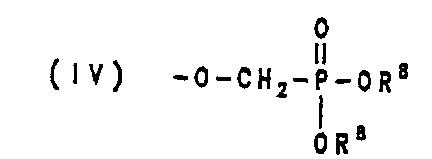 dans lesquelles R⁸ représente un groupe alkyle en C₁ à C₄ ou un atome d'hydrogène,
15) un groupe de formule V 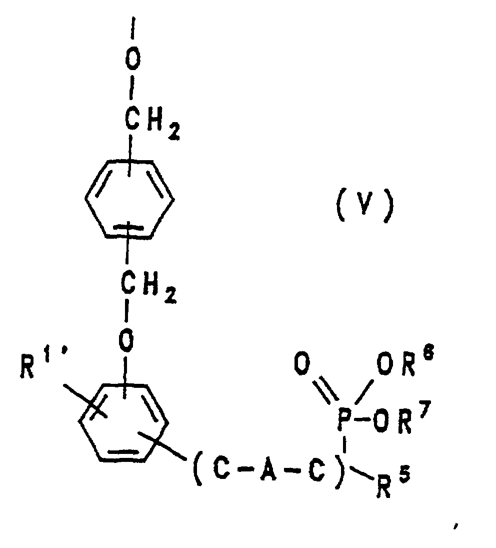 dans laquelle
R^{1'} est défini comme pour R¹ de 1) à 12) et (C-A-C), R⁵, R⁶ et R⁷ sont définis comme ci-dessous, ou
16) R¹ et R⁷ sont une liaison covalente et forment alors un composé de formule Ia 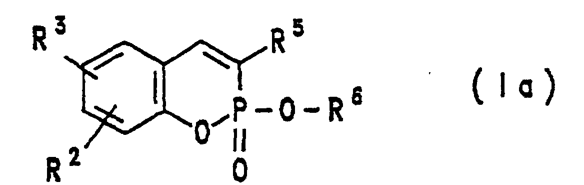 dans laquelle, R² et R³ sont définis comme ci-dessus et R⁵ et R⁶ comme ci-dessous, ou
17) R¹ et R⁵ sont une liaison covalente et forment ainsi un composé de formule Ib 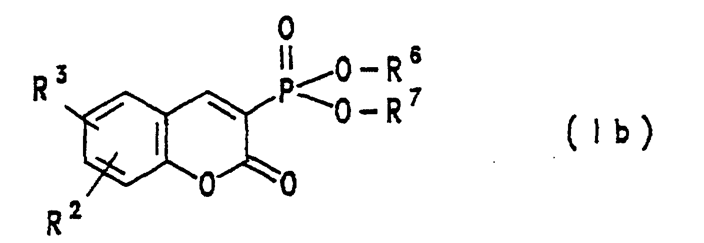 dans laquelle, R² et R³ sont définis comme ci-dessus et R⁶ et R⁷ comme ci-dessous, et
R⁵ représente
1) CN,
2) CH₂NHR⁹, dans. laquelle R⁹ représente un atome d'hydrogène ou -C(O)-(alkyle en C₁ à C₃), ou
3) un groupe de formule VI 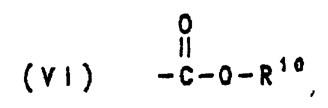 dans laquelle
R¹⁰ représente
1) un groupe alkyle en C₁ à C₆, non substitué ou une à quatre fois substitué par 1.1 -COOH, 1.2 -C(O)-O-alkyle en C₁ à C₃ ou 1.3, 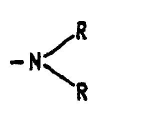 dans laquelle R représente 1.3.1 un atome d'hydrogène, 1.3.2. un groupe alkyle en C₁ à C₃ ou 1.3.3 forme ensemble avec l'atome N auquel il est lié un cycle morpholine, ou
2) un groupe trialkylsilyle,
R⁶ et R⁷ représentent indépendamment l'un de l'autre
1) un atome d'hydrogène ou
2) un groupe alkyle en C₁ à C₆,
(C-A-C) représente
1) (CH=CH-CH=C),
2) (CH₂-CH₂-CH₂-CH),
3) (CH₂-CH₂-CH)
4) (-CH₂-CH) ou
5) (CH=CH),
avec la condition, que
lorsque le groupe R⁵ représente un groupe CN, au maximum un des groupes R¹, R² ou R³ représente un groupe hydroxyle, ou
lorsque le groupe R¹⁰ représente le méthyle, l'éthyle ou le tert.-butyle, les groupes R¹, R² ou R³ ne représentent pas un groupe méthoxy, et les composés 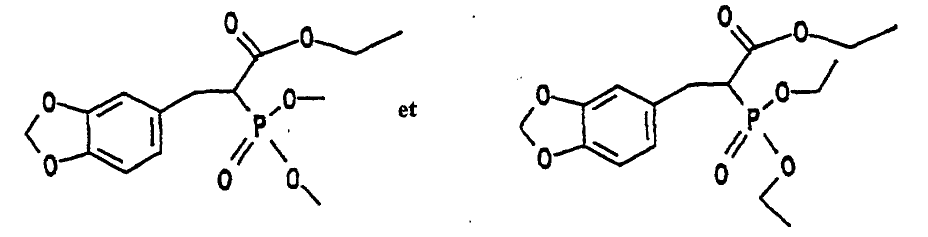 sont exclus.

2. Composé de formule I selon la revendication 1, dans lequel au moins deux des groupes R¹, R² et R³ sont présents et représentent indépendamment les uns des autres un groupe
1) OH
2) alcoxy en C₁ à C₆,
3) -O-(alkyl en C₁ à C₆)-COOH,
4) -O-(alkyl en C₁ à C₆)-C(O)O-(alkyle en C₁ à C₆),
5) cycloalcoxy en C₅ à C₇,
6) alcényloxy en C₃ à C₆,
7) cycloalkyl en C₅ à C₇-alcoxy en C₁ à C₂,
8) hétéroaryl-alcoxy en C₁ à C₂, dans lequel les hétéroatomes sont N et/ou O
9) hétérocycloalkyl-alcoxy en C₁ à C₂, dans lequel les hétéroatomes sont N et/ou O, le groupe hétérocycloalkyle est non substitué ou une à trois fois substitué avec un groupe alkyle en C₁ à C₃ et le groupe hétérocycloalkyle est à cinq ou six chaînons,
10) phényl-alcoxy en C₁ à C₂,
11) benzyloxy, une à trois fois substitué par un groupe halogénométhyle ou alcoxy en C₁ à C₃ ou
12) phénoxy, une à trois fois substitué par un groupe alcoxy en C₁ à C₃,
13) deux des groupes R¹, R² ou R³ représentent en même temps un groupe de formule (II) 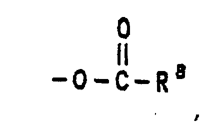 dans laquelle R⁸ représente un groupe alkyle en C₁ à C₄, et
R⁵ représente
1) CN,
2) CH₂NHR⁹, dans laquelle R⁹ représente un atome d'hydrogène ou
-C(O)-(alkyle en C₁ à C₃), ou
3) un groupe de formule VI 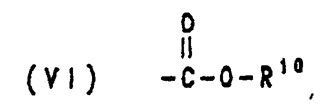 dans laquelle
R¹⁰ représente
1) un groupe alkyle en C₁ à C₆, non substitué ou une fois substitué par 1) -COOH, 2) -C(O)-O-alkyle en C₁ à C₃ ou 3) 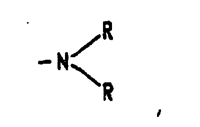 dans laquelle R représente un groupe alkyle en C₁ à C₃, ou
2) un groupe trialkylsilyle, R⁶ et R⁷ représentent indépendamment l'un de l'autre un atome d'hydrogène ou un groupe alkyle en C₁ à C₄, et (C-A-C) représente 1) (-CH₂-CH) ou 2) (-CH=C).

3. Composé de formule I selon les revendications 1 ou 2 dans lequel
R¹ représente un groupe méthoxy,
R² un groupe méthoxy ou benzyloxy,
R³ un groupe méthoxy ou benzyloxy, et
R⁵ représente
1) CN,
2) CH₂NHR⁹, dans laquelle R⁹ représente un atome d'hydrogène ou -C(O)-(alkyle en C₁ à C₃), ou
3) un groupe de formule VI, dans laquelle
R¹⁰ représente un groupe alkyle en C₁ à C₄ substitué par
1) -COOH, ou
2) 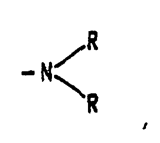 dans laquelle R représente un groupe alkyle en C₁ à C₃,
R⁶ et R⁷ représentent un atome d'hydrogène ou un groupe alkyle en C₁ à C₄ et
(C-A-C) représente un groupe (-CH=C).

4. Composé de formule I selon une ou plusieurs des revendications 1 à 3, dans lequel
R¹ représente un atome d'hydrogène et
R² et R³ représentent tous les deux un groupe méthoxy, et
R⁵ représente un groupe de formule VI, dans laquelle R¹⁰ représente un groupe isopropyle,
R⁶ et R⁷ représentent en même temps un groupe éthyle ou méthyle et (C-A-C) représente un groupe (-CH₂-CH).

5. Composé de formule I selon une ou plusieurs des revendications 1 à 4, dans lequel
R¹ représente un atome d'hydrogène et
R² et R³ représentent tous les deux un groupe benzyloxy, et
R⁵ représente un groupe de formule VI, dans laquelle R¹⁰ représente un groupe isopropyle,
R⁶ et R⁷ représentent un groupe méthyle ou éthyle et (C-A-C) représente un groupe (-CH=C).

6. Procédé pour la préparation du composé de formule I, **caractérisé en ce que**
a) l'on met à réagir un composé de formule VII 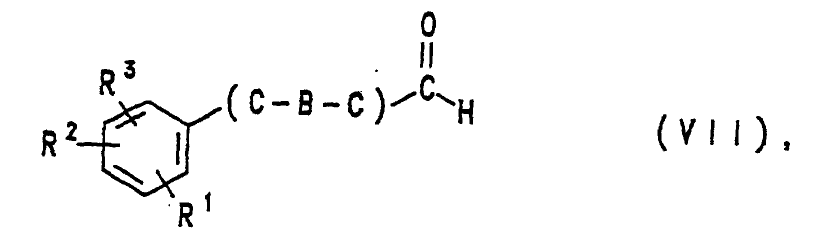 dans laquelle R¹, R² et R³ sont comme définis à la formule I et (C-B-C) représente une liaison covalente, (-CH=CH-), (-CH₂-CH₂-CH₂), (-CH₂-CH₂) ou (-CH₂-), avec un composé de formule VIII ou avec un sel d'un composé de formule VIII 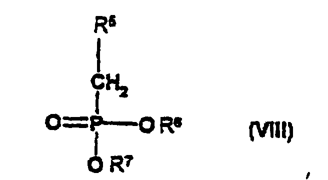 dans laquelle R⁵, R⁶ et R⁷ sont définis comme à la formule I,
en présence de tétrahydrofurane et de tétrachlorure de titane ou en présence de tétrahydrofurane et d'un triester d'acide orthotitanique de formule IX
(IX) (R¹¹)₃TiCl
dans laquelle R¹¹ représente -O-alkyle en C₁ à C₆, ou
b) on sépare un composé de formule I préparé selon le procédé a), qui se présente par suite de sa structure chimique sous des formes énantiomères ou stéréoisomères, par formation de sel avec des acides ou des bases énantiomères purs, chromatographie sur des phases stationnaires chirales ou transformation en dérivés au moyen de composés chiraux énantiomères purs comme des acides aminés, séparation des diastéréoisomères ainsi obtenus, et clivage du groupe auxiliaire chiral pour obtenir les énantiomères purs ou on sépare les stéréoisomères par chromatographie, ou
c) on saponifie un composé de formule 1 préparé selon a), dans laquelle au moins l'un de R¹, R² ou R³ est un groupe de formule II ou III, pour obtenir le phénol correspondant, ou
d) on réalise la réaction selon le procédé c) en présence d'hydrogénocarbonate de sodium,
e) on saponifie un composé de formule 1 préparé selon a), dans laquelle R⁵ représente un groupe de formule VI et/ou au moins l'un de R¹, R² ou R³ est un groupe -O-alkyl en C₁ à C₁₂-C(O)-O-alkyle en C₁ à C₁₂, pour obtenir un acide carboxylique, ou
f) on réalise la réaction selon le procédé e) en présence d'une solution éthanolique d'hydroxyde de potassium ou d'une solution d'acide chlorhydrique, ou
g) on effectue l'hydrogénation d'un composé de formule I préparé selon a), contenant une ou deux doubles liaisons avec α) l'hydrogène et un catalyseur Pd/C ou nickel de Raney ou β) le borohydrure de sodium, ou
h) on saponifie un ester monoalkylique ou dialkylique d'acide phosphonique de formule I préparé selon a) pour obtenir un hémiester d'acide phosphonique ou l'acide phosphonique, dans lequel le clivage de l'ester d'acide phosphonique est réalisé en présence de bromotriméthylsilane dans le dichlorométhane, ou
i) on isole le composé de formule I préparé selon le procédé a), b), c), e), g), h), k) ou 1) soit sous forme libre ou on le transforme, dans le cas de la présence de groupes acides ou basiques, éventuellement en sels cristallins physiologiquement acceptables, ou
k) on met à réagir un composé de formule I, dans laquelle au moins l'un des groupes R¹, R² ou R³ est un groupe hydroxyle avec un composé de formule XI
(XI) X-R¹³,
dans laquelle
X est un atome d'halogène ou un groupe phénylsulfonyloxy éventuellement substitué et
R¹³ est défini comme R¹ 2) à 12) dans la formule I, dans laquelle X remplace l'atome O de R¹, qui est relié au groupe phényle,
après traitement avec l'hydrure de sodium ou en présence de carbonate de potassium dans l'acétonitrile, le diméthylformamide ou les cétones cycliques ou on le met à réagir avec R¹³COCl ou 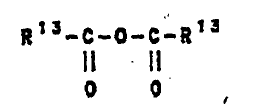 dans laquelle R¹³ est défini comme R⁸, on catalyse éventuellement par des bases azotées comme la pyridine, pour obtenir les éthers de phénol ou les esters de phénol correspondants.
1) on réalise l'hydrogénation d'un composé de formule I préparé selon le procédé a), dans laquelle R⁶ représente CN, en présence d'hydrogène et d'un catalyseur Pd/C ou nickel de Raney, selon le procédé décrit en C)α), ou
m) on met à réagir un composé amino obtenu selon le procédé 1) avec un anhydride d'acide alkylcarboxylique, pour obtenir le carboxamide correspondant, ou
n) on met à réagir un composé de formule I préparé selon le procédé a), dans laquelle R⁵ représente COOH, par estérification pour obtenir l'ester d'acide carboxylique correspondant, dans lequel on transforme les acides carboxyliques au moyen de chlorure d'oxalyle en chlorure d'acide carboxylique et on met celui-ci à réagir avec R¹⁰-OH.

7. Médicament, contenant une quantité efficace d'au moins un composé de formule (I) 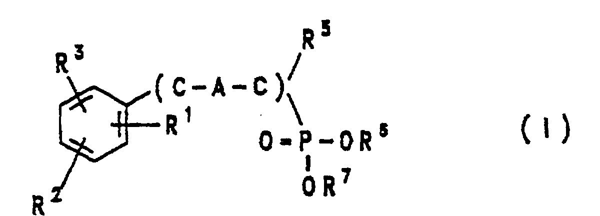 et/ou d'un sel physiologiquement acceptable du composé de formule (I) et/ou d'une forme stéréoisomère du composé de formule (I), dans lequel au moins deux des groupes R¹, R² et R³ sont présents et représentent indépendamment un groupe
1) OH
2) alcoxy en C₁ à C₁₂,
3) -O-(alkyl en C₁ à C₁₂)-COOH,
4) -O-(alkyl en C₁ à C₁₂)-C(O)-O-(alkyle en C₁ à C₁₂),
5) cycloalcoxy en C₃ à C₁₂,
6) alcényloxy en C₃ à C₆,
7) cycloalkyl en C₅ à C₇-alcoxy en C₁ à C₃,
8) hétéroaryl-alcoxy en C₁ à C₃, dans lequel les hétéroatomes sont N, S et/ou O
9) hétérocycloalkyl-alcoxy en C₁ à C₃, dans lequel les hétéroatomes sont N, S et/ou O, le groupe hétérocycloalkyle est non substitué ou une à trois fois substitué avec un groupe alkyle en C₁ à C₃ et le groupe hétérocycloalkyle est à cinq ou six chaînons,
10) phényl-alcoxy en C₁ à C₃,
11) benzyloxy, une à trois fois substitué par un groupe halogénométhyle ou alcoxy en C₁ à C₃,
12) phénoxy, une à trois fois substitué par un groupe alcoxy en C₁ à C₃,
13) deux des groupes R¹, R² ou R³, qui sont des substituants de deux atomes de carbone directement voisins du cycle aromatique, forment ensemble un groupe méthylènedioxy ou éthylènedioxy sur le cycle aromatique,
14) représentent un groupe des formules II, III ou IV 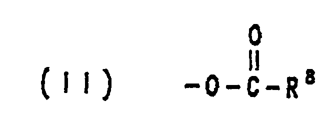 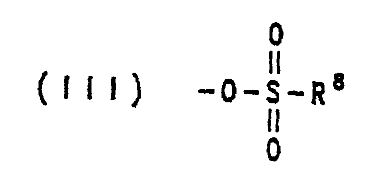 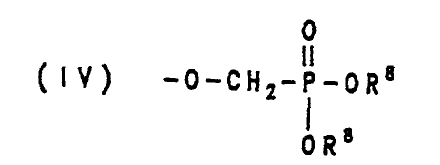 dans lesquelles R⁸ représente un groupe alkyle en C₁ à C₄ ou un atome d'hydrogène,
15) un groupe de formule V 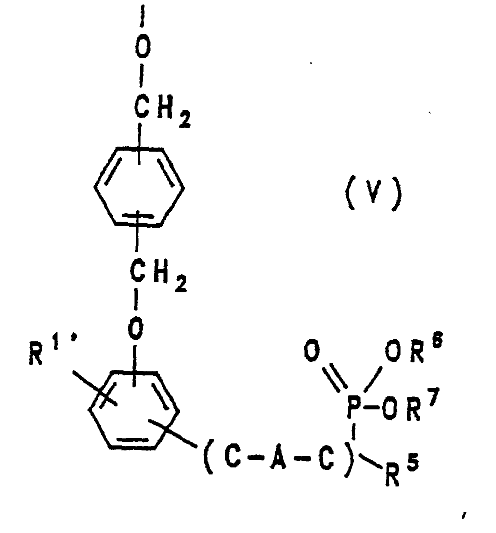 dans laquelle
R^{1'} est défini comme pour R¹ de 1) à 12) et (C-A-C), R⁵, R⁶ et R⁷ sont définis comme ci-dessous, ou
16) R¹ et R⁷ forment un composé de formule la 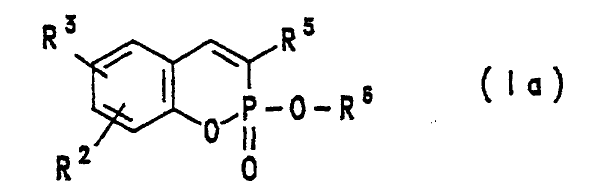 dans laquelle, R² et R³ sont définis comme ci-dessus et R⁵ et R⁶ comme ci-dessous, ou
17) R¹ et R⁵ forment un composé de formule Ib 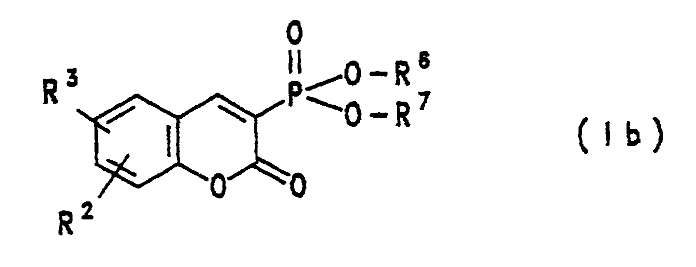 dans laquelle, R² et R³ sont définis comme ci-dessus et R⁶ et R⁷ comme ci-dessous, et
R⁵ représente
1) CN,
2) CH₂NHR⁹, dans laquelle R⁹ représente un atome d'hydrogène ou
-C(O)-(alkyle en C₁ à C₃), ou
3) un groupe de formule VI 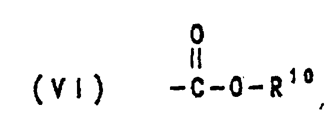 dans laquelle
R¹⁰ représente
1) un groupe alkyle en C₁ à C₆, non substitué ou une à quatre fois substitué par 1.1 -COOH, 1.2 -C(O)-O-alkyle en C₁ à C₃ ou 1.3 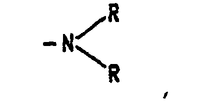 dans laquelle R représente 1.3.1 un atome d'hydrogène, 1.3.2 un groupe alkyle en C₁ à C₃ ou 1.3.3 forme ensemble avec l'atome N auquel il est lié un cycle morpholine, ou
2) un groupe trialkylsilyle,
R⁶ et R⁷ représentent indépendamment l'un de l'autre
1) un atome d'hydrogène ou
2) un groupe alkyle en C₁ à C₆, et
(C-A-C) représente
1) (CH=CH-CH=CH-CH),
2) (CH₂-CH₂-CH₂-CH₂-CH),
3) (CH₂-CH₂-CH₂-CH)
4) (CH₂-CH₂-CH),
5) (-CH₂-CH) ou
6) (-CH=C),
avec la condition, que soit exclu
le composé de formule I, dans laquelle (C-A-C) représente (C=CH), R⁵ représente un groupe CN, R⁶ et R⁷ sont identiques et représentent un groupe éthyle, R¹ et R² représentent en même temps OH et sont liés en position méta et para sur (C-A-C) et R³ représente un atome d'hydrogène.

8. Utilisation d'au moins un composé de formule (I) selon une ou plusieurs des revendications 1 à 5, pour la préparation de médicaments pour le traitement et la prophylaxie de maladies dégénératives des articulations, de maladies de type rhumatoide qui sont accompagnées d'une décomposition du cartilage comme la polyarthrite chronique, le traumatisme articulaire ainsi que l'atrophie des articulations à la suite d'une très longue immobilisation de l'articulation, d'inflammations, de chocs septiques, de maladies avec une adhésion des leucocytes perturbée, qui sont dues à une augmentation de la concentration du Facteur de nécrose des tumeurs comme la cachexie ou Morbus Crohn.

9. Procédé pour la préparation d'un médicament selon la revendication 7, **caractérisé en ce qu'**on met sous une forme d'administration appropriée au moins un composé de formule (I) et/ou un composé obtenu avec le procédé selon la revendication 6 avec des substances d'adjuvants et de véhicules physiologiquement acceptables et éventuellement d'autres additifs et/ou d'autres substances actives.
